Europäisches Patentamt

European Patent Office

Office européen des brevets

(19)

(11) **EP 0 832 422 B1**

(12) **EUROPEAN PATENT SPECIFICATION**

(45) Date of publication and mention
of the grant of the patent:
**04.12.2002  Bulletin 2002/49**

(51) Int Cl.⁷: $G01J\ 3/50$, A61B 5/103

(86) International application number:
**PCT/IB96/00732**

(21) Application number: **96922188.6**

(22) Date of filing: **06.06.1996**

(87) International publication number:
**WO 96/041140 (19.12.1996 Gazette 1996/55)**

(54) **METHOD AND APPARATUS FOR DETECTING AND MEASURING CONDITIONS AFFECTING COLOR**

VERFAHREN UND VORRICHTUNG ZUM NACHWEIS UND MESSEN VON ZUSTÄNDEN,WELCHE DIE FARBE BEEINFLUSSEN

PROCEDE ET APPAREIL DE DETECTION ET DE MESURE D'ETATS AFFECTANT LA COULEUR

(84) Designated Contracting States:
**AT BE CH DE DK ES FI FR GB GR IE IT LI LU MC NL PT SE**

(30) Priority:  **07.06.1995  US 481177**

(43) Date of publication of application:
**01.04.1998  Bulletin 1998/14**

(73) Proprietor: **CHROMATICS COLOR SCIENCES INTERNATIONAL, INC.**
**New York, NY 10021 (US)**

(72) Inventors:
• **MACFARLANE, Darby, S.**
**Hastings-on-Hudson, NY 10706 (US)**
• **MACFARLANE, David, Kenneth**
**Hastings-on-Hudson, NY 10706 (US)**

• **BILLMEYER, Fred, W., Jr.**
**Schenectady, NY 12309-5746 (US)**

(74) Representative: **Lawrence, John Gordon**
**Lloyd Wise, McNeight & Lawrence**
**Regent House**
**Heaton Lane**
**Stockport, Cheshire SK4 1BS (GB)**

(56) References cited:
**DE-A- 3 827 457**       **US-A- 4 029 085**
**US-A- 5 313 267**

• **MANFRED RICHTER: "Einführung in die Farbmetrik, 2.Auflage", 1981, WALTER DE GRUYTER, BERLIN**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## EP 0 832 422 B1

**Description**

Visual observation of a subject for changes in coloration indicative of a particular condition has often occurred. The subject may be a person or animal being observed to determine the presence or absence of a medical condition. The color characteristics or a single color characteristic of other test subjects such as biopsy or excretions have diagnostic value.

An individual person's skin color is often assessed by her or his doctor. Hypertension, tuberculosis, sclerosis of the liver, to name just a few, are examples of ailments with symptomatic skin color changes among at least a sizeable population segment. Hair color evaluation and dental coloration evaluation are valuable. These may bear on the health of the individual, or on the health of the individual's hair and teeth, or these may permit accurate cosmetic activities, for example, to counteract graying or to accurately match new dental work to existing teeth.

Likewise, the condition of plants and agricultural products is visually inspected for color as an indication of condition. Contamination of soil is likewise apparent from visual inspection. Such visual inspections are subjective. Measuring by instrument the color characteristics that are key to the visual inspection has the benefit of objectivity and consistency.

In the past, hyperbilirubinemia in newborns has been detected by visually observing an individual for jaundice or by routinely taking and testing a blood sample. Upon detection, hyperbilirubinemia has been treated by phototherapy. During the course of phototherapy, blood samples have been taken and tested at regular intervals until it was determined that the level of serum bilirubin had decreased to an acceptable level.

In infants, there is little blood available for use in the blood testing for hyperbilirubinemia. So much blood is drawn that transfusions are often necessary to replace the drawn blood. The newborn is thereby exposed to all of the risks that transfusions bring. Blood sampling and transfusions are, of course, painful to the newborn, and as with any invasive procedure, both present medical risks, such as for example, risk of infection. There is a need, therefore, for a reliable noninvasive technique for detecting and measuring a skin color affecting medical condition such as hyperbilirubinemia.

This is one example of a wider need for procedures and instruments to objectively and consistently determine a color characteristic or factor indicative of the condition of a test subject or indicative of a particular ailment or condition. The methods and apparatus of this invention can be employed where previously visual inspection, of which examples are given above, has been carried out at least in part on the basis of observable color characteristics.

The disclosure of U.S. patents No. 5,311,293 and 5,313,267 teach measurement of the value of Hunter b in skin coloration for the purpose of assigning individuals to one of several color compatibility categories as a means for determining compatibility with non-skin objects of various colors.

The disclosure of U.S. patent No. 4,909,632 of Macfarlane teaches the measurement of blue to yellow ratio in skin coloration for the purpose of classification of an individual in categories of color compatibility. Non-skin matter is classified for its compatibility with the skin coloration of individuals thus classified.

The disclosure of U.S. patent No. 4,029,085 suggests a method for determining bilirubin concentration in blood serum by measuring skin reflectance at a number of specific wave lengths in the visible spectrum. The patent does not suggest the measurement of one or more color factor values, the establishment of a range of color factor values for comparison with a measured value, the measurement of a color factor at different points in time and determination of a change in measured color factor value or the use of a lightness measure color factor value for detection of a condition in a test subject.

The disclosure of German offenlegungschrift DE 38 27 457 A1 suggests measuring pigment-bearing elements in skin layers or in hair by measuring "remission," which is to say return radiation. The document mentions the possibility of using separate sensors for separate spectral intervals. The document also mentions use of the device for various medical measurements such as measuring bilirubin. However, there is no teaching of what should be measured for any of the medical purposes mentioned. Nor is it said what spectral regions should be detected for any particular purpose. Moreover, the measurement of a color factor indicative of a medical condition, or any condition, is not described.

Other publications, such as those cited in the above-listed documents, fail to suggest the measurement of change in a reliably measurable color factor value, or a quantification of the measurements that will indicate the condition for purposes of comparison, or the effect that lightness has on the measurement, and how that effect can be successfully addressed.

According to a first aspect of the present invention, a process for forming a compilation of colour factor values indicative of a medical or non-cosmetic physiological condition of a human or animal test subject, which condition is associated with a symptomatic, detectable skin coloration, the process comprises the steps of:

(a) compiling a group of lightness measure value ranges, and,

2

(b) associating with each lightness measure value range a value or range of values of a colour factor that indicates the condition for a test subject having a measured lightness within the lightness measure value range for use in comparison with a measurement of the value of that colour factor in the coloration of a test subject.

According to a second aspect of the invention, an apparatus for evaluating a medical or non-cosmetic physiological condition of a human or animal test subject, the condition is associated with a symptomatic, detectable skin coloration comprises:

(a) means for compiling a group of lightness measure value ranges, and,

(b) means for associating with each lightness measure value range a value or range of values of a colour factor for use in comparison with a measurement of the value of that colour factor in the coloration of a test subject having a measured lightness within said range, with the proviso that the apparatus is not used to determine a skin-coloured cosmetic for the test subject.

In one exemplary procedure making use of the invention, at least one skin color characteristic is measured at least at first and second points in time. To test for hyperbilirubinemia, the two measurements are then compared for change. A lightness skin color characteristic is also measured. On the basis of this measurement the subject can be assigned to one of plural categories among which varying amounts of change in the first-mentioned skin color characteristic are indicative of the presence of a medical condition. The first characteristic is then observed for a change of measured value sufficient to indicate the medical condition for a subject in that category. Preferably, a base reading of at least the first color characteristic is first made at a time the subject is without characteristic skin coloration indicative of the medical condition for which he or she is to be tested.

In the case of hyperbilirubinemia detection, the first skin color characteristic is Hunter b, which is a color factor dependent on the relative content, in a color, of two opponent colors, yellow and blue. Hunter b is a factor comprising a first function (Y) weighted in a first portion of the spectrum, the yellower portion, a second function (Z) weighted in a second portion of the spectrum, the bluer portion, and a weighting term $(1/Y^{1/2})$ that is a function of the lightness of a color and that decreases the value of the color factor as lightness increases. Y and Z are part of the three tristimulus values X, Y and Z known to the color scientist for the purpose of defining a color. They are measurable by commercially available instruments such as colorimeters.

In the case of testing newborns for hyperbilirubinemia, readings of Hunter b and the Hunter lightness measure L are made shortly after birth. These can provide the base reading since hyperbilirubinemia does not manifest itself immediately after birth. The first reading is preferably made within five hours, but as soon as possible after birth. Subsequent readings are then made during the next few days. The subsequent readings of Hunter b are compared with the first, baseline reading of Hunter b to determine whether Hunter b has increased to an extent that indicates a degree of jaundice characteristic of hyperbilirubinemia for a person having the range of the subject's particular skin lightness L. L is measured during each subsequent test to be sure that it remains close to the original reading. This gives a degree of confidence that the test procedures are being conducted appropriately.

In the event that the medical condition affecting skin color is detected in a procedure like that described above for hyperbilirubinemia, then the measuring of skin color characteristics continues at regular intervals until the symptomatic color characteristic abates sufficiently to indicate the individual's recovery from the medical condition. In the case of hyperbilirubinemia, phototherapy is administered once a sufficient change in Hunter b is observed to indicate the jaundice of hyperbilirubinemia. Throughout the course of phototherapy, then, the Hunter b and L characteristics are continually monitored until the jaundice has been eliminated. This has the value of permitting early removal of the newborn from under the phototherapy lamps, since there is the danger of damage to the newborn's eyes in the event eye protection is prematurely removed or accidentally dislodged.

The apparatus used in accordance with this invention includes a color measuring device such as a colorimeter and computational means for storing and comparing the characteristic or characteristics that are measured when testing for the condition. Where Hunter b is measured for the purpose of detecting hyperbilirubinemia, a colorimeter capable of calculating Hunter b and L can be used. This can be a commercially available colorimeter with this capability. The computational means preferably has sufficient memory to store one or more previous readings and should be programmed to compare previous and current readings to detect changes in Hunter b and L. Preferably the colorimeter and the computational means are integrated in a single instrument, but the commercial colorimeter can be utilized in cooperation with, for example, a personal computer, which stores and can compare Hunter b and L values from measurements taken at timed intervals. Likewise, the computational means, whether an integrated part of the instrument or a separate computer, can be used to store ranges of lightness L and the increases in Hunter b that, for the various lightness ranges, indicate an unacceptable increase in serum bilirubin.

Hunter L and b can be used to detect hyperbilirubinemia. Hunter L is monitored for consistency each time measurements are made. The change in Hunter b is monitored for a warning of hyperbilirubinemia. In another method according to the invention Hunter L, a and b are used. Hunter L is monitored for consistency, Hunter b is monitored for a warning of hyperbilirubinemia, and Hunter a is observed for additional information as to the infant's condition. The ordinary range of Hunter a for individuals is known. If Hunter a lies outside the ordinary range the reason for this should be determined. If it is because the infant is flushed from crying or has just been washed and rubbed dry, the Hunter a variation from the norm is not an indication of a medical problem. If Hunter a is above the ordinary range, but the infant has not been crying, has not been recently washed, phototherapy is not in progress, or some other non-medical reason is not apparent, a circulatory problem could be the reason. The situation bears watching to observe if a medical condition is present. Also, Hunter a sometimes increases just before the jaundice due to hyperbilirubinemia increases Hunter b. Hunter a, then, may be a warning, calling for closer observation to observe if a medical condition is present. A decrease in Hunter a along with an increase in both Hunter L and Hunter b such that the ratio of Hunter L to Hunter b remains essentially constant can mean that the infant is anemic and therefore pale, in which case the increase in Hunter b (with a simultaneous increase in Hunter L) would not be indicative of hyperbilirubinemia. The observation of Hunter a then may suggest various medical conditions and it allows one to understand the Hunter L and b readings better and to be more certain whether they are or are not indicative of hyperbilirubinemia.

Preferably, each skin color characteristic measurement used to assess the presence or absence of the condition for which testing is carried out is actually an average of multiple tests. For example, when newborns are tested for the jaundice that signals hyperbilirubinemia, multiple readings are made at multiple sites. Five or six Hunter value readings are made at, for example, each of several locations which may include some or all of a forehead location, at least one chest location, a cheek location and two back locations. Out of range Hunter L, a and b values are discarded. At each site, the Hunter readings that have the highest and lowest values of L, a and b are discarded, then all of the readings of each Hunter characteristic are averaged for each site. Subsequent readings are made in the same manner at exactly the same sites and compared. As used herein, the terms "Hunter a," "Hunter b," and "Hunter L" include such average values, but are not limited to just the values arrived at by the averaging technique unless expressly so-limited. The discarding and averaging is readily accomplished by the computational provisions of the test equipment. The averaging technique may improve the testing of other than skin color where the testing steps of this invention are used, for example in the evaluation of hair by color measurement.

In skin color testing, it is important to cleanse the site utilizing a cleansing agent that does not contribute any coloration. Likewise, when testing is carried out on test subjects other than an individual's skin, the test subject should be free of any color altering contaminant. In skin color testing, the site on the test subject should be dry, and in all cases the instrument should have the capability of being applied to the site in such a manner that ambient light does not enter the instrument.

Determination of the first and second skin color characteristics, Hunter L and b, at just one point in time can indicate or strongly suggest a medical condition affecting skin color if the first characteristic measurement is observed to lie outside a range of values for that characteristic known by experience to be normal for a subject having the particular measured value of the second characteristic. Again the value of Hunter a should be observed and if abnormal the reason should be sought. For example, in many individuals hyperbilirubinemia is strongly suggested if Hunter b and L are measured and it is determined that, based on skin color categories previously observed, Hunter b is above any ordinary value for a subject with skin having the L value measured. Also, even if baseline readings of Hunter b and L (and preferably a) are not made to establish values when no jaundice is present in the individual, changes in the value of Hunter b can nevertheless signal the presence of hyperbilirubinemia if measurements of the Hunter values are made at timed intervals in the foregoing fashion. Out of the ordinary increases in Hunter b, of for example two or more points, can be an indication of hyperbilirubinemia when the measured L value remains in a constant range from one measurement to the next. Similarly, large decreases in Hunter b, of for example two or more points, can be an indication of hyperbilirubinemia from which the infant is recovering, again if L remains relatively constant. If Hunter a changes due to a medical condition such as anemia and the ratio of Hunter L and Hunter b changes, then it is likely necessary to take the anemia into account, for example by using a different change in Hunter b to indicate hyperbilirubinemia or by multiplying Hunter b by a compensatory factor.

Significant testing has established the value of the foregoing techniques in detecting hyperbilirubinemia. The same techniques will indicate other jaundice-producing medical conditions in human and animal subjects. Hepatitis or liver disorders are examples of such medical conditions susceptible to diagnosis with the methods and apparatus of this invention.

Tuberculosis has been observed to affect skin color in dark skinned individuals such as many persons of African descent. Appropriate color measurement in accordance with this invention may provide a valuable diagnostic tool.

The above and further advantages of this invention will be better understood with reference to the following description of the preferred embodiment taken in combination with the attached drawings, in which:

Figure 1a is a block diagram illustration of an instrument for determining Hunter L, a and b values and for comparing changes in Hunter b to Hunter b changes predetermined to be indicative of hyperbilirubinemia.

Figure 1b is a diagrammatic illustration of exemplary memory content in an instrument like that of Fig. 1a.

Figure 2 is a schematic illustration in block diagram form illustrating the steps in the process of monitoring an infant for hyperbilirubinemia based upon changes in Hunter b in skin color and including measuring and reviewing Hunter b and L.

Figure 3 is a schematic illustration in block diagram form illustrating the steps in the process of monitoring an infant for hyperbilirubinemia based on Hunter b including measuring and reviewing Hunter a as well as Hunter b and L.

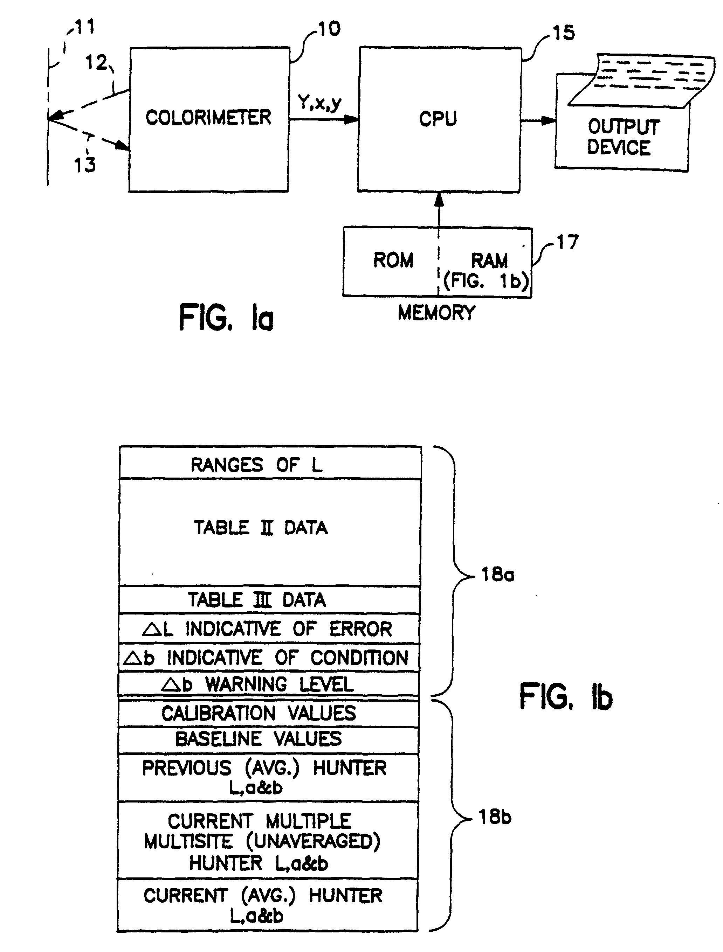

Any modern version of two general types of color-measuring instruments, colorimetrics and spectrophotometers, is an example of instruments suitable for the skin color measurement according to a preferred embodiment of this invention. The basic components of either type of instrument are a light source, a sample illumination and viewing arrangement, a means of selecting certain wavelengths of light for the measurement, a detector of the light reflected from the sample, and some relatively simple computing capacity. In commercially available instruments the main purposes of the computing capacity are to store and apply calibration information and to calculate various color coordinates for later use. In Fig. 1a, a color measuring instrument 10 is illustrated. An individual person's skin 11 is illuminated by the instrument as generally indicated by the broken line arrow 12, and the instrument receives illumination reflected from the skin 11 as generally indicated by the broken line arrow 13. Based on the illumination received by reflection from the skin, the instrument 10 develops the coordinates Y, x and y. In Fig. 1a the instrument 10 is a colorimeter, commercially available and suitable for development of the values Y, x and y.

Another type of instrument that can be used in the skin color categorization method according to this invention is the spectrophotometer that measures the skin reflectance at discrete wavelengths and from these data derives tristimulus values, from which can be computed the Hunter color values used to measure skin color for diagnostic purposes as discussed below.

Important to the use of a commercial colorimeter of the kind employed for the color measurement instrument 10 of Fig. 1a is the calibration of the instrument using a standard. In the early use of an instrument of this kind by the inventors, the "Light Skin" sample from the Macbeth Color Checker, described in the publication of C.S. McCamy, H. Marcus, and J.G. Davidson, "A Color-Rendition Chart," J. Appl. Photogr. Eng. 2, 95-99 (1976) was used. A tile of this approximate color was selected for its greater durability as an instrument standard. It was found, however, that the use of the "Light Skin" painted paper as the primary standard did not adequately avoid the phenomenon known as metamerism, by which objects that look alike (have the same perceived color) under some kinds of light sources, or to some observers, do not match under other types of light sources, or to other observers. By this phenomenon colorimeters may not read their colors the same as the average human observer would under the daylight type light source usually employed for visual observation. This could lead to an error in colorimeter calibration.

As an improved primary standard, the skin was selected of a subject whose skin color measurements were highly reproducible, and in the approximate center of the range of skin colors of the human population. The spectral reflectance factors of the skin of this subject were carefully measured on a Macbeth 1500 Plus spectrophotometer (Macbeth, New Windsor, New York); these data are given in column 2 (second from left) of Table I at the wavelengths listed in column 1 (the leftmost column). By using well-established techniques of computer color matching, carried out on an ACS 1800 system equipped with an ACS SpectroSensor II color measuring instrument (Datacolor International, Lawrenceville, New Jersey), a colorant formulation matching this skin color was developed. The spectral reflectance factors for this match are given in column 3 of Table I. It may be seen that the data closely match those of column 2, indicating the absence of metamerism. Calculations according to the CIE 1976 CIELAB system showed that the two data sets match to within 0.27-0.36 units, less than can be perceived by human color vision, for daylight, incandescent light, and cool white fluorescent light, the three most commonly used light sources for the proposed applications.

The above-mentioned formulation was made up in a stable, durable material, and tiles were prepared as instrument standards. The spectral reflectance factors of one of these tiles are given in column 4 of Table I. It was found, however, that the improvement in calibration resulted in color coordinates that were significantly different from those obtained in the many studies made with the earlier system. A decision was made to adjust the calibration values of the new tiles in order to achieve consistent results between the new and old methods of calibration. Column 5 of Table I gives the adjusted set of spectral reflectance factors for the tile of column 4. The CIE and Hunter color coordinates, for measurement with the specular component excluded and calculated for CIE standard illuminant C and the 1931 2° CIE standard observer, are also tabulated for each of the samples in the table.

TABLE I

| Wavelengths, nm. | Skin Standard | Formulation | Tile, correct | Tile, adjusted |
|---|---|---|---|---|
| 400 | 19.03 | 20.70 | 21.51 | 16.67 |
| 420 | 18.96 | 20.69 | 21.10 | 16.93 |
| 440 | 21.53 | 21.68 | 20.99 | 17.65 |
| 460 | 25.36 | 24.43 | 23.27 | 20.56 |
| 480 | 28.06 | 28.30 | 27.82 | 25.67 |
| 500 | 30.13 | 30.77 | 29.03 | 27.94 |
| 520 | 31.19 | 31.31 | 29.38 | 28.24 |
| 540 | 30.01 | 30.84 | 28.48 | 27.59 |
| 560 | 31.41 | 30.76 | 28.22 | 27.33 |
| 580 | 32.85 | 34.01 | 31.49 | 30.12 |
| 600 | 44.37 | 43.54 | 42.58 | 40.52 |
| 620 | 51.24 | 51.57 | 51.27 | 47.93 |
| 640 | 54.56 | 55.09 | 55.56 | 51.10 |
| 660 | 57.09 | 57.60 | 59.22 | 53.82 |
| 680 | 58.67 | 60.41 | 61.82 | 56.55 |
| 700 | 59.95 | 62.69 | 63.93 | 58.87 |
| X | 37.14 | 37.28 | 36.14 | 33.76 |
| Y | 34.66 | 34.89 | 33.07 | 31.53 |
| Z | 28.50 | 28.54 | 27.63 | 24.20 |
| x | 0.3703 | 0.3702 | 0.3732 | 0.3732 |
| y | 0.3456 | 0.3464 | 0.3415 | 0.3523 |
| L | 58.87 | 59.07 | 57.51 | 56.15 |
| a | 9.31 | 9.02 | 11.54 | 9.05 |
| b | 12.51 | 12.70 | 11.77 | 13.75 |

With a suitable standard, basically, calibration is carried out by forcing the colorimeter 10 to give the desired color coordinates Y, x and y mentioned above, while utilizing the colorimeter with the standard tile chosen. The method of calibration is known for particular instruments and follows a series of steps prescribed by the manufacturer that need not be detailed here.

In skin color testing, prior to each test of a subject, each test site is cleansed. A cleansing agent, such as isopropyl alcohol, which leaves behind no coloration, is suitable. The site is well dried to avoid any wetness which may interfere with the reflection of light from the skin 11 to the instrument 10. In all cases of testing, with the instrument correctly calibrated, the measuring head or instrument orifice is placed against the test site to be measured. Care is taken to avoid the admission of ambient light to the instrument. Pressing the head firmly against the test site prevents the entry of ambient light. Additionally, it was determined that best results are obtained if one removes the instrument from the test site briefly, between illuminations. This can be provided for in software by a conventional delaying routine and, if desired, with an appropriate display instructing the user to remove the instrument briefly well away from the skin.

In a colorimeter of the type shown in Fig. 1a, at block 10 the instrument has an internal microprocessor or other computing capability so that it is able to develop the color coordinates Y, x and y from the measured values X, Y and Z (Y being the same in each case). Certain colorimeters develop the Hunter color coordinates L, a, and b. Since the degree of computation that the color measuring device 10 (i.e. colorimeter or spectrophotometer) internally performs varies, the manner of calculating the Hunter values from the tristimulus coordinates is useful to an understanding and practice of the invention and will enable correct use of a CPU by appropriate calculation to perform the invention with any commercially available colorimeter or spectrophotometer. Most modern color measuring instruments begin with measurement of the tristimulus values X, Y, and Z. From these can be derived the CIE chromaticity coordinates x and y:

$$x = X / (X + Y + Z) \tag{1}$$

$$y = Y / (X + Y + Z) \tag{2}$$

The instrument 10 of Fig. 1a outputs the triplet of values x, y and Y as the starting point for further calculations by a central processing unit which can be dedicated microprocessor circuitry or personal computer 15. The remaining two tristimulus values X and Z are available by computation as follows:

$$X = xY/y, \tag{3}$$

and

$$Z = (1-x-y)Y/y \tag{4}$$

In the preferred embodiment, in any event, the CPU according to Fig. 1a develops the Hunter value b discovered in accordance with this invention to be capable of use to detect and monitor hyperbilirubinemia. The Hunter b value is one of three values derived by Richard S. Hunter in 1958. Richard S. Hunter, "Photoelectric Color Difference Meter," J. Opt. Soc. Am. 48, 985-995 (1958). The equations for these are:

$$L = 10 \, (Y)^{1/2} \tag{5}$$

$$a = 17.5 \, (1.02 \, X - Y) \, / \, Y^{1/2} \tag{6}$$

$$b = 7.0 \, (Y - 0.847 \, Z) \, / \, Y^{1/2} \tag{7}$$

where L is a lightness coordinate whose values correlate better with the visual perceptions of the lightness of object colors than do values of Y; a is a coordinate denoting redness or greenness, for which positive values denote that the color is red rather than its opponent color green, and negative values of a denote the opposite; and b is a yellowness-blueness coordinate, for which positive values denote that the color is yellow rather than the opponent color blue, and negative values of b denote the opposite. For yellow colors, starting with a = b = 0 and an appropriate high value of L, which would be a light grey, increasing positive values of b result in a series of colors that may be described as light yellowish grey, pale yellow, light yellow, brilliant yellow and vivid yellow, in turn. Thus b is a measure of the "intensity" of the yellow color.

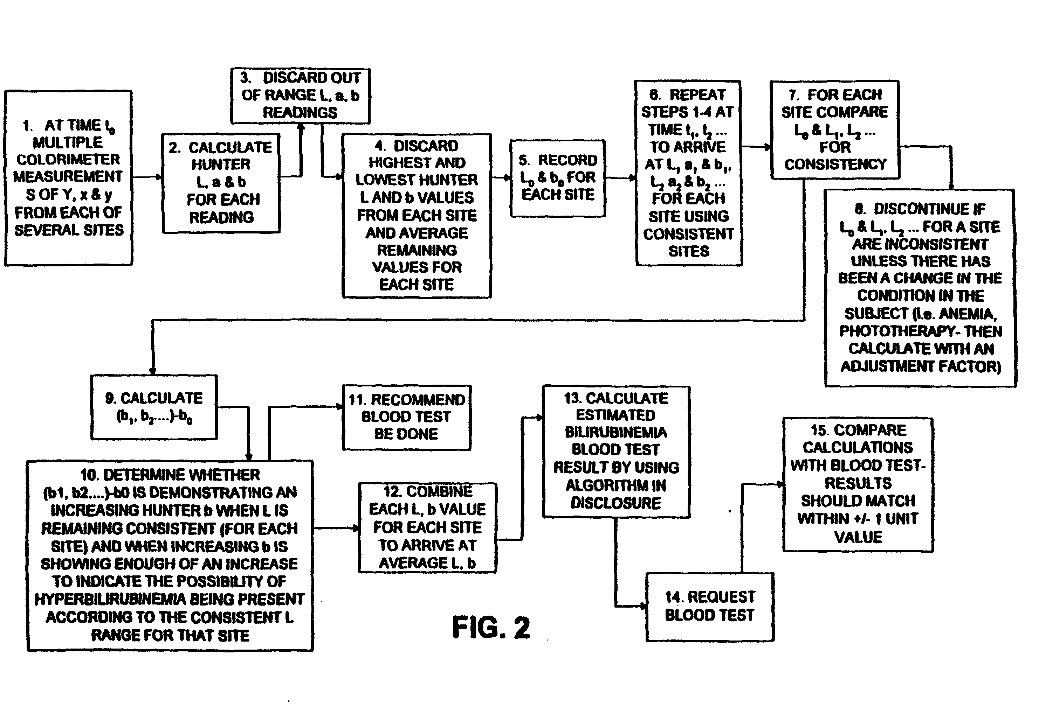

Historically, all three Hunter values, a, b and L, have been utilized to describe a color. The inventors have determined that one can use the Hunter skin lightness measure L and comparative determinations of the Hunter value b developed at time intervals to measure the jaundice that is symptomatic of hyperbilirubinemia and by that measurement of jaundice detect the presence or absence of the ailment. The coordinate b provides a reliable measure of the yellow undertone of the color of human skin. This does not mean that Hunter a and Hunter L should be ignored, but they are not used in the usual way to define a color. In the particular arrangement of Fig. 1a, wherein the colorimeter 10 produces the values Y, x, y, the computer 15 derives the Hunter values L and b. The Hunter lightness skin color characteristic L affects the amount of increase in the yellow measure Hunter b that indicates hyperbilirubinemia. Following the procedure represented in Fig. 2, steps 1 to 4 and preferably using an averaging technique described below, a newborn is measured, preferably within 2-5 or 2-6 hours of birth, to establish the initial, baseline values of Hunter L and b, $L_0$ and $b_0$. A baseline Hunter a, $a_0$, may be calculated at this time, too, for the purposes explained below. At step 3, out of range values are discarded, i.e. values outside the range 20>L>80, 2>a>50 and 2>b>40. At step 4, highest and lowest values of Hunter a and b from each site are discarded. The values are recorded, at step S, for example by placement in machine memory 17. Thereafter, again preferably using the averaging technique described below, throughout the next several days, Hunter L and b (and a, if used) are measured at intervals as represented by step 6 of Fig. 2. L is compared to the value originally measured as indicated at step 7. It should not vary more than 3 to 5 points (depending on the range of L being measured) or the test is discontinued as at step 8. This is so unless there is another explanation. Unless discontinued as explained above, Hunter b is compared at step 9 to the baseline value established shortly after birth. As determined at step 10, if Hunter L remains consistent, if at any time Hunter b increases two points or more for skins with L values at or below approximately 51 or three points or more for skins with L values above approximately 51, then hyperbilirubinemia is indicated, a confirming blood test should be conducted, and phototherapy, the usual treatment for this condition, may be prescribed. Hunter b increases of one to two points for L values at or below approximately 51 and Hunter b increases of two to three points for L values above approximately 51 can be used as red flags or warning signs requiring closer monitoring.

When the measured value of Hunter L at any time is found to have varied more than 3-5 points the test procedure is suspect and the test may be discontinued. Hunter L variations of this magnitude do not ordinarily occur in skin color measurement. Unless this change can be explained by a change in the condition of the subject (such as anemia or phototherapy treatment, which would lighten the subjects entire skin color measurement and range, but in direct ratio of L and b) the test would be suspect. But if the change in Hunter L can be explained as above, then an adjustment factor would be used to calculate L and b.

During phototherapy, then, the testing procedure can be used with an adjustment factor for the lightening of the skin color while under the phototherapy lights.

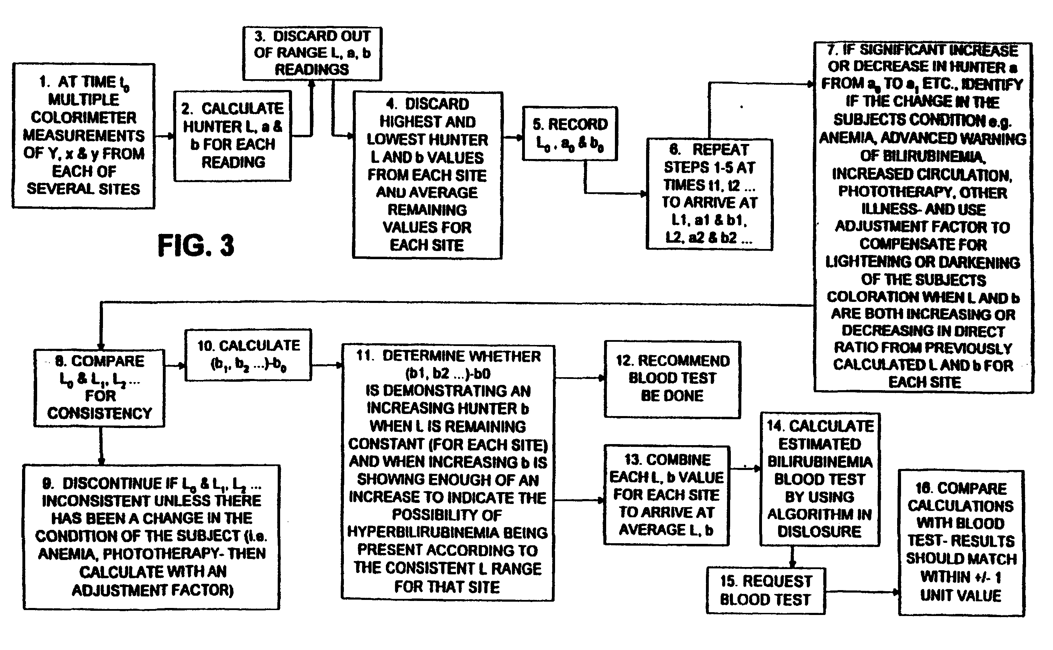

As indicated, it has been the inventors' practice to require the additional measurement of Hunter a at each testing. The best procedure in which Hunter a is utilized is illustrated in Fig. 3. Again the averaging technique is preferably used as described below. Based upon the testing of the skin color of several million individuals, the inventors have identified some 210 broad categories of skin coloration. That is to say, 210 broad ranges of Hunter L, a and b have been identified. Hunter L and b values for each of these categories are shown in Table II, Appendix A hereto. Table III, below, provides the ranges of Hunter a reasonably to be expected. For certain values of L, Hunter a above a particular value has not typically been observed. Should the test indicate a Hunter a outside any previously observed range for a particular L and b, this would be taken as at least a further indication of some disorder in a condition of the subject if the Hunter a value cannot otherwise be accounted for, e.g. from crying or drying after bathing. This occurrence is represented at step 7 of Fig. 3.

The measurement of Hunter a can have the further value of a warning that the jaundice associated with hyperbilirubinemia may shortly occur since, at times Hunter a will increase in value just before Hunter b increases.

If, then, an infant has not been crying (which boosts Hunter a), and an increase in Hunter a is observed, Hunter b bears watching for signs of hyperbilirubinemia.

TABLE III

| If Hunter L values are: | Then Hunter a values are: |
| --- | --- |
| 24 (or less) to 44 | 4 to 16 |
| 45 to 54 | 4 to 18 |
| 55 to 59 | 5 to 25 |
| 60 to 71 (or more) | 6 to 30 |

As mentioned, for greater accuracy an averaging technique is employed. Multiple Y, x and y readings are made with the colorimeter 10 at several different sites. For example, measurements are made at one or more locations on some or all of the subject's forehead, cheek, chest and back, as suggested in the steps of the method outlined in Fig. 2. In a preferred embodiment, 5 or 6 readings at each of 5 different sites are made. Hunter a, b and L values are calculated for each reading. The high and low values of a, b and L from each site are discarded by the computer or computational provisions of the Fig. 1a instrumentation, for example. The instrument or the computer 15 then averages all of the remaining values of Hunter a, b and L for each site. The average a, b and L thus calculated for each site are then used as the Hunter a, b and L values in the previously described testing for hyperbilirubinemia.

Some variation of b value occurs in dependence on the body location where readings are taken. Consistently averaging the values of Hunter a, b and L calculated from measurements taken at the same several locations on each individual can be used to eliminate any uncertainty resulting from such variations. The consistent measurement of consistent sites is essential throughout the entire procedure.

A hospital's measure of serum bilirubin typically uses a scale different from the measure of Hunter b detected by the above procedure. In extensive tests at one hospital, a linear relation was observed between serum bilirubin measured using the hospital's scale and the Hunter b measurement according to the invention. In that hospital, 12 was the serum bilirubin value that signalled monitoring or treatment of hyperbillrubinemia. Steps 12-15 of Fig. 2 and 13-16 of Fig. 3 calculate the serum bilirubin level from the above procedures and compare it to the determination made by blood test.

Correlation between Hunter b and the hospital bilirubin count (BRC) was determined to be in accordance with the following equation:

$$BRC = 2.5 \left([\{ 47/L \}^{1/2} b] - 6.8\right) \tag{8}$$

where BRC equals the hospital bilirubin count, the number 47 is the average L for the entire database gathered over the course of research, and L and b are the average Hunter values determined as described above.

The term in braces modifies b according to the value L relative to its average, in this case 47, according to a square root (superscript 1/2) function. It may be easier to understand the above equation if it is written another way. If the modified b (in square brackets) is called MODB:

$$MODB = 6.8 + 0.4 \, BRC \tag{9}$$

The numbers 6.8 and 0.4 (= 1/2.5) are, respectively, the intercept and slope of the straight line relation between modified b and BRC. The 6.8 is the value of MODB when BRC = 0 and is related to the average baseline skin color. The 0.4 shows how rapidly MODB changes as BRC increases, an increase of 2.5 in BRC raises MODB by one point.

The equation is exemplary only and may vary in detail when applied to a larger database or to bilirubin count values from another hospital since hospitals do not have a standard scale used consistently from one hospital to the next. However, the linear relationship between MODB and BRC indicates relatively straightforward conversion of measured L and b to arrive at a particular hospital's bilirubin count value so that the medical practitioner can employ the optical measurement of jaundice in accordance with this invention in the same way she or he employed bilirubin count previously.

In the system of Fig. 1a, following the routine of Fig. 2, from the initial measurement preferably within 2-5 or 2-6 hours of birth, the CPU calculates the initial Hunter values $L_0$, $a_0$ and $b_0$ and stores these in the Baseline Values addresses of the data portion or RAM of memory 17. The data RAM (or nonprogram) portion 18 of the memory 17 is indicated in Fig. 1b. A relatively permanent section 18a of RAM 18 stores the data of Table II (and Table III if Hunter a is to be checked) and data such as the ranges of L that establish categories of skin coloration for which varying Hunter b value changes are significant. A more often revised memory segment stores the results of the measurements performed with the instrument. Based on a relatively straightforward program retained in the permanent ROM memory, from the measurements taken at intervals, the CPU calculates the new values of L, a, and b (or L and b to follow the procedure of Fig. 2), retrieves $L_0$, $a_0$ and $b_0$, and subtracts those from the new values of $L_1$, $a_1$ and $b_1$. The change in Hunter L, a and b, $\Delta L$, $\Delta a$ and $\Delta b$, can be displayed, or preferably, the CPU determines if the change in L indicates an error by comparing the change in L to that value, stored in the RAM 18 of the memory 17, that raises the suspicion of test error. If there is no suspicion of error, then the CPU determines whether an increase in b is above the value, again stored in memory, that indicates monitoring or treatment of hyperbilirubinemia for the particular value of L that has been measured. Similarly, for an infant that has previously been diagnosed with hyperbilirubinemia and is undergoing phototherapy, the same order of decrease to within 2 or 3 points of baseline, depending on L, can indicate recovery and phototherapy may be ended. The CPU memory 17 can be provided with Table II, or another compilation of the categories of skin coloration, which the CPU then can use as a look-up table to determine if Hunter a has a value outside of previously observed ranges for the particular Hunter L and b. Also, if desired, the CPU can calculate and display the hospital's measure of serum bilirubin based upon changes in Hunter b, for example by applying equation 8 above.

Even in the absence of an initial reading, based on observed ranges of skin coloration, measurement of either Hunter L and b or L, a and b can warn of the likelihood of hyperbilirubinemia if a Hunter b value is measured that is in excess of Hunter b ordinarily observed for subjects with that value of L. Hunter b values exceeding those ordinarily observed for individuals in a particular range of Hunter L values can be determined by reference to Table II. For example, it will be apparent that no individual whose skin has a Hunter L value between 24 and 26 has measured above 13 in Hunter b. Such a measurement may be used to determine that a blood test is advisable. In all instances, however, even where there has not been a Hunter b baseline established, an increase over time of 2, 3 or more Hunter b points indicates the likelihood of hyperbilirubinemia, and if the change is a decrease, this is indicative of a recovering newborn.

Table IV is an actual set of measurements made on a three day old infant. Using the averaging technique described above, Hunter L of 48.0 and Hunter b of 11.1 is calculated. Converting to the hospital bilirubin count in the equation (9) above, a bilirubin count of 10.5 was calculated.

## TABLE IV

|          | L    | a    | b    | Y    | x     | y     |
|----------|------|------|------|------|-------|-------|
| Forehead | 47.8 | 21.6 | 11.6 | 22.9 | 0.411 | 0.333 |
|          | 48.6 | 19.5 | 11.5 | 23.6 | 0.404 | 0.335 |
|          | 48.8 | 21.2 | 11.6 | 23.8 | 0.407 | 0.333 |
|          | 46.7 | 21.6 | 11.6 | 21.8 | 0.413 | 0.333 |
|          | 48.6 | 21.6 | 11.8 | 23.6 | 0.410 | 0.333 |
|          | 48.0 | 22.1 | 11.7 | 23.1 | 0.412 | 0.332 |
| Forehead | 46.4 | 20.5 | 11.2 | 21.5 | 0.409 | 0.333 |
|          | 46.0 | 20.3 | 11.1 | 21.1 | 0.409 | 0.333 |
|          | 47.4 | 21.4 | 11.6 | 22.4 | 0.411 | 0.333 |
|          | 46.1 | 21.4 | 10.7 | 21.2 | 0.409 | 0.330 |
|          | 46.3 | 20.4 | 11.2 | 21.5 | 0.409 | 0.333 |
|          | 46.9 | 20.7 | 11.3 | 22.0 | 0.409 | 0.333 |
| Chest    | 50.5 | 16.5 | 11.2 | 25.5 | 0.391 | 0.336 |
|          | 50.9 | 15.3 | 11.2 | 25.9 | 0.388 | 0.338 |
|          | 50.1 | 17.5 | 11.2 | 25.1 | 0.395 | 0.336 |
|          | 50.7 | 16.9 | 11.2 | 25.7 | 0.392 | 0.336 |
|          | 50.4 | 16.4 | 11.1 | 25.4 | 0.391 | 0.336 |
|          | 50.1 | 17.3 | 11.1 | 25.1 | 0.394 | 0.335 |
| Back     | 49.0 | 17.1 | 11.1 | 24.0 | 0.395 | 0.336 |
|          | 48.7 | 16.3 | 11.0 | 23.7 | 0.394 | 0.337 |
|          | 48.3 | 16.6 | 10.6 | 23.3 | 0.393 | 0.335 |
|          | 49.2 | 16.6 | 10.9 | 24.2 | 0.393 | 0.336 |
|          | 49.1 | 18.3 | 11.3 | 24.1 | 0.399 | 0.335 |
|          | 50.0 | 18.0 | 11.4 | 25.0 | 0.397 | 0.336 |

|  | L | a | b | Y | x | y |
|---|---|---|---|---|---|---|
| Back | 46.2 | 15.8 | 10.5 | 21.4 | 0.395 | 0.337 |
|  | 45.3 | 16.5 | 10.2 | 20.5 | 0.397 | 0.335 |
|  | 45.9 | 16.0 | 10.4 | 21.1 | 0.395 | 0.336 |
|  | 45.5 | 14.4 | 10.3 | 20.7 | 0.392 | 0.338 |
|  | 46.3 | 16.1 | 11.0 | 21.4 | 0.398 | 0.339 |
|  | 47.3 | 16.9 | 10.9 | 22.3 | 0.397 | 0.336 |

Good evidence of jaundice resulting from medical conditions other than hyperbilirubinemia can be made. Liver disorders in adults and children produce jaundice, for example. These and other skin color characteristics can be factors in diagnosing additional diseases that affect skin color. It has been observed, for example, that at least among dark skinned individuals, such as African Americans or others of African descent, skin color is affected by tuberculosis.

The method and apparatus is not limited to the jaundice-related testing described above. Experiments with rhesus monkeys have shown a correlation between hormone levels and the coloration of the female monkey's very visible reddened hind end. An instrument like that described above was to distinguish varying levels of reddening in an individual test subject's posterior using Hunter a and Hunter L in a similar fashion to that described above. The hormone level of the subject was thus indicated by the methods and apparatus of this invention.

Successful experimentation has begun on the evaluation of the condition of laboratory mice based upon the use of Hunter a and Hunter L in a similar fashion to that described above.

Table V, Appendix B, is a broad categorization of human hair coloration.

APPENDIX A

## TABLE II

| No. | Hunter L | Hunter b | No. | Hunter L | Hunter b |
|-----|----------|----------|-----|----------|----------|
| 1. | <27 | -5* | 33. | 36 to <39 | -5 |
| 2. | <27 | 6 | 34. | 36 to <39 | 6 |
| 3. | <27 | 7 | 35. | 36 to <39 | 7 |
| 4. | <27 | 8 | 36. | 36 to <39 | 8 |
| 5. | <27 | 9 | 37. | 36 to <39 | 9 |
| 6. | <27 | 10 | 38. | 36 to <39 | 10 |
| 7. | <27 | 11 | 39. | 36 to <39 | 11 |
| 8. | <27 | 12+** | 40. | 36 to <39 | 12 |
| 9. | 27 to <30 | -5 | 41. | 36 to <39 | 13 |
| 10. | 27 to <30 | 6 | 42. | 36 to <39 | 14 |
| 11. | 27 to <30 | 7 | 43. | 36 to <39 | 15+ |
| 12. | 27 to <30 | 8 | 44. | 39 to <42 | -5 |
| 13. | 27 to <30 | 9 | 45. | 39 to <42 | 6 |
| 14. | 27 to <30 | 10 | 46. | 39 to <42 | 7 |
| 15. | 27 to <30 | 11 | 47. | 39 to <42 | 8 |
| 16. | 27 to <30 | 12+ | 48. | 39 to <42 | 9 |
| 17. | 30 to <33 | -5 | 49. | 39 to <42 | 10 |
| 18. | 30 to <33 | 6 | 50. | 39 to <42 | 11 |
| 19. | 30 to <33 | 7 | 51. | 39 to <42 | 12 |
| 20. | 30 to <33 | 8 | 52. | 39 to <42 | 13 |
| 21. | 30 to <33 | 9 | 53. | 39 to <42 | 14 |
| 22. | 30 to <33 | 10 | 54. | 39 to <42 | 15+ |
| 23. | 30 to <33 | 11 | 55. | 42 to <45 | -5 |
| 24. | 30 to <33 | 12+ | 56. | 42 to <45 | 6 |
| 25. | 33 to <36 | -5 | 57. | 42 to <45 | 7 |
| 26. | 33 to <36 | 6 | 58. | 42 to <45 | 8 |
| 27. | 33 to <36 | 7 | 59. | 42 to <45 | 9 |
| 28. | 33 to <36 | 8 | 60. | 42 to <45 | 10 |
| 29. | 33 to <36 | 9 | 61. | 42 to <45 | 11 |
| 30. | 33 to <36 | 10 | 62. | 42 to <45 | 12 |
| 31. | 33 to <36 | 11 | 63. | 42 to <45 | 13 |
| 32. | 33 to <36 | 12+ | 64. | 42 to <45 | 14 |

\* The designation -5 means less than 5 but more than 4.
\*\* The designation 12+ means more than 12 but less than 13.

| No. | Hunter L | Hunter b | No. | Hunter L | Hunter b |
|-----|----------|----------|-----|----------|----------|
| 65. | 42 to <45 | 15 | 102. | 51 to <54 | 8 |
| 66. | 42 to <45 | 16 | 103. | 51 to <54 | 9 |
| 67. | 42 to <45 | 17 | 104. | 51 to <54 | 10 |
| 68. | 42 to <45 | 18+ | 105. | 51 to <54 | 11 |
| 69. | 45 to <48 | -5 | 106. | 51 to <54 | 12 |
| 70. | 45 to <48 | 6 | 107. | 51 to <54 | 13 |
| 71. | 45 to <48 | 7 | 108. | 51 to <54 | 14 |
| 72. | 45 to <48 | 8 | 109. | 51 to <54 | 15 |
| 73. | 45 to <48 | 9 | 110. | 51 to <54 | 16 |
| 74. | 45 to <48 | 10 | 111. | 51 to <54 | 17 |
| 75. | 45 to <48 | 11 | 112. | 51 to <54 | 18 |
| 76. | 45 to <48 | 12 | 113. | 51 to <54 | 19 |
| 77. | 45 to <48 | 13 | 114. | 51 to <54 | 20+ |
| 78. | 45 to <48 | 14 | 115. | 54 to <57 | -5 |
| 79. | 45 to <48 | 15 | 116. | 54 to <57 | 6 |
| 80. | 45 to <48 | 16 | 117. | 54 to <57 | 7 |
| 81. | 45 to <48 | 17 | 118. | 54 to <57 | 8 |
| 82. | 45 to <48 | 18+ | 119. | 54 to <57 | 9 |
| 83. | 48 to <51 | -5 | 120. | 54 to <57 | 10 |
| 84. | 48 to <51 | 6 | 121. | 54 to <57 | 11 |
| 85. | 48 to <51 | 7 | 122. | 54 to <57 | 12 |
| 86. | 48 to <51 | 8 | 123. | 54 to <57 | 13 |
| 87. | 48 to <51 | 9 | 124. | 54 to <57 | 14 |
| 88. | 48 to <51 | 10 | 125. | 54 to <57 | 15 |
| 89. | 48 to <51 | 11 | 126. | 54 to <57 | 16 |
| 90. | 48 to <51 | 12 | 127. | 54 to <57 | 17 |
| 91. | 48 to <51 | 13 | 128. | 54 to <57 | 18 |
| 92. | 48 to <51 | 14 | 129. | 54 to <57 | 19 |
| 93. | 48 to <51 | 15 | 130. | 54 to <57 | 20+ |
| 94. | 48 to <51 | 16 | 131. | 57 to <60 | -5 |
| 95. | 48 to <51 | 17 | 132. | 57 to <60 | 6 |
| 96. | 48 to <51 | 18 | 133. | 57 to <60 | 7 |
| 97. | 48 to <51 | 19 | 134. | 57 to <60 | 8 |
| 98. | 48 to <51 | 20+ | 135. | 57 to <60 | 9 |
| 99. | 51 to <54 | -5 | 136. | 57 to <60 | 10 |
| 100. | 51 to <54 | 6 | 137. | 57 to <60 | 11 |
| 101. | 51 to <54 | 7 | 138. | 57 to <60 | 12 |

| No. | Hunter L | Hunter b | No. | Hunter L | Hunter b |
|---|---|---|---|---|---|
| 139. | 57 to <60 | 13 | 175. | 63 to <66 | 17 |
| 140. | 57 to <60 | 14 | 176. | 63 to <66 | 18 |
| 141. | 57 to <60 | 15 | 177. | 63 to <66 | 19 |
| 142. | 57 to <60 | 16 | 178. | 63 to <66 | 20+ |
| 143. | 57 to <60 | 17 | 179. | 66 to <69 | -5 |
| 144. | 57 to <60 | 18 | 180. | 66 to <69 | 6 |
| 145. | 57 to <60 | 19 | 181. | 66 to <69 | 7 |
| 146. | 57 to <60 | 20+ | 182. | 66 to <69 | 8 |
| 147. | 60 to <63 | -5 | 183. | 66 to <69 | 9 |
| 148. | 60 to <63 | 6 | 184. | 66 to <69 | 10 |
| 149. | 60 to <63 | 7 | 185. | 66 to <69 | 11 |
| 150. | 60 to <63 | 8 | 186. | 66 to <69 | 12 |
| 151. | 60 to <63 | 9 | 187. | 66 to <69 | 13 |
| 152. | 60 to <63 | 10 | 188. | 66 to <69 | 14 |
| 153. | 60 to <63 | 11 | 189. | 66 to <69 | 15 |
| 154. | 60 to <63 | 12 | 190. | 66 to <69 | 16 |
| 155. | 60 to <63 | 13 | 191. | 66 to <69 | 17 |
| 156. | 60 to <63 | 14 | 192. | 66 to <69 | 18 |
| 157. | 60 to <63 | 15 | 193. | 66 to <69 | 19 |
| 158. | 60 to <63 | 16 | 194. | 66 to <69 | 20+ |
| 159. | 60 to <63 | 17 | 195. | ≥69 | -5 |
| 160. | 60 to <63 | 18 | 196. | ≥69 | 6 |
| 161. | 60 to <63 | 19 | 197. | ≥69 | 7 |
| 162. | 60 to <63 | 20+ | 198. | ≥69 | 8 |
| 163. | 63 to <66 | -5 | 199. | ≥69 | 9 |
| 164. | 63 to <66 | 6 | 200. | ≥69 | 10 |
| 165. | 63 to <66 | 7 | 201. | ≥69 | 11 |
| 166. | 63 to <66 | 8 | 202. | ≥69 | 12 |
| 167. | 63 to <66 | 9 | 203. | ≥69 | 13 |
| 168. | 63 to <66 | 10 | 204. | ≥69 | 14 |
| 169. | 63 to <66 | 11 | 205. | ≥69 | 15 |
| 170. | 63 to <66 | 12 | 206. | ≥69 | 16 |
| 171. | 63 to <66 | 13 | 207. | ≥69 | 17 |
| 172. | 63 to <66 | 14 | 208. | ≥69 | 18 |
| 173. | 63 to <66 | 15 | 209. | ≥69 | 19 |
| 174. | 63 to <66 | 16 | 210. | ≥69 | 20+ |

APPENDIX B

TABLE V

| CATEGORY NAME | L | | a | | b | |
|---|---|---|---|---|---|---|
| | Min | Max | Min | Max | Min | Max |
| Black | 0.00 | 14.00 | -10.00 | 3.00 | -10.00 | 5.00 |
| Darkest Dark Brown | 14.00 | 16.00 | -10.00 | 3.00 | -10.00 | 1.00 |
| Darkest Dark Brown | 14.00 | 16.00 | -10.00 | 3.00 | 1.00 | 1.15 |
| Darkest Dark Brown | 14.00 | 16.00 | -10.00 | 3.00 | 1.15 | 1.25 |
| Darkest Dark Brown | 14.00 | 16.00 | -10.00 | 3.00 | 1.25 | 3.00 |
| Darker Dark Brown | 16.00 | 19.00 | -10.00 | 3.00 | -10.00 | 2.70 |
| Darker Dark Brown | 16.00 | 19.00 | -10.00 | 3.00 | 2.70 | 2.95 |
| Darker Dark Brown | 16.00 | 19.00 | -10.00 | 3.00 | 2.95 | 3.20 |
| Darker Dark Brown | 16.00 | 19.00 | -10.00 | 3.00 | 3.20 | 10.00 |
| Darker Dark Brown (Cool Auburn Tones) | 16.00 | 19.00 | 2.00 | 3.00 | -10.00 | 2.70 |
| Darker Dark Brown (Warm Auburn Tones) | 16.00 | 19.00 | 2.00 | 3.00 | 3.20 | 10.00 |
| Brown | 19.00 | 22.00 | 0.00 | 6.00 | -10.00 | 2.95 |
| Brown | 19.00 | 22.00 | 0.00 | 6.00 | 2.95 | 3.20 |
| Brown | 19.00 | 22.00 | 0.00 | 6.00 | 3.20 | 3.45 |
| Brown | 19.00 | 22.00 | 0.00 | 6.00 | 3.45 | 10.00 |
| Brown (Warm Auburn Tones) | 19.00 | 22.00 | 3.50 | 6.00 | 3.45 | 10.00 |
| Brown (Cool Auburn Tones) | 19.00 | 22.00 | 3.50 | 6.00 | -10.00 | 3.45 |
| Medium Brown | 22.00 | 27.00 | 1.00 | 6.00 | -10.00 | 3.75 |
| Medium Brown | 22.00 | 27.00 | 1.00 | 6.00 | 3.75 | 4.00 |
| Golden Med. Brown | 22.00 | 27.00 | 1.00 | 6.00 | 4.00 | 4.25 |
| Golden Med. Brown | 22.00 | 27.00 | 1.00 | 6.00 | 4.25 | 10.00 |
| Medium Brown (Warm Auburn Tones) | 22.00 | 27.00 | 3.50 | 6.00 | 4.25 | 10.00 |
| Medium Brown (Cool Auburn Tones) | 22.00 | 27.00 | 3.50 | 6.00 | -10.00 | 4.25 |
| Darkest Med. Blonde | 27.00 | 28.00 | 1.80 | 6.00 | -5.00 | 6.00 |
| Darkest Med. Blonde | 27.00 | 28.00 | 1.80 | 5.00 | 6.00 | 6.50 |
| Darkest Med. Blonde | 27.00 | 28.00 | 5.00 | 6.00 | 6.00 | 6.50 |
| Darkest Med. Blonde | 27.00 | 28.00 | 1.80 | 6.00 | 6.50 | 15.00 |
| Medium Blonde | 28.00 | 31.00 | 1.80 | 6.00 | -5.00 | 6.00 |
| Medium Blonde | 28.00 | 31.00 | 1.80 | 5.00 | 6.00 | 6.50 |
| Med. Golden Blonde | 28.00 | 31.00 | 5.00 | 6.00 | 6.00 | 6.50 |

TABLE V   (continued)

| CATEGORY NAME | L | | a | | b | |
|---|---|---|---|---|---|---|
| | Min | Max | Min | Max | Min | Max |
| Med. Golden Blonde | 28.00 | 31.00 | 1.80 | 6.00 | 6.50 | 15.00 |
| Lightest Med. Blonde | 31.00 | 33.00 | 1.80 | 6.00 | -5.00 | 6.00 |
| Lightest Med. Blonde | 31.00 | 33.00 | 1.80 | 5.00 | 6.00 | 6.50 |
| Lightest Med. Blonde | 31.00 | 33.00 | 5.00 | 6.00 | 6.00 | 6.50 |
| Lightest Med. Blonde | 31.00 | 33.00 | 1.80 | 6.00 | 6.50 | 15.00 |
| Light Blonde | 33.00 | 36.00 | 1.80 | 6.00 | -5.00 | 7.00 |
| Light Blonde | 33.00 | 36.00 | 1.80 | 5.00 | 7.00 | 7.50 |
| Light Blonde | 33.00 | 36.00 | 5.00 | 6.00 | 7.00 | 7.50 |
| Light Blonde | 33.00 | 36.00 | 1.80 | 6.00 | 7.50 | 20.00 |
| Lighter Blonde | 36.00 | 40.00 | 1.80 | 6.00 | -5.00 | 8.00 |
| Lighter Blonde | 36.00 | 40.00 | 1.80 | 5.00 | 8.00 | 8.50 |
| Lighter Blonde | 36.00 | 40.00 | 5.00 | 6.00 | 8.00 | 8.50 |
| Lighter Blonde | 36.00 | 40.00 | 1.80 | 6.00 | 8.50 | 20.00 |
| Lightest Blonde | 40.00 | 80.00 | 1.80 | 7.00 | -5.00 | 9.00 |
| Lightest Blonde | 40.00 | 80.00 | 1.80 | 5.00 | 9.00 | 10.00 |
| Lightest Blonde | 40.00 | 80.00 | 5.00 | 7.00 | 9.00 | 10.00 |
| Lightest Blonde | 40.00 | 80.00 | 1.80 | 7.00 | 10.00 | 30.00 |
| Light Red | 22.00 | 28.00 | 6.00 | 30.00 | -5.00 | 3.50 |
| Light Red | 22.00 | 28.00 | 6.00 | 30.00 | 3.50 | 3.75 |
| Light Red | 22.00 | 28.00 | 6.00 | 30.00 | 3.75 | 4.00 |
| Light Red | 22.00 | 28.00 | 6.00 | 30.00 | 4.00 | 30.00 |
| Medium Red | 19.00 | 22.00 | 6.00 | 30.00 | -10.00 | 3.50 |
| Medium Red | 19.00 | 22.00 | 6.00 | 30.00 | 3.50 | 3.75 |
| Med. Golden Red | 19.00 | 22.00 | 6.00 | 30.00 | 3.75 | 4.00 |
| Med. Golden Red | 19.00 | 22.00 | 6.00 | 30.00 | 4.00 | 30.00 |
| Dark Red | 14.00 | 19.00 | 3.00 | 30.00 | -10.00 | 2.50 |
| Dark Red | 14.00 | 19.00 | 3.00 | 30.00 | 2.50 | 2.75 |
| Dark Red | 14.00 | 19.00 | 3.00 | 30.00 | 2.75 | 3.00 |
| Dark Red | 14.00 | 19.00 | 3.00 | 30.00 | 3.00 | 30.00 |
| Red Blonde | 27.00 | 40.00 | 6.00 | 30.00 | 6.00 | 30.00 |
| Red Blonde | 40.00 | 80.00 | 7.00 | 30.00 | 6.00 | 30.00 |
| CATEGORY NAME | L | | a | | b | |
| | Min | Max | Min | Max | Min | Max |
| Black/Dk Brown/Med Brown/Brown w/ 70% - 90% Grey | 27.00 | 50.00 | -10.00 | 1.80 | -10.00 | 3.75 |

TABLE V   (continued)

| CATEGORY NAME | L | | a | | b | |
|---|---|---|---|---|---|---|
| | Min | Max | Min | Max | Min | Max |
| Black/Dk grown/Med Brown/Brown w/ 70% - 90% Grey | 27.00 | 50.00 | -10.00 | 1.80 | 3.75 | 4.00 |
| Black/Dk Brown/Med Brown/Brown w/ 70% - 90% Grey | 27.00 | 50.00 | -10.00 | 1.80 | 4.00 | 4.25 |
| Black/Dk Brown/Med Brown/Brown w/ 70% - 90% Grey | 27.00 | 50.00 | -10.00 | 1.80 | 4.25 | 10.00 |
| Black/Dk Brown/Med Brown/Brown w/ 40% - 60% Grey | 23.00 | 27.00 | -10.00 | 1.00 | -10.00 | 3.75 |
| Black/Dk Brown/Med Brown/Brown w/ 40% - 60% Grey | 23.00 | 27.00 | -10.00 | 1.00 | 3.75 | 4.00 |
| Black/Dk Brown/Med Brown/Brown w/ 40% - 60% Grey | 23.00 | 27.00 | -10.00 | 1.00 | 4.00 | 4.25 |
| Black/Dk Brown/Med Brown/Brown w/ 40% - 60% Grey | 23.00 | 27.00 | -10.00 | 1.00 | 4.25 | 10.00 |
| Grey Hair Program | | | | | | |
| Light Brown/Darkest Blonde | | | | | | |
| 40% - 60% Grey | 4.00 | 10.00 | -10.00 | -0.08 | | |
| 70% - 90% Grey | 10.00 | 999.00 | -10.00 | -0.08 | | |
| Dark Red, Medium Red or Medium Light Red | | | | | | |
| 40% - 60% Grey | 6.00 | 10.00 | -10.00 | -0.80 | | |
| 70% - 90% Grey | 10.00 | 999.00 | -10.00 -10.00 | -0.80 | | |
| Light Red or Red Blonde | | | | | | |
| 40% - 60% Grey | 5.00 | 7.00 | -10.00 | -0.80 | | |
| 70% - 90% Grey | 7.00 | 999.00 | -10.00 | -0.80 | | |
| Medium to Medium Dark Blonde | | | | | | |
| 40% - 60% Grey | 1.70 | 4.00 | 0.00 | 0.00 | | |
| 70% - 90% Grey | 4.00 | 999.00 | 0.00 | 0.00 | | |
| Light Blonde Hair | | | | | | |
| 40% - 60% Grey | -99.99 | -0.25 | -1.75 | -1.25 | | |
| 70% - 90% Grey | -99.99 | -0.25 | -99.99 | -1.75 | | |
| * Negative values are used in this table in their ordinary sense, to denote values less than zero. | | | | | | |

**Claims**

1.   A process for forming a compilation of colour factor values indicative of a medical or non-cosmetic physiological condition of a human or animal test subject, which condition is associated with a symptomatic, detectable skin coloration, the process comprising the steps of:

(a) compiling a group of lightness measure value ranges, and,

(b) associating with each lightness measure value range a value or range of values of a colour factor that indicates the condition for a test subject having a measured lightness within the lightness measure value range for use in comparison with a measurement of the value of that colour factor in the coloration of a test subject

2.  The process according to claim 1, wherein the condition is a condition that causes jaundice in a human child or adult test subject.

3.  The process according to claim 1, wherein the condition is one of hyperbilirubinemia, hypertension, tuberculosis or liver disorder.

4.  The process according to any one of the preceding claim, wherein the test subject is a human infant.

5.  The process according to any one of the preceding claims, wherein step (a) comprises assembling the group of lightness measure value ranges in computer processable form, and step (b) comprises associating the colour factor value with each lightness measure value range in said computer processable form.

6.  The process according to any one of the preceding claims, wherein step (b) comprises associating a value of colour factor Hunter b with each lightness measure value range.

7.  The process according to any one of the preceding claims, wherein step (a) comprises compiling a group of values of lightness measure Hunter L.

8.  The process according to any one of Claims 1 to 5, wherein step (a) comprises compiling a group of lightness measure colour factor value ranges that are substantially those of colour factor Hunter L, and step (b) comprises associating a value of a colour factor whose value is substantially that of colour factor Hunter a with each lightness measure colour factor value range, the relationship between said lightness measure colour factor value ranges and said associated colour factor values being substantially as follows:

| Hunter L | Hunter a |
|---|---|
| 24 (or less) to 44 | 4 to 16 |
| 45 to 54 to | 18 |
| 55 to 59 to | 25 |
| 60 to 71 (or more) | 6 to 30. |

9.  The process according to any one of Claims 1 to 5, wherein step (b) comprises associating with each lightness measure value range a value of a yellowness-dependent colour factor.

10. The process according to any one of Claims 1 to 7, wherein the colour factor of step (b) is Hunter b and the lightness measure value ranges of step (a) are ranges of Hunter L, and in which a determination of the condition may include the step of measuring by instrument a Hunter L value of the test subject's skin coloration.

11. The process according to any one of Claims 1 to 7, wherein said ranges of values of step (a) are determined by ranges of Hunter L values; said ranges of Hunter L values being bounded by at least one of the Hunter L values substantially as follows:
Hunter L = 27, 30, 33, 36, 39, 42, 45, 48, 51, 54, 57, 60, 63, 66 and 69.

12. The process according to any one of Claims 1 to 7, wherein the condition is hyperbilirubinemia, step (a) comprises compiling ranges that are substantially ranges of values of Hunter L; and step (b) comprises associating a range of values substantially those of Hunter b with each lightness measure value range; the condition in the test subject being determined by:

(c) measuring with a colour measuring instrument a value of a first colour factor substantially that of Hunter b in the skin coloration of the test subject;

(d) measuring with a colour measuring instrument a value of a second colour factor substantially that of Hunter L in said skin coloration of the test subject;

(e) comparing the measured second colour factor value with the ranges compiled in step (a) to locate a comparable lightness in the coloration of the test subject and the compiled ranges; and

(f) comparing the measured first colour factor value with a first colour factor value range associated in step (b) with the comparable compiled lightness measure value range to determine if the measured value of the first colour factor lies inside or outside the associated range of first colour factor values; wherein said associated range of first colour factor values is selected from ranges substantially as follows:

| No. | Hunter L | Hunter b | | No. | Hunter L | Hunter b |
|-----|----------|----------|---|-----|----------|----------|
| 1. | <27 | -5 | | 33. | 36 to <39 | -5 |
| 2. | <27 | 6 | | 34. | 36 to <39 | 6 |
| 3. | <27 | 7 | | 35. | 36 to <39 | 7 |
| 4. | <27 | 8 | | 36. | 36 to <39 | 8 |
| 5. | <27 | 9 | | 37. | 36 to <39 | 9 |
| 6. | <27 | 10 | | 38. | 36 to <39 | 10 |
| 7. | <27 | 11 | | 39. | 36 to <39 | 11 |
| 8. | <27 | 12+ | | 40. | 36 to <39 | 12 |
| 9. | 27 to <30 | -5 | | 41. | 36 to <39 | 13 |
| 10. | 27 to <30 | 6 | | 42. | 36 to <39 | 14 |
| 11. | 27 to <30 | 7 | | 43. | 36 to <39 | 15+ |
| 12. | 27 to <30 | 8 | | 44. | 39 to <42 | -5 |
| 13. | 27 to <30 | 9 | | 45. | 39 to <42 | 6 |
| 14. | 27 to <30 | 10 | | 46. | 39 to <42 | 7 |
| 15. | 27 to <30 | 11 | | 47. | 39 to <42 | 8 |
| 16. | 27 to <30 | 12+ | | 48. | 39 to <42 | 9 |
| 17. | 30 to <33 | -5 | | 49. | 39 to <42 | 10 |
| 18. | 30 to <33 | 6 | | 50. | 39 to <42 | 11 |
| 19. | 30 to <33 | 7 | | 51. | 39 to <42 | 12 |
| 20. | 30 to <33 | 8 | | 52. | 39 to <42 | 13 |
| 21. | 30 to <33 | 9 | | 53. | 39 to <42 | 14 |
| 22. | 30 to <33 | 10 | | 54. | 39 to <42 | 15+ |
| 23. | 30 to <33 | 11 | | 55. | 42 to <45 | -5 |
| 24. | 30 to <33 | 12+ | | 56. | 42 to <45 | 6 |
| 25. | 33 to <36 | -5 | | 57. | 42 to <45 | 7 |
| 26. | 33 to <36 | 6 | | 58. | 42 to <45 | 8 |
| 27. | 33 to <36 | 7 | | 59. | 42 to <45 | 9 |
| 28. | 33 to <36 | 8 | | 60. | 42 to <45 | 10. |
| 29. | 33 to <36 | 9 | | 61. | 42 to <45 | 11. |
| 30. | 33 to <36 | 10 | | 62. | 42 to <45 | 12 |
| 31. | 33 to <36 | 11 | | 63. | 42 to <45 | 13 |
| 32. | 33 to <36 | 12+ | | 64. | 42 to <45 | 14 |

| No. | Hunter L | Hunter b | No. | Hunter L | Hunter b |
|---|---|---|---|---|---|
| 55. | 42 to <45 | 15 | 102. | 51 to <54 | 8 |
| 56. | 42 to <45 | 16 | 103. | 51 to <54 | 9 |
| 57. | 42 to <45 | 17 | 104. | 51 to <54 | 10 |
| 68. | 42 to <45 | 19+ | 105. | 51 to <54 | 11 |
| 69. | 45 to <48 | -5 | 106. | 51 to <54 | 12 |
| 70. | 45 to <48 | 6 | 107. | 51 to <54 | 13 |
| 71. | 45 to <48 | 7 | 108. | 51 to <54 | 14 |
| 72. | 45 to <48 | 8 | 109. | 51 to <54 | 15 |
| 73. | 45 to <48 | 9 | 110. | 51 to <54 | 16 |
| 74. | 45 to <48 | 10 | 111. | 51 to <54 | 17 |
| 75. | 45 to <48 | 11 | 112. | 51 to <54 | 18 |
| 76. | 45 to <48 | 12 | 113. | 51 to <54 | 19 |
| 77. | 45 to <48 | 13 | 114. | 51 to <54 | 20+ |
| 78. | 45 to <48 | 14 | 115. | 54 to <57 | -5 |
| 79. | 45 to <48 | 15 | 116. | 54 to <57 | 6 |
| 80. | 45 to <48 | 16 | 117. | 54 to <57 | 7 |
| 81. | 45 to <48 | 17 | 118. | 54 to <57 | 8 |
| 82. | 45 to <48 | 18+ | 119. | 54 to <57 | 9 |
| 83. | 48 to <51 | -5 | 120. | 54 to <57 | 10 |
| 84. | 48 to <51 | 6 | 121. | 54 to <57 | 11 |
| 85. | 48 to <51 | 7 | 122. | 54 to <57 | 12 |
| 86. | 48 to <51 | 8 | 123. | 54 to <57 | 13 |
| 87. | 48 to <51 | 9 | 124. | 54 to <57 | 14 |
| 88. | 48 to <51 | 10 | 125. | 54 to <57 | 15 |
| 89. | 48 to <51 | 11 | 126. | 54 to <57 | 16 |
| 90. | 48 to <51 | 12 | 127. | 54 to <57 | 17 |
| 91. | 48 to <51 | 13 | 128. | 54 to <57 | 18 |
| 92. | 48 to <51 | 14 | 129. | 54 to <57 | 19 |
| 93. | 48 to <51 | 15 | 130. | 54 to <57 | 20+ |
| 94. | 48 to <51 | 16 | 131. | 57 to <60 | -5 |
| 95. | 48 to <51 | 17 | 132. | 57 to <60 | 6 |
| 96. | 48 to <51 | 18 | 133. | 57 to <60 | 7 |
| 97. | 48 to <51 | 19 | 134. | 57 to <60 | 8 |
| 98. | 48 to <51 | 20+ | 135. | 57 to <60 | 9 |
| 99. | 51 to <54 | -5 | 136. | 57 to <60 | 10 |
| 100. | 51 to <54 | 6 | 137. | 57 to <60 | 11 |
| 101. | 51 to <54 | 7 | 138. | 57 to <60 | 12 |

| No. | Hunter L | Hunter b |
|---|---|---|
| 139. | 57 to <60 | 13 |
| 140. | 57 to <60 | 14 |
| 141. | 57 to <60 | 15 |
| 142. | 57 to <60 | 16 |
| 143. | 57 to <60 | 17 |
| 144. | 57 to <60 | 18 |
| 145. | 57 to <60 | 19 |
| 146. | 57 to <60 | 20+ |
| 147. | 60 to <63 | -5 |
| 148. | 60 to <63 | 6 |
| 149. | 60 to <63 | 7 |
| 150. | 60 to <63 | 8 |
| 151. | 60 to <63 | 9 |
| 152. | 60 to <63 | 10 |
| 153. | 60 to <63 | 11 |
| 154. | 60 to <63 | 12 |
| 155. | 60 to <63 | 13 |
| 156. | 60 to <63 | 14 |
| 157. | 60 to <63 | 15 |
| 158. | 60 to <63 | 16 |
| 159. | 60 to <63 | 17 |
| 160. | 60 to <63 | 18 |
| 161. | 60 to <63 | 19 |
| 162. | 60 to <63 | 20+ |
| 163. | 63 to <66 | -5 |
| 164. | 63 to <66 | 6 |
| 165. | 63 to <66 | 7 |
| 166. | 63 to <66 | 8 |
| 167. | 63 to <66 | 9 |
| 168. | 63 to <66 | 10 |
| 169. | 63 to <66 | 11 |
| 170. | 63 to <66 | 12 |
| 171. | 63 to <66 | 13 |
| 172. | 63 to <66 | 14 |
| 173. | 63 to <66 | 15 |
| 174. | 63 to <66 | 16 |

| No. | Hunter L | Hunter b |
|---|---|---|
| 175. | 63 to <66 | 17 |
| 176. | 63 to <66 | 13 |
| 177. | 63 to <66 | 13 |
| 178. | 63 to <66 | 20+ |
| 179. | 66 to <69 | -5 |
| 180. | 66 to <69 | 6 |
| 181. | 66 to <69 | 7 |
| 182. | 66 to <69 | 8 |
| 183. | 66 to <69 | 9 |
| 184. | 66 to <69 | 10 |
| 185. | 66 to <69 | 11 |
| 186. | 66 to <69 | 12 |
| 187. | 66 to <69 | 13 |
| 188. | 66 to <69 | 14 |
| 189. | 66 to <69 | 15 |
| 190. | 66 to <69 | 16 |
| 191. | 66 to <69 | 17 |
| 192. | 66 to <69 | 18 |
| 193. | 66 to <69 | 19 |
| 194. | 66 to <69 | 20+ |
| 195. | ≥69 | -5 |
| 196. | ≥69 | 6 |
| 197. | ≥69 | 7 |
| 198. | ≥69 | 8 |
| 199. | ≥69 | 9 |
| 200. | ≥69 | 10 |
| 201. | ≥69 | 11 |
| 202. | ≥69 | 12 |
| 203. | ≥69 | 13 |
| 204. | ≥69 | 14 |
| 205. | ≥69 | 15 |
| 206. | ≥69 | 16 |
| 207. | ≥69 | 17 |
| 208. | ≥69 | 18 |
| 209. | ≥69 | 19 |
| 210. | ≥69 | 20+ |

Wherein the designation -5 means less than 5 but more than 4 and the designation +12 means more than 12 but less than 13.

**13.** The process according to any one of Claims 1 to 7, wherein:

   (a) the step of compiling a group of lightness measure value ranges comprises compiling ranges of a colour factor whose value is substantially that of colour factor Hunter L, and

   (b) the step of associating with each lightness measure value range a value of a colour factor for use in comparison with a measurement of the value of that colour factor in the coloration of a test subject having a measured lightness within said range comprises associating with each lightness measure value range values of a colour factor whose value is substantially that of colour factor Hunter b, and the relationship between said lightness measure colour factor value ranges and said associated colour factor values being substantially as follows:

| No. | Hunter L | Hunter b | | No. | Hunter L | Hunter b |
|---|---|---|---|---|---|---|
| 1. | <27 | -5 | | 33. | 36 to <39 | -5 |
| 2. | <27 | 6 | | 34. | 36 to <39 | 5 |
| 3. | <27 | 7 | | 35. | 36 to <39 | 7 |
| 4. | <27 | 8 | | 36. | 36 to <39 | 3 |
| 5. | <27 | 9 | | 37. | 36 to <39 | 9 |
| 6. | <27 | 10 | | 38. | 36 to <39 | 10 |
| 7. | <27 | 11 | | 39. | 36 to <39 | 11 |
| 8. | <27 | 12+ | | 40. | 36 to <39 | 12 |
| 9. | 27 to <30 | -5 | | 41. | 36 to <39 | 13 |
| 10. | 27 to <30 | 6 | | 42. | 36 to <39 | 14 |
| 11. | 27 to <30 | 7 | | 43. | 36 to <39 | 15+ |
| 12. | 27 to <30 | 8 | | 44. | 39 to <42 | -5 |
| 13. | 27 to <30 | 9 | | 45. | 39 to <42 | 6 |
| 14. | 27 to <30 | 10 | | 46. | 39 to <42 | 7 |
| 15. | 27 to <30 | 11 | | 47. | 39 to <42 | 8 |
| 16. | 27 to <30 | 12+ | | 48. | 39 to <42 | 9 |
| 17. | 30 to <33 | -5 | | 49. | 39 to <42 | 10 |
| 18. | 30 to <33 | 6 | | 50. | 39 to <42 | 11 |
| 19. | 30 to <33 | 7 | | 51. | 39 to <42 | 12 |
| 20. | 30 to <33 | 8 | | 52. | 39 to <42 | 13 |
| 21. | 30 to <33 | 9 | | 53. | 39 to <42 | 14 |
| 22. | 30 to <33 | 10 | | 54. | 39 to <42 | 15+ |
| 23. | 30 to <33 | 11 | | 55. | 42 to <45 | -5 |
| 24. | 30 to <33 | 12+ | | 56. | 42 to <45 | 6 |
| 25. | 33 to <36 | -5 | | 57. | 42 to <45 | 7 |
| 26. | 33 to <36 | 6 | | 58. | 42 to <45 | 8 |
| 27. | 33 to <36 | 7 | | 59. | 42 to <45 | 9 |
| 28. | 33 to <36 | 8 | | 60. | 42 to <45 | 10 |
| 29. | 33 to <36 | 9 | | 61. | 42 to <45 | 11 |
| 30. | 33 to <36 | 10 | | 62. | 42 to <45 | 12 |
| 31. | 33 to <36 | 11 | | 63. | 42 to <45 | 13 |
| 32. | 33 to <36 | 12+ | | 64. | 42 to <45 | 14 |

| No. | Hunter L | Hunter b |
|-----|----------|----------|
| 55. | 42 to <45 | 15 |
| 56. | 42 to <45 | 16 |
| 57. | 42 to <45 | 17 |
| 68. | 42 to <45 | 19+ |
| 69. | 45 to <48 | -5 |
| 70. | 45 to <48 | 6 |
| 71. | 45 to <48 | 7 |
| 72. | 45 to <48 | 8 |
| 73. | 45 to <48 | 9 |
| 74. | 45 to <48 | 10 |
| 75. | 45 to <48 | 11 |
| 76. | 45 to <48 | 12 |
| 77. | 45 to <48 | 13 |
| 78. | 45 to <48 | 14 |
| 79. | 45 to <48 | 15 |
| 80. | 45 to <48 | 16 |
| 81. | 45 to <48 | 17 |
| 82. | 45 to <48 | 18+ |
| 83. | 48 to <51 | -5 |
| 84. | 48 to <51 | 6 |
| 85. | 48 to <51 | 7 |
| 86. | 48 to <51 | 8 |
| 87. | 48 to <51 | 9 |
| 88. | 48 to <51 | 10 |
| 89. | 48 to <51 | 11 |
| 90. | 48 to <51 | 12 |
| 91. | 48 to <51 | 13 |
| 92. | 48 to <51 | 14 |
| 93. | 48 to <51 | 15 |
| 94. | 48 to <51 | 16 |
| 95. | 48 to <51 | 17 |
| 96. | 48 to <51 | 18 |
| 97. | 48 to <51 | 19 |
| 98. | 48 to <51 | 20+ |
| 99. | 51 to <54 | -5 |
| 100. | 51 to <54 | 6 |
| 101. | 51 to <54 | 7 |

| No. | Hunter L | Hunter b |
|-----|----------|----------|
| 102. | 51 to <54 | 8 |
| 103. | 51 to <54 | 9 |
| 104. | 51 to <54 | 10 |
| 105. | 51 to <54 | 11 |
| 106. | 51 to <54 | 12 |
| 107. | 51 to <54 | 13 |
| 108. | 51 to <54 | 14 |
| 109. | 51 to <54 | 15 |
| 110. | 51 to <54 | 16 |
| 111. | 51 to <54 | 17 |
| 112. | 51 to <54 | 18 |
| 113. | 51 to <54 | 19 |
| 114. | 51 to <54 | 20+ |
| 115. | 54 to <57 | -5 |
| 116. | 54 to <57 | 6 |
| 117. | 54 to <57 | 7 |
| 118. | 54 to <57 | 8 |
| 119. | 54 to <57 | 9 |
| 120. | 54 to <57 | 10 |
| 121. | 54 to <57 | 11 |
| 122. | 54 to <57 | 12 |
| 123. | 54 to <57 | 13 |
| 124. | 54 to <57 | 14 |
| 125. | 54 to <57 | 15 |
| 126. | 54 to <57 | 16 |
| 127. | 54 to <57 | 17 |
| 128. | 54 to <57 | 18 |
| 129. | 54 to <57 | 19 |
| 130. | 54 to <57 | 20+ |
| 131. | 57 to <60 | -5 |
| 132. | 57 to <60 | 6 |
| 133. | 57 to <60 | 7 |
| 134. | 57 to <60 | 8 |
| 135. | 57 to <60 | 9 |
| 136. | 57 to <60 | 10 |
| 137. | 57 to <60 | 11 |
| 138. | 57 to <60 | 12 |

| No. | Hunter L | Hunter b | No. | Hunter L | Hunter b |
|---|---|---|---|---|---|
| 139. | 57 to <60 | 13 | 175. | 63 to <66 | 17 |
| 140. | 57 to <60 | 14 | 176. | 63 to <66 | 18 |
| 141. | 57 to <60 | 15 | 177. | 63 to <66 | 19 |
| 142. | 57 to <60 | 16 | 178. | 63 to <66 | 20+ |
| 143. | 57 to <60 | 17 | 179. | 66 to <69 | -5 |
| 144. | 57 to <60 | 18 | 180. | 66 to <69 | 6 |
| 145. | 57 to <60 | 19 | 181. | 66 to <69 | 7 |
| 146. | 57 to <60 | 20+ | 182. | 66 to <69 | 8 |
| 147. | 60 to <63 | -5 | 183. | 66 to <69 | 9 |
| 148. | 60 to <63 | 6 | 184. | 66 to <69 | 10 |
| 149. | 60 to <63 | 7 | 185. | 66 to <69 | 11 |
| 150. | 60 to <63 | 8 | 186. | 66 to <69 | 12 |
| 151. | 60 to <63 | 9 | 187. | 66 to <69 | 13 |
| 152. | 60 to <63 | 10 | 188. | 66 to <69 | 14 |
| 153. | 60 to <63 | 11 | 189. | 66 to <69 | 15 |
| 154. | 60 to <63 | 12 | 190. | 66 to <69 | 16 |
| 155. | 60 to <63 | 13 | 191. | 66 to <69 | 17 |
| 156. | 60 to <63 | 14 | 192. | 66 to <69 | 18 |
| 157. | 60 to <63 | 15 | 193. | 66 to <69 | 19 |
| 158. | 60 to <63 | 16 | 194. | 66 to <69 | 20+ |
| 159. | 60 to <63 | 17 | 195. | ≥69 | -5 |
| 160. | 60 to <63 | 18 | 196. | ≥69 | 6 |
| 161. | 60 to <63 | 19 | 197. | ≥69 | 7 |
| 162. | 60 to <63 | 20+ | 198. | ≥69 | 8 |
| 163. | 63 to <66 | -5 | 199. | ≥69 | 9 |
| 164. | 63 to <66 | 6 | 200. | ≥69 | 10 |
| 165. | 63 to <66 | 7 | 201. | ≥69 | 11 |
| 166. | 63 to <66 | 8 | 202. | ≥69 | 12 |
| 167. | 63 to <66 | 9 | 203. | ≥69 | 13 |
| 168. | 63 to <66 | 10 | 204. | ≥69 | 14 |
| 169. | 63 to <66 | 11 | 205. | ≥69 | 15 |
| 170. | 63 to <66 | 12 | 206. | ≥69 | 16 |
| 171. | 63 to <66 | 13 | 207. | ≥69 | 17 |
| 172. | 63 to <66 | 14 | 208. | ≥69 | 18 |
| 173. | 63 to <66 | 15 | 209. | ≥69 | 19 |
| 174. | 63 to <66 | 16 | 210. | ≥69 | 20+ |

Wherein the designation -5 means less than 5 but more than 4 and the designation +12 means more than 12 but less than 13.

**14.** The process according to any one of Claims 1 to 7, wherein:

step (a) comprises compiling at least one range of a colour factor whose value is substantially that of colour factor Hunter L, said at least one range being selected from the ranges substantially as follows:

Hunter L=<27, 27 to <30, 30 to <33, 33 to <36, 36 to <39, 39 to <42, 42 to <45, 45 to <48, 48 to <51, 51 to <54, 54 to <57, 57 to <60, 60 to <63, 63 to <66, 66 to <69, and >=69.

**15.** The process according to any one of the preceding claims, comprising:

(c) measuring with a colour measuring instrument a value of said lightness measure in the test subject's skin coloration, and

(d) measuring with a colour measuring instrument a value of said colour factor in the test subject's skin coloration.

**16.** The process according to Claim 15, wherein said lightness measure is a lightness colour factor dependent on the lightness of the coloration of said test subject's skin, ranges of values of said lightness colour factor constituting said lightness measure value ranges, said colour factor of step (b) being dependent on the relative content of opponent colours in the coloration of said test subject's skin, wherein at least one of steps (a) and (b) comprises arriving at a measured value that correlates to a measure of coloration having established utility.

**17.** The process according to Claim 15 or 16, wherein:

step (a) comprises compiling a group of lightness measure value ranges that are found in the coloration of the skin colour of subjects other than the test subject;
step (b) comprises associating with each lightness measure value range a range of values of said colour factor in the coloration of subjects other than the test subject, the process further comprising comparing the measured values of said lightness measure and said colour factor with said ranges of values of said lightness measure and said colour factor that are found in the colorations of subjects other than said test subject as compiled and assembled in steps (a) and (b), to determine where within said range the measured values lie.

**18.** The process according to any one of Claims 15 to 17, wherein said colour factor is dependent on relative content of blue and yellow in 'said coloration.

**19.** The process according to any one of Claims 15 to 17, wherein said colour factor is dependent on relative content of red and green in said coloration.

**20.** The process according to any one of Claims 15 to 19, wherein each of steps (c) and (d) comprises measuring the values of said lightness measure and said colour factor at different locations on the test subject.

**21.** The process according to any one of Claims 15 to 19, wherein each of steps (c) and (d) comprises making a set of multiple measurements of the values of each of said lightness measure said colour factor and averaging each set of multiple measurements.

**22.** Apparatus for evaluating a medical or non-cosmetic physiological condition of a human or animal test subject, the condition is associated with a symptomatic, detectable skin coloration comprises:

(a) means for compiling a group of lightness measure value ranges, and.
(b) means for associating with each lightness measure value range a value or range of values of a colour factor for use in comparison with a measurement of the value of that colour factor in the coloration of a test subject having a measured lightness within said range, with the proviso that the apparatus is not used to determine a skin-coloured cosmetic for the test subject.

**23.** The apparatus according to Claim 22, wherein the condition comprises a condition that causes jaundice in a human child or adult test subject.

**24.** The apparatus according to Claim 22, wherein the condition is hyperbilirubinemia, hypertension, tuberculosis or liver disorder.

**25.** The apparatus according to any one of Claims 22 to 24, wherein the apparatus is adapted for evaluating a condition of a human infant.

**26.** The apparatus according to any one of Claims 22 to 25, wherein the means for compiling comprises means for assembling the group of lightness measure value ranges in computer processable form, and the means for associating comprises means for associating the colour factor value with each lightness measure value range in said computer processable form.

27. The apparatus according to any one of Claims 22 to 26, wherein the means for associating comprises means for associating a value of colour factor Hunter b with each lightness measure value range.

28. The apparatus according to any one of Claims 22 to 27, wherein the means for compiling comprises means for compiling a group of values of lightness measure Hunter L.

29. The apparatus according to any one of Claims 22 to 26, wherein the means for compiling comprises means for compiling a group of lightness measure colour factor values that are substantially those of colour factor Hunter L, and the means for associating comprises means for associating a value of a colour factor whose value is substantially that of colour factor Hunter a with each lightness measure colour factor value range, the relationship between said lightness measure colour factor value ranges and said associated colour factor values being substantially as follows:

| Hunter L | Hunter a |
|---|---|
| 24 (or less) to 44 | 4 to 16 |
| 45 to 54 to | 18 |
| 55 to 59 to | 25 |
| 60 to 71 (or more) | 6 to 30. |

30. The apparatus according to any one of Claims 22 to 26, wherein the means for associating comprises means for associating with each lightness measure value range a value of a yellowness-dependent colour factor.

31. The apparatus according to any one of Claims 22 to 26, wherein the colour factor is Hunter b and the lightness measure value ranges are ranges of Hunter L, the apparatus further comprising the means for measuring by instrument a Hunter L value of the test subject's skin coloration.

32. The apparatus according to any one of Claims 22 to 28, wherein said ranges of values are determined by ranges of Hunter L values; said ranges of Hunter L values being bounded by at least one of the Hunter L values substantially as follows:

Hunter L 27, 30, 33, 36, 39, 42, 45, 48, 51, 54, 57, 60, 63, 66 and 69.

33. The apparatus according to any one of Claims 22 to 28, wherein the coloration is skin coloration and the condition is hyperbilirubinemia, the means for compiling a group of lightness measure value ranges comprises means for compiling ranges that are substantially ranges of values of Hunter L, and the means for associating with each lightness measure value range a value of a colour factor comprises means for associating a range of values substantially those of Hunter b with each lightness measure value range, the apparatus further comprising:

(c) means for measuring with a colour measuring instrument a value of a first colour factor substantially that of Hunter b in skin coloration of the test subject;

(d) means for measuring with a colour measuring instrument a value of a second colour factor substantially that of Hunter L in said skin coloration of the test subject;

(e) means for comparing the measured second colour factor with the ranges compiled by the means for compiling ranges that are substantially ranges of Hunter L to locate a comparable lightness in the coloration of the test subject and the compiled ranges; and

(f) means for comparing the measured first colour factor value that is substantially that of Hunter b with a value range of that colour factor associated by the means for associating a value substantially that of Hunter b with the comparable compiled lightness measure value range to determine if the measured value of the first colour factor lies inside or outside the associated range of first colour factor values; wherein said range of associated values is selected from ranges substantially as follows:

| No. | Hunter L | Hunter b | | No. | Hunter L | Hunter b |
|---|---|---|---|---|---|---|
| 1. | <27 | -5 | | 33. | 36 to <39 | -5 |
| 2. | <27 | 6 | | 34. | 36 to <39 | 5 |
| 3. | <27 | 7 | | 35. | 36 to <39 | 7 |
| 4. | <27 | 8 | | 36. | 36 to <39 | 3 |
| 5. | <27 | 9 | | 37. | 36 to <39 | 9 |
| 6. | <27 | 10 | | 38. | 36 to <39 | 10 |
| 7. | <27 | 11 | | 39. | 36 to <39 | 11 |
| 8. | <27 | 12+ | | 40. | 36 to <39 | 12 |
| 9. | 27 to <30 | -5 | | 41. | 36 to <39 | 13 |
| 10. | 27 to <30 | 6 | | 42. | 36 to <39 | 14 |
| 11. | 27 to <30 | 7 | | 43. | 36 to <39 | 15+ |
| 12. | 27 to <30 | 8 | | 44. | 39 to <42 | -5 |
| 13. | 27 to <30 | 9 | | 45. | 39 to <42 | 6 |
| 14. | 27 to <30 | 10 | | 46. | 39 to <42 | 7 |
| 15. | 27 to <30 | 11 | | 47. | 39 to <42 | 8 |
| 16. | 27 to <30 | 12+ | | 48. | 39 to <42 | 9 |
| 17. | 30 to <33 | -5 | | 49. | 39 to <42 | 10 |
| 18. | 30 to <33 | 6 | | 50. | 39 to <42 | 11 |
| 19. | 30 to <33 | 7 | | 51. | 39 to <42 | 12 |
| 20. | 30 to <33 | 8 | | 52. | 39 to <42 | 13 |
| 21. | 30 to <33 | 9 | | 53. | 39 to <42 | 14 |
| 22. | 30 to <33 | 10 | | 54. | 39 to <42 | 15+ |
| 23. | 30 to <33 | 11 | | 55. | 42 to <45 | -5 |
| 24. | 30 to <33 | 12+ | | 56. | 42 to <45 | 6 |
| 25. | 33 to <36 | -5 | | 57. | 42 to <45 | 7 |
| 26. | 33 to <36 | 6 | | 58. | 42 to <45 | 8 |
| 27. | 33 to <36 | 7 | | 59. | 42 to <45 | 9 |
| 28. | 33 to <36 | 8 | | 60. | 42 to <45 | 10 |
| 29. | 33 to <36 | 9 | | 61. | 42 to <45 | 11 |
| 30. | 33 to <36 | 10 | | 62. | 42 to <45 | 12 |
| 31. | 33 to <36 | 11 | | 63. | 42 to <45 | 13 |
| 32. | 33 to <36 | 12+ | | 64. | 42 to <45 | 14 |

| No. | Hunter L | Hunter b |
|-----|----------|----------|
| 55. | 42 to <45 | 15 |
| 56. | 42 to <45 | 16 |
| 67. | 42 to <45 | 17 |
| 68. | 42 to <45 | 19+ |
| 69. | 45 to <48 | -5 |
| 70. | 45 to <48 | 6 |
| 71. | 45 to <48 | 7 |
| 72. | 45 to <48 | 8 |
| 73. | 45 to <48 | 9 |
| 74. | 45 to <48 | 10 |
| 75. | 45 to <48 | 11 |
| 76. | 45 to <48 | 12 |
| 77. | 45 to <48 | 13 |
| 78. | 45 to <48 | 14 |
| 79. | 45 to <48 | 15 |
| 80. | 45 to <48 | 16 |
| 81. | 45 to <48 | 17 |
| 82. | 45 to <48 | 18+ |
| 83. | 48 to <51 | -5 |
| 84. | 48 to <51 | 6 |
| 85. | 48 to <51 | 7 |
| 86. | 48 to <51 | 8 |
| 87. | 48 to <51 | 9 |
| 88. | 48 to <51 | 10 |
| 89. | 48 to <51 | 11 |
| 90. | 48 to <51 | 12 |
| 91. | 48 to <51 | 13 |
| 92. | 48 to <51 | 14 |
| 93. | 48 to <51 | 15 |
| 94. | 48 to <51 | 16 |
| 95. | 48 to <51 | 17 |
| 96. | 48 to <51 | 18 |
| 97. | 48 to <51 | 19 |
| 98. | 48 to <51 | 20+ |
| 99. | 51 to <54 | -5 |
| 100. | 51 to <54 | 6 |
| 101. | 51 to <54 | 7 |

| No. | Hunter L | Hunter b |
|-----|----------|----------|
| 102. | 51 to <54 | 8 |
| 103. | 51 to <54 | 9 |
| 104. | 51 to <54 | 10 |
| 105. | 51 to <54 | 11 |
| 106. | 51 to <54 | 12 |
| 107. | 51 to <54 | 13 |
| 108. | 51 to <54 | 14 |
| 109. | 51 to <54 | 15 |
| 110. | 51 to <54 | 16 |
| 111. | 51 to <54 | 17 |
| 112. | 51 to <54 | 18 |
| 113. | 51 to <54 | 19 |
| 114. | 51 to <54 | 20+ |
| 115. | 54 to <57 | -5 |
| 116. | 54 to <57 | 6 |
| 117. | 54 to <57 | 7 |
| 118. | 54 to <57 | 8 |
| 119. | 54 to <57 | 9 |
| 120. | 54 to <57 | 10 |
| 121. | 54 to <57 | 11 |
| 122. | 54 to <57 | 12 |
| 123. | 54 to <57 | 13 |
| 124. | 54 to <57 | 14 |
| 125. | 54 to <57 | 15 |
| 126. | 54 to <57 | 16 |
| 127. | 54 to <57 | 17 |
| 128. | 54 to <57 | 18 |
| 129. | 54 to <57 | 19 |
| 130. | 54 to <57 | 20+ |
| 131. | 57 to <60 | -5 |
| 132. | 57 to <60 | 6 |
| 133. | 57 to <60 | 7 |
| 134. | 57 to <60 | 8 |
| 135. | 57 to <60 | 9 |
| 136. | 57 to <60 | 10 |
| 137. | 57 to <60 | 11 |
| 138. | 57 to <60 | 12 |

| No. | Hunter L | Hunter b | No. | Hunter L | Hunter b |
|---|---|---|---|---|---|
| 139. | 57 to <60 | 13 | 175. | 63 to <66 | 17 |
| 140. | 57 to <60 | 14 | 176. | 63 to <66 | 18 |
| 141. | 57 to <60 | 15 | 177. | 63 to <66 | 19 |
| 142. | 57 to <60 | 16 | 178. | 63 to <66 | 20+ |
| 143. | 57 to <60 | 17 | 179. | 66 to <69 | -5 |
| 144. | 57 to <60 | 18 | 180. | 66 to <69 | 6 |
| 145. | 57 to <60 | 19 | 181. | 66 to <69 | 7 |
| 146. | 57 to <60 | 20+ | 182. | 66 to <69 | 8 |
| 147. | 60 to <63 | -5 | 183. | 66 to <69 | 9 |
| 148. | 60 to <63 | 6 | 184. | 66 to <69 | 10 |
| 149. | 60 to <63 | 7 | 185. | 66 to <69 | 11 |
| 150. | 60 to <63 | 8 | 186. | 66 to <69 | 12 |
| 151. | 60 to <63 | 9 | 187. | 66 to <69 | 13 |
| 152. | 60 to <63 | 10 | 188. | 66 to <69 | 14 |
| 153. | 60 to <63 | 11 | 189. | 66 to <69 | 15 |
| 154. | 60 to <63 | 12 | 190. | 66 to <69 | 16 |
| 155. | 60 to <63 | 13 | 191. | 66 to <69 | 17 |
| 156. | 60 to <63 | 14 | 192. | 66 to <69 | 18 |
| 157. | 60 to <63 | 15 | 193. | 66 to <69 | 19 |
| 158. | 60 to <63 | 16 | 194. | 66 to <69 | 20+ |
| 159. | 60 to <63 | 17 | 195. | ≥69 | -5 |
| 160. | 60 to <63 | 18 | 196. | ≥69 | 6 |
| 161. | 60 to <63 | 19 | 197. | ≥69 | 7 |
| 162. | 60 to <63 | 20+ | 198. | ≥69 | 8 |
| 163. | 63 to <66 | -5 | 199. | ≥69 | 9 |
| 164. | 63 to <66 | 6 | 200. | ≥69 | 10 |
| 165. | 63 to <66 | 7 | 201. | ≥69 | 11 |
| 166. | 63 to <66 | 8 | 202. | ≥69 | 12 |
| 167. | 63 to <66 | 9 | 203. | ≥69 | 13 |
| 168. | 63 to <66 | 10 | 204. | ≥69 | 14 |
| 169. | 63 to <66 | 11 | 205. | ≥69 | 15 |
| 170. | 63 to <66 | 12 | 206. | ≥69 | 16 |
| 171. | 63 to <66 | 13 | 207. | ≥69 | 17 |
| 172. | 63 to <66 | 14 | 208. | ≥69 | 18 |
| 173. | 63 to <66 | 15 | 209. | ≥69 | 19 |
| 174. | 63 to <66 | 16 | 210. | ≥69 | 20+ |

Wherein the designation -5 means less than 5 but more than 4 and the designation +12 means more than 12 but less than 13.

**34.** The apparatus according to any one of Claims 22 to 28, wherein:

the means for compiling a group of lightness measure value ranges comprises means for compiling ranges of a colour factor whose value is substantially that of colour factor Hunter L, and

the means for associating with each lightness measure value range a value of a colour factor for use in comparison with a measurement of the value of that colour factor in the coloration of a test subject having a measured lightness within said range comprises means for associating with each lightness measure value range values of a colour factor whose value is substantially that of colour factor Hunter b, and the relationship between said lightness measure colour factor value ranges and said associated colour factor values being substantially as follows:

| No. | Hunter L | Hunter b | No. | Hunter L | Hunter b |
|------|----------|----------|------|-----------|----------|
| 1. | <27 | -5 | 33. | 36 to <39 | -5 |
| 2. | <27 | 6 | 34. | 36 to <39 | 5 |
| 3. | <27 | 7 | 35. | 36 to <39 | 7 |
| 4. | <27 | 8 | 36. | 36 to <39 | 3 |
| 5. | <27 | 9 | 37. | 36 to <39 | 9 |
| 6. | <27 | 10 | 38. | 36 to <39 | 10 |
| 7. | <27 | 11 | 39. | 36 to <39 | 11 |
| 8. | <27 | 12+ | 40. | 36 to <39 | 12 |
| 9. | 27 to <30 | -5 | 41. | 36 to <39 | 13 |
| 10. | 27 to <30 | 6 | 42. | 36 to <39 | 14 |
| 11. | 27 to <30 | 7 | 43. | 36 to <39 | 15+ |
| 12. | 27 to <30 | 8 | 44. | 39 to <42 | -5 |
| 13. | 27 to <30 | 9 | 45. | 39 to <42 | 6 |
| 14. | 27 to <30 | 10 | 46. | 39 to <42 | 7 |
| 15. | 27 to <30 | 11 | 47. | 39 to <42 | 8 |
| 16. | 27 to <30 | 12+ | 48. | 39 to <42 | 9 |
| 17. | 30 to <33 | -5 | 49. | 39 to <42 | 10 |
| 18. | 30 to <33 | 6 | 50. | 39 to <42 | 11 |
| 19. | 30 to <33 | 7 | 51. | 39 to <42 | 12 |
| 20. | 30 to <33 | 8 | 52. | 39 to <42 | 13 |
| 21. | 30 to <33 | 9 | 53. | 39 to <42 | 14 |
| 22. | 30 to <33 | 10 | 54. | 39 to <42 | 15+ |
| 23. | 30 to <33 | 11 | 55. | 42 to <45 | -5 |
| 24. | 30 to <33 | 12+ | 56. | 42 to <45 | 6 |
| 25. | 33 to <36 | -5 | 57. | 42 to <45 | 7 |
| 26. | 33 to <36 | 6 | 58. | 42 to <45 | 8 |
| 27. | 33 to <36 | 7 | 59. | 42 to <45 | 9 |
| 28. | 33 to <36 | 8 | 60. | 42 to <45 | 10 |
| 29. | 33 to <36 | 9 | 61. | 42 to <45 | 11 |
| 30. | 33 to <36 | 10 | 62. | 42 to <45 | 12 |
| 31. | 33 to <36 | 11 | 63. | 42 to <45 | 13 |
| 32. | 33 to <36 | 12+ | 64. | 42 to <45 | 14 |

| No. | Hunter L | Hunter b | | No. | Hunter L | Hunter b |
|-----|----------|----------|---|-----|----------|----------|
| 55. | 42 to <45 | 15 | | 102. | 51 to <54 | 3 |
| 56. | 42 to <45 | 16 | | 103. | 51 to <54 | 3 |
| 57. | 42 to <45 | 17 | | 104. | 51 to <54 | 10 |
| 68. | 42 to <45 | 19+ | | 105. | 51 to <54 | 11 |
| 69. | 45 to <48 | -5 | | 106. | 51 to <54 | 12 |
| 70. | 45 to <48 | 6 | | 107. | 51 to <54 | 13 |
| 71. | 45 to <48 | 7 | | 108. | 51 to <54 | 14 |
| 72. | 45 to <48 | 8 | | 109. | 51 to <54 | 15 |
| 73. | 45 to <48 | 9 | | 110. | 51 to <54 | 16 |
| 74. | 45 to <48 | 10 | | 111. | 51 to <54 | 17 |
| 75. | 45 to <48 | 11 | | 112. | 51 to <54 | 18 |
| 76. | 45 to <48 | 12 | | 113. | 51 to <54 | 19 |
| 77. | 45 to <48 | 13 | | 114. | 51 to <54 | 20+ |
| 78. | 45 to <48 | 14 | | 115. | 54 to <57 | -5 |
| 79. | 45 to <48 | 15 | | 116. | 54 to <57 | 6 |
| 80. | 45 to <48 | 16 | | 117. | 54 to <57 | 7 |
| 81. | 45 to <48 | 17 | | 118. | 54 to <57 | 8 |
| 82. | 45 to <48 | 18+ | | 119. | 54 to <57 | 9 |
| 83. | 48 to <51 | -5 | | 120. | 54 to <57 | 10 |
| 84. | 48 to <51 | 6 | | 121. | 54 to <57 | 11 |
| 85. | 48 to <51 | 7 | | 122. | 54 to <57 | 12 |
| 86. | 48 to <51 | 8 | | 123. | 54 to <57 | 13 |
| 87. | 48 to <51 | 9 | | 124. | 54 to <57 | 14 |
| 88. | 48 to <51 | 10 | | 125. | 54 to <57 | 15 |
| 89. | 48 to <51 | 11 | | 126. | 54 to <57 | 16 |
| 90. | 48 to <51 | 12 | | 127. | 54 to <57 | 17 |
| 91. | 48 to <51 | 13 | | 128. | 54 to <57 | 18 |
| 92. | 48 to <51 | 14 | | 129. | 54 to <57 | 19 |
| 93. | 48 to <51 | 15 | | 130. | 54 to <57 | 20+ |
| 94. | 48 to <51 | 16 | | 131. | 57 to <60 | -5 |
| 95. | 48 to <51 | 17 | | 132. | 57 to <60 | 6 |
| 96. | 48 to <51 | 18 | | 133. | 57 to <60 | 7 |
| 97. | 48 to <51 | 19 | | 134. | 57 to <60 | 8 |
| 98. | 48 to <51 | 20+ | | 135. | 57 to <60 | 9 |
| 99. | 51 to <54 | -5 | | 136. | 57 to <60 | 10 |
| 100. | 51 to <54 | 6 | | 137. | 57 to <60 | 11 |
| 101. | 51 to <54 | 7 | | 138. | 57 to <60 | 12 |

| No. | Hunter L | Hunter b | No. | Hunter L | Hunter b |
|---|---|---|---|---|---|
| 139. | 57 to <60 | 13 | 175. | 63 to <66 | 17 |
| 140. | 57 to <60 | 14 | 176. | 63 to <66 | 18 |
| 141. | 57 to <60 | 15 | 177. | 63 to <66 | 19 |
| 142. | 57 to <60 | 16 | 178. | 63 to <66 | 20+ |
| 143. | 57 to <60 | 17 | 179. | 66 to <69 | -5 |
| 144. | 57 to <60 | 18 | 180. | 66 to <69 | 6 |
| 145. | 57 to <60 | 19 | 181. | 66 to <69 | 7 |
| 146. | 57 to <60 | 20+ | 182. | 66 to <69 | 8 |
| 147. | 60 to <63 | -5 | 183. | 66 to <69 | 9 |
| 148. | 60 to <63 | 6 | 184. | 66 to <69 | 10 |
| 149. | 60 to <63 | 7 | 185. | 66 to <69 | 11 |
| 150. | 60 to <63 | 8 | 186. | 66 to <69 | 12 |
| 151. | 60 to <63 | 9 | 187. | 66 to <69 | 13 |
| 152. | 60 to <63 | 10 | 188. | 66 to <69 | 14 |
| 153. | 60 to <63 | 11 | 189. | 66 to <69 | 15 |
| 154. | 60 to <63 | 12 | 190. | 66 to <69 | 16 |
| 155. | 60 to <63 | 13 | 191. | 66 to <69 | 17 |
| 156. | 60 to <63 | 14 | 192. | 66 to <69 | 18 |
| 157. | 60 to <63 | 15 | 193. | 66 to <69 | 19 |
| 158. | 60 to <63 | 16 | 194. | 66 to <69 | 20+ |
| 159. | 60 to <63 | 17 | 195. | ≥69 | -5 |
| 160. | 60 to <63 | 18 | 196. | ≥69 | 6 |
| 161. | 60 to <63 | 19 | 197. | ≥69 | 7 |
| 162. | 60 to <63 | 20+ | 198. | ≥69 | 8 |
| 163. | 63 to <66 | -5 | 199. | ≥69 | 9 |
| 164. | 63 to <66 | 6 | 200. | ≥69 | 10 |
| 165. | 63 to <66 | 7 | 201. | ≥69 | 11 |
| 166. | 63 to <66 | 8 | 202. | ≥69 | 12 |
| 167. | 63 to <66 | 9 | 203. | ≥69 | 13 |
| 168. | 63 to <66 | 10 | 204. | ≥69 | 14 |
| 169. | 63 to <66 | 11 | 205. | ≥69 | 15 |
| 170. | 63 to <66 | 12 | 206. | ≥69 | 16 |
| 171. | 63 to <66 | 13 | 207. | ≥69 | 17 |
| 172. | 63 to <66 | 14 | 208. | ≥69 | 18 |
| 173. | 63 to <66 | 15 | 209. | ≥69 | 19 |
| 174. | 63 to <66 | 16 | 210. | ≥69 | 20+ |

Wherein the designation -5 means less than 5 but more than 4 and the designation +12 means more than 12 but less than 13.

**35.** The apparatus according to any one of Claims 22 to 28, wherein:

the means for compiling comprises means for compiling at least one range of a colour factor whose value is substantially that of colour factor Hunter L, said at least one range being selected from the ranges substantially as follows:
Hunter L=<27, 27 to <30, 30 to <33, 33 to <36, 36 to <39, 39 to <42, 42 to <45, 45 to <48, 48 to <51, 51 to <54, 54 to <57, 57 to <60, 60 to <63, 63 to <66, 66 to <69, and >= 69.

36. The apparatus according to any one of Claims 22 to 35, further comprising:

(c) means for measuring with a colour measuring instrument (i) a value of said lightness measure in a test subject's coloration, and (ii) a value of said colour factor of (b) in said test subject's coloration.

37. The apparatus according to Claim 36, wherein said lightness measure is a lightness colour factor dependent on the lightness of the coloration of said test subject, ranges of values of said lightness colour factor constituting said lightness measure value ranges, said colour factor of (b) being dependent on the relative content of opponent colours in the coloration of said test subject, wherein at least one of the colour factors comprises a measured value that correlates to a measure of coloration having established utility.

38. The apparatus according to Claim 36 or 37, further comprising means for comparing the measured values of said first colour factor and said second colour factor with ranges of values of said colour factors that are found in the colorations of subjects other than said test subject as compiled and assembled by the means for compiling and the means for associating, to determine where within said ranges the measured values of said colour factors lie.

39. The apparatus according to any one of Claims 36 to 38, wherein the means for measuring comprises means for measuring the value of a colour factor that is dependent on relative content of blue and yellow in said coloration.

40. The apparatus according to any one of Claims 36 to 38, wherein the means for measuring comprises means for measuring the value of a colour factor that is dependent on relative content of red and green in said coloration.

41. The apparatus according to any one of Claims 36 to 40, wherein the means for measuring comprises means for measuring the values of said first colour factor and said second colour factor at different locations on the test subject.

42. The apparatus according to any one of Claims 36 to 41, wherein the means for measuring comprises means for making a set of multiple measurements of the values of each of said first colour factor and said second colour factor and averaging each set of multiple measurements.

**Patentansprüche**

1. Verfahren zur Bildung einer Zusammenstellung von Farbfaktorwerten, die auf einen medizinischen oder nicht-kosmetischen, physiologischen Zustand einer menschlichen oder tierischen Testperson hinweisen, wobei der Zustand mit einer symptomatischen, nachweisbaren Hautfärbung verbunden ist, mit den Schritten:

(a) Zusammenstellung einer Gruppe von Helligkeits-Meßwertbereichen und

(b) Assoziation jedes Helligkeits-Meßwertbereiches mit einem Wert oder Bereich von Farbfaktorwerten, die auf den Zustand einer Testperson, die eine gemessene Helligkeit im Helligkeits-Meßwertbereich hat, hinweist, für die Verwendung im Vergleich mit einer Messung des Farbfaktorwertes bei der Färbung einer Testperson.

2. Verfahren nach Anspruch 1, wobei der Zustand ein bei kindlichen oder erwachsenen menschlichen Testpersonen Gelbsucht verursachender Zustand ist.

3. Verfahren nach Anspruch 1, wobei der Zustand einer von Hyperbilirubinemia, Hypertension, Tuberkulose oder Leberstörung ist.

4. Verfahren nach einem der vorhergehenden Ansprüche, wobei die Testperson ein menschliches Kleinkind ist.

5. Verfahren nach einem der vorhergehenden Ansprüchen, wobei Schritt (a) die Einordnung der Gruppe der Helligkeit-Meßwertbereiche in mit einem Computer bearbeitbarer Form enthält und Schritt (b) die Assoziation der Farbfaktorwerte mit jedem Helligkeits-Meßwertbereich in der genannten mit einem Computer bearbeitbaren Form enthält.

6. Verfahren nach einem der vorhergehenden Ansprüche, wobei Schritt (b) die Assoziation eines Wertes des Farbfaktors Hunter b mit jedem Helligkeits-Meßwertbereich enthält.

7. Verfahren nach einem der vorhergehenden Ansprüche, wobei Schritt (a) die Zusammenstellung einer Gruppe von Helligkeits-Meßwerten Hunter L enthält.

8. Verfahren nach einem der Ansprüche 1 bis 5, wobei Schritt (a) die Zusammenstellung einer Gruppe von Helligkeits-Farbfaktor-Meßwertbereichen, die im wesentlichen die vom Farbfaktor
Hunter L sind und Schritt (b) die Assoziation eines Wertes eines Farbfaktors, mit jedem Helligkeits-Farbfaktor-Meßwertbereich umfaßt, wobei der Wert im wesentlichen der des Farbfaktors Hunter a ist, und das Verhältnis zwischen dem besagten Helligkeits-Farbfaktor-Meßwertbereich und den besagten assoziierten Farbfaktorwerten im wesentlichen folgendes ist:

| Hunter L | Hunter a |
|---|---|
| 24 (oder weniger) bis 44 | 4 bis 16 |
| 45 bis 54 bis | 18 |
| 55 bis 59 bis | 25 |
| 60 bis 71 (oder mehr) | 6 bis 30 |

9. Verfahren nach einem der Ansprüche 1 bis 5, wobei Schritt (b) die Assoziation eines Wertes eines vergilbungsabhängigen Farbfaktors mit jedem Helligkeits-Meßwertbereich enthält.

10. Verfahren nach einem der Ansprüche 1 bis 7, wobei der Farbfaktor aus Schritt (b) Hunter b ist und die Helligkeits-Meßwertbereiche von Schritt (a) Bereiche von Hunter L sind, und wobei eine Bestimmung des Zustands den Schritt der Messung des Hunter L-Wertes der Hautfärbung der Testperson mittels Instrument einschließen kann.

11. Verfahren nach einem der Ansprüche 1 bis 7, wobei die erwähnten Wertebereiche aus Schritt (a) durch Bereiche der Hunter L-Werte bestimmt sind; die erwähnten Bereiche der Hunter L-Werte im wesentlichen an wenigstens einen der Hunter L-Werte wie folgt gebunden sind:
Hunter L = 27,30,33,36,39,42,45,48,51,57,60,63, 66 und 69.

12. Verfahren nach mindestens einem der Ansprüche 1 bis 7, wobei der Zustand Hyperbilirubinemia ist, Schritt (a) die Zusammenstellung von Bereichen, die im wesentlichen Bereiche der Hunter L-Werte sind, enthält; und Schritt (b) die Assoziation eines Wertebereiches, im wesentlichen der von Hunter b, mit jeden Helligkeits-Meßwertbereich enthält; und der Zustand der Testperson bestimmt wird durch:

(c) Messung eines Wertes eines ersten Farbfaktors, der im wesentlichen der des Hunter b bei der Hautfärbung der Testperson ist, mit einem Farbmeßinstrument;

(d) Messung eines Wertes eines zweiten Farbfaktors, der im wesentlichen der des Hunter L bei der Hautfärbung der Testperson ist, mit einem Farbmeßinstrument;

(e) Vergleich des gemessenen zweiten Farbfaktorwertes mit den Bereichen, die in Schritt (a) zusammengestellt wurden, um eine vergleichbare Helligkeit bei der Färbung des Testobjektes und den zusammengestellten Bereichen einzugrenzen; und

(f) Vergleich des gemessenen ersten Farbfaktorwertes mit einem ersten Farbfaktorwertbereich, der in Schritt (b) mit dem vergleichbaren zusammengestellten Helligkeits-Meßwertbereich assoziiert wurde, um zu bestimmen, ob der gemessene Wert des ersten Farbfaktors innerhalb oder außerhalb des assoziierten Bereiches der ersten Farbfaktorwerte liegt; wobei der erwähnte assoziierte Bereich der ersten Farbfaktorwerte im wesentlichen aus den Bereichen wie folgt ausgewählt ist:

| Nr. | Hunter L | Hunter b |
|-----|----------|----------|
| 1. | < 27 | - 5 |
| 2. | < 27 | 6 |
| 3. | < 27 | 7 |
| 4. | < 27 | 8 |
| 5. | < 27 | 9 |
| 6. | < 27 | 10 |
| 7. | < 27 | 11 |
| 8. | < 27 | 12 + |
| 9. | 27 bis < 30 | - 5 |
| 10. | 27 bis < 30 | 6 |
| 11. | 27 bis < 30 | 7 |
| 12. | 27 bis < 30 | 8 |
| 13. | 27 bis < 30 | 9 |
| 14. | 27 bis < 30 | 10 |
| 15. | 27 bis < 30 | 11 |
| 16. | 27 bis < 30 | 12 + |
| 17. | 30 bis < 33 | - 5 |
| 18. | 30 bis < 33 | 6 |
| 19. | 30 bis < 33 | 7 |
| 20. | 30 bis < 33 | 8 |
| 21. | 30 bis < 33 | 9 |
| 22. | 30 bis < 33 | 10 |
| 23. | 30 bis < 33 | 11 |
| 24. | 30 bis < 33 | 12 + |
| 25. | 30 bis < 33 | - 5 |
| 26. | 30 bis < 33 | 6 |
| 27. | 30 bis < 33 | 7 |
| 28. | 33 bis < 36 | 8 |
| 29. | 33 bis < 36 | 9 |
| 30. | 33 bis < 36 | 10 |
| 31. | 33 bis < 36 | 11 |
| 32. | 33 bis < 36 | 12 + |

| Nr. | Hunter L | Hunter b |
|-----|----------|----------|
| 33. | 36 bis < 39 | - 5 |
| 34. | 36 bis < 39 | 6 |
| 35. | 36 bis < 39 | 7 |
| 36. | 36 bis < 39 | 8 |
| 37. | 36 bis < 39 | 9 |
| 38. | 36 bis < 39 | 10 |
| 39. | 36 bis < 39 | 11 |
| 40. | 36 bis < 39 | 12 |
| 41. | 36 bis < 39 | 13 |
| 42. | 36 bis < 39 | 14 |
| 43. | 36 bis < 39 | 15 + |
| 44. | 39 bis < 42 | - 5 |
| 45. | 39 bis < 42 | 6 |
| 46. | 39 bis < 42 | 7 |
| 47. | 39 to < 42 | 8 |
| 48. | 39 bis < 42 | 9 |
| 49. | 39 bis < 42 | 10 |
| 50. | 39 bis < 42 | 11 |
| 51. | 39 bis < 42 | 12 |
| 52. | 39 bis < 42 | 13 |
| 53. | 39 bis < 42 | 14 |
| 54. | 39 bis < 42 | 15 + |
| 55. | 42 bis< 45 | - 5 |
| 56. | 42 bis < 45 | 6 |
| 57. | 42 bis < 45 | 7 |
| 58. | 42 bis < 45 | 8 |
| 59. | 42 bis < 45 | 9 |
| 60. | 42 bis < 45 | 10 |
| 61. | 42 bis < 45 | 11 |
| 62. | 42 bis < 45 | 12 |
| 63. | 42 bis < 45 | 13 |
| 64. | 42 bis < 45 | 14 |

| Nr. | Hunter L | Hunter b |
|------|-----------|----------|
| 65. | 42 bis < 45 | 15 |
| 66. | 42 bis < 45 | 16 |
| 67. | 42 bis < 45 | 17 |
| 68. | 42 bis < 45 | 18 + |
| 69. | 45 bis < 48 | - 5 |
| 70. | 45 bis < 48 | 6 |
| 71. | 45 bis < 48 | 7 |
| 72. | 45 bis < 48 | 8 |
| 73. | 45 bis < 48 | 9 |
| 74. | 45 bis < 48 | 10 |
| 75. | 45 bis < 48 | 11 |
| 76. | 45 bis < 48 | 12 |
| 77. | 45 bis < 48 | 13 |
| 78. | 45 bis < 48 | 14 |
| 79. | 45 bis < 48 | 15 |
| 80. | 45 bis < 48 | 16 |
| 81. | 45 bis < 48 | 17 |
| 82. | 45 bis < 48 | 18 + |
| 83. | 48 bis < 51 | - 5 |
| 84. | 48 bis < 51 | 6 |
| 85. | 48 bis < 51 | 7 |
| 86. | 48 bis < 51 | 8 |
| 87. | 48 bis < 51 | 9 |
| 88. | 48 bis < 51 | 10 |
| 89. | 48 bis < 51 | 11 |
| 90. | 48 bis < 51 | 12 |
| 91. | 48 bis < 51 | 13 |
| 92. | 48 bis < 51 | 14 |
| 93. | 48 bis < 51 | 15 |
| 94. | 48 bis < 51 | 16 |
| 95. | 48 bis < 51 | 17 |
| 96. | 48 bis < 51 | 18 |
| 97. | 48 bis < 51 | 19 |
| 98. | 48 bis < 51 | 20 + |
| 99. | 51 bis < 54 | - 5 |
| 100. | 51 bis < 54 | 6 |
| 101. | 51 bis < 54 | 7 |

| Nr. | Hunter L | Hunter b |
|------|-----------|----------|
| 102. | 51 bis < 54 | 8 |
| 103. | 51 bis < 54 | 9 |
| 104. | 51 bis < 54 | 10 |
| 105. | 51 bis < 54 | 11 |
| 106. | 51 bis < 54 | 12 |
| 107. | 51 bis < 54 | 13 |
| 108. | 51 bis < 54 | 14 |
| 109. | 51 bis < 54 | 15 |
| 110. | 51 bis < 54 | 16 |
| 111. | 51 bis < 54 | 17 |
| 112. | 51 bis < 54 | 18 |
| 113. | 51 bis < 54 | 19 |
| 114. | 51 bis < 54 | 20 + |
| 115. | 54 bis < 57 | - 5 |
| 116. | 54 bis < 57 | 6 |
| 117. | 54 bis < 57 | 7 |
| 118. | 54 bis < 57 | 8 |
| 119. | 54 bis < 57 | 9 |
| 120. | 54 bis < 57 | 10 |
| 121. | 54 bis < 57 | 11 |
| 122. | 54 bis < 57 | 12 |
| 123. | 54 bis < 57 | 13 |
| 124. | 54 bis < 57 | 14 |
| 125. | 54 bis < 57 | 15 |
| 126. | 54 bis < 57 | 16 |
| 127. | 54 bis < 57 | 17 |
| 128. | 54 bis < 57 | 18 |
| 129. | 54 bis < 57 | 19 |
| 130. | 54 bis < 57 | 20 + |
| 131. | 57 bis < 60 | - 5 |
| 132. | 57 bis < 60 | 6 |
| 133. | 57 bis < 60 | 7 |
| 134. | 57 bis < 60 | 8 |
| 135. | 57 bis < 60 | 9 |
| 136. | 57 bis < 60 | 10 |
| 137. | 57 bis < 60 | 11 |
| 138. | 57 bis < 60 | 12 |

| Nr. | Hunter L | Hunter b | | Nr. | Hunter L | Hunter b |
|---|---|---|---|---|---|---|
| 139. | 57 bis < 60 | 13 | | 175. | 63 bis < 66 | 17 |
| 140. | 57 bis < 60 | 14 | | 176. | 63 bis < 66 | 18 |
| 141. | 57 bis < 60 | 15 | | 177. | 63 bis < 66 | 19 |
| 142. | 57 bis < 60 | 16 | | 178. | 63 bis < 66 | 20 + |
| 143. | 57 bis < 60 | 17 | | 179. | 66 bis < 69 | - 5 |
| 144. | 57 bis < 60 | 18 | | 180. | 66 bis < 69 | 6 |
| 145. | 57 bis < 60 | 19 | | 181. | 66 bis < 69 | 7 |
| 146. | 57 bis < 60 | 20 + | | 182. | 66 bis < 69 | 8 |
| 147. | 60 bis < 63 | - 5 | | 183. | 66 bis < 69 | 9 |
| 148. | 60 bis < 63 | 6 | | 184. | 66 bis < 69 | 10 |
| 149. | 60 bis < 63 | 7 | | 185. | 66 bis < 69 | 11 |
| 150. | 60 bis < 63 | 8 | | 186. | 66 bis < 69 | 12 |
| 151. | 60 bis < 63 | 9 | | 187. | 66 bis < 69 | 13 |
| 152. | 60 bis < 63 | 10 | | 188. | 66 bis < 69 | 14 |
| 153. | 60 bis < 63 | 11 | | 189. | 66 bis < 69 | 15 |
| 154. | 60 bis < 63 | 12 | | 190. | 66 bis < 69 | 16 |
| 155. | 60 bis < 63 | 13 | | 191. | 66 bis < 69 | 17 |
| 156. | 60 bis < 63 | 14 | | 192. | 66 bis < 69 | 18 |
| 157. | 60 bis < 63 | 15 | | 193. | 66 bis < 69 | 19 |
| 158. | 60 bis < 63 | 16 | | 194. | 66 bis < 69 | 20 + |
| 159. | 60 bis < 63 | 17 | | 195. | ≥ 69 | - 5 |
| 160. | 60 bis < 63 | 18 | | 196. | ≥ 69 | 6 |
| 161. | 60 bis < 63 | 19 | | 197. | ≥ 69 | 7 |
| 162. | 60 bis < 63 | 20 + | | 198. | ≥ 69 | 8 |
| 163. | 63 bis < 66 | - 5 | | 199. | ≥ 69 | 9 |
| 164. | 63 bis < 66 | 6 | | 200. | ≥ 69 | 10 |
| 165. | 63 bis < 66 | 7 | | 201. | ≥ 69 | 11 |
| 166. | 63 bis < 66 | 8 | | 202. | ≥ 69 | 12 |
| 167. | 63 bis < 66 | 9 | | 203. | ≥ 69 | 13 |
| 168. | 63 bis < 66 | 10 | | 204. | ≥ 69 | 14 |
| 169. | 63 bis < 66 | 11 | | 205. | ≥ 69 | 15 |
| 170. | 63 bis < 66 | 12 | | 206. | ≥ 69 | 16 |
| 171. | 63 bis < 66 | 13 | | 207. | ≥ 69 | 17 |
| 172. | 63 bis < 66 | 14 | | 208. | ≥ 69 | 18 |
| 173. | 63 bis < 66 | 15 | | 209. | ≥ 69 | 19 |
| 174. | 63 bis < 66 | 16 | | 210. | ≥ 69 | 20 + |

wobei die Bezeichnung -5 weniger als 5 aber mehr als 4 bedeutet und die Bezeichnung 12+ mehr als 12 aber weniger als 13 bedeutet.

**13.** Verfahren nach einem der Ansprüche 1 bis 7, wobei:

(a) der Schritt der Zusammenstellung einer Gruppe von Helligkeits-Meßwertbereichen die Zusammenstellung von Bereichen eines Farbfaktors, dessen Wert im wesentlichen der des Farbfaktors Hunter L ist, enthält, und

(b) der Schritt der Assoziierung eines Wertes eines Farbfaktors mit jedem Helligkeits-Meßwertbereich zum Vergleich mit einer Messung des Wertes des Faktors bei der Färbung einer Testperson, der eine gemessene Helligkeit innerhalb des erwähnten Bereiches hat, die Assoziation von Werten eines Farbfaktors, dessen Wert im wesentlichen der vom Farbfaktor Hunter b ist, mit jedem Helligkeits-Meßwertbereich enthält, und das Verhältnis zwischen den erwähnten Helligkeits-Farbfaktor-Meßwertbereichen und den erwähnten assoziierten Farbfaktorwerten im wesentlichen wie folgt ist:

| Nr. | Hunter L | Hunter b |
|-----|----------|----------|
| 1. | < 27 | - 5 |
| 2. | < 27 | 6 |
| 3. | < 27 | 7 |
| 4. | < 27 | 8 |
| 5. | < 27 | 9 |
| 6. | < 27 | 10 |
| 7. | < 27 | 11 |
| 8. | < 27 | 12 + |
| 9. | 27 bis < 30 | - 5 |
| 10. | 27 bis < 30 | 6 |
| 11. | 27 bis < 30 | 7 |
| 12. | 27 bis < 30 | 8 |
| 13. | 27 bis < 30 | 9 |
| 14. | 27 bis < 30 | 10 |
| 15. | 27 bis < 30 | 11 |
| 16. | 27 bis < 30 | 12 + |
| 17. | 30 bis < 33 | - 5 |
| 18. | 30 bis < 33 | 6 |
| 19. | 30 bis < 33 | 7 |
| 20. | 30 bis < 33 | 8 |
| 21. | 30 bis < 33 | 9 |
| 22. | 30 bis < 33 | 10 |
| 23. | 30 bis < 33 | 11 |
| 24. | 30 bis < 33 | 12 + |
| 25. | 33 bis < 36 | - 5 |
| 26. | 33 bis < 36 | 6 |
| 27. | 33 bis < 36 | 7 |
| 28. | 33 bis < 36 | 8 |
| 29. | 33 bis < 36 | 9 |
| 30. | 33 bis < 36 | 10 |
| 31. | 33 bis < 36 | 11 |
| 32. | 33 bis < 36 | 12 + |

| Nr. | Hunter L | Hunter b |
|-----|----------|----------|
| 33. | 36 bis < 39 | - 5 |
| 34. | 36 bis < 39 | 6 |
| 35. | 36 bis < 39 | 7 |
| 36. | 36 bis < 39 | 8 |
| 37. | 36 bis < 39 | 9 |
| 38. | 36 bis < 39 | 10 |
| 39. | 36 bis < 39 | 11 |
| 40. | 36 bis < 39 | 12 |
| 41. | 36 bis < 39 | 13 |
| 42. | 36 bis < 39 | 14 |
| 43. | 36 bis < 39 | 15 + |
| 44. | 39 bis < 42 | - 5 |
| 45. | 39 bis < 42 | 6 |
| 46. | 39 bis < 42 | 7 |
| 47. | 39 bis < 42 | 8 |
| 48. | 39 bis < 42 | 9 |
| 49. | 39 bis < 42 | 10 |
| 50. | 39 bis < 42 | 11 |
| 51. | 39 bis < 42 | 12 |
| 52. | 39 bis < 42 | 13 |
| 53. | 39 bis < 42 | 14 |
| 54. | 39 bis < 42 | 15 + |
| 55. | 42 bis < 45 | - 5 |
| 56. | 42 bis < 45 | 6 |
| 57. | 42 bis < 45 | 7 |
| 58. | 42 bis < 45 | 8 |
| 59. | 42 bis < 45 | 9 |
| 60. | 42 bis < 45 | 10 |
| 61. | 42 bis < 45 | 11 |
| 62. | 42 bis < 45 | 12 |
| 63. | 42 bis < 45 | 13 |
| 64. | 42 bis < 45 | 14 |

| No. | Hunter L | Hunter b | | No. | Hunter L | Hunter b |
|---|---|---|---|---|---|---|
| 65. | 42 bis < 45 | 15 | | 102. | 51 bis < 54 | 8 |
| 66. | 42 bis < 45 | 16 | | 103. | 51 bis < 54 | 9 |
| 67. | 42 bis < 45 | 17 | | 104. | 51 bis < 54 | 10 |
| 68. | 42 bis < 45 | 18 + | | 105. | 51 bis < 54 | 11 |
| 69. | 45 bis < 48 | - 5 | | 106. | 51 bis < 54 | 12 |
| 70. | 45 bis < 48 | 6 | | 107. | 51 bis < 54 | 13 |
| 71. | 45 bis < 48 | 7 | | 108. | 51 bis < 54 | 14 |
| 72. | 45 bis < 48 | 8 | | 109. | 51 bis < 54 | 15 |
| 73. | 45 bis < 48 | 9 | | 110. | 51 bis < 54 | 16 |
| 74. | 45 bis < 48 | 10 | | 111. | 51 bis < 54 | 17 |
| 75. | 45 bis < 48 | 11 | | 112. | 51 bis < 54 | 18 |
| 76. | 45 bis < 48 | 12 | | 113. | 51 bis < 54 | 19 |
| 77. | 45 bis < 48 | 13 | | 114. | 51 bis < 54 | 20 + |
| 78. | 45 bis < 48 | 14 | | 115. | 54 bis < 57 | - 5 |
| 79. | 45 bis < 48 | 15 | | 116. | 54 bis < 57 | 6 |
| 80. | 45 bis < 48 | 16 | | 117. | 54 bis < 57 | 7 |
| 81. | 45 bis < 48 | 17 | | 118. | 54 bis < 57 | 8 |
| 82. | 45 bis < 48 | 18 + | | 119. | 54 bis < 57 | 9 |
| 83. | 48 bis < 51 | - 5 | | 120. | 54 bis < 57 | 10 |
| 84. | 48 bis < 51 | 6 | | 121. | 54 bis < 57 | 11 |
| 85. | 48 bis < 51 | 7 | | 122. | 54 bis < 57 | 12 |
| 86. | 48 bis < 51 | 8 | | 123. | 54 bis < 57 | 13 |
| 87. | 48 bis < 51 | 9 | | 124. | 54 bis < 57 | 14 |
| 88. | 48 bis < 51 | 10 | | 125. | 54 bis < 57 | 15 |
| 89. | 48 bis < 51 | 11 | | 126. | 54 bis < 57 | 16 |
| 90. | 48 bis < 51 | 12 | | 127. | 54 bis < 57 | 17 |
| 91. | 48 bis < 51 | 13 | | 128. | 54 bis < 57 | 18 |
| 92. | 48 bis < 51 | 14 | | 129. | 54 bis < 57 | 19 |
| 93. | 48 bis < 51 | 15 | | 130. | 54 bis < 57 | 20 + |
| 94. | 48 bis < 51 | 16 | | 131. | 57 bis < 60 | - 5 |
| 95. | 48 bis < 51 | 17 | | 132. | 57 bis < 60 | 6 |
| 96. | 48 bis < 51 | 18 | | 133. | 57 bis < 60 | 7 |
| 97. | 48 bis < 51 | 19 | | 134. | 57 bis < 60 | 8 |
| 98. | 48 bis < 51 | 20 + | | 135. | 57 bis < 60 | 9 |
| 99. | 51 bis < 54 | - 5 | | 136. | 57 bis < 60 | 10 |
| 100. | 51 bis < 54 | 6 | | 137. | 57 bis < 60 | 11 |
| 101. | 51 bis < 54 | 7 | | 138. | 57 bis < 60 | 12 |

| Nr. | Hunter L | Hunter b | | Nr. | Hunter L | Hunter b |
|---|---|---|---|---|---|---|
| 139. | 57 bis < 60 | 13 | | 175. | 63 bis < 66 | 17 |
| 140. | 57 bis < 60 | 14 | | 176. | 63 bis < 66 | 18 |
| 141. | 57 bis < 60 | 15 | | 177. | 63 bis < 66 | 19 |
| 142. | 57 bis < 60 | 16 | | 178. | 63 bis < 66 | 20 + |
| 143. | 57 bis < 60 | 17 | | 179. | 66 bis < 69 | - 5 |
| 144. | 57 bis < 60 | 18 | | 180. | 66 bis < 69 | 6 |
| 145. | 57 bis < 60 | 19 | | 181. | 66 bis < 69 | 7 |
| 146. | 57 bis < 60 | 20 + | | 182. | 66 bis < 69 | 8 |
| 147. | 60 bis < 63 | - 5 | | 183. | 66 bis < 69 | 9 |
| 148. | 60 bis < 63 | 6 | | 184. | 66 bis < 69 | 10 |
| 149. | 60 bis < 63 | 7 | | 185. | 66 bis < 69 | 11 |
| 150. | 60 bis < 63 | 8 | | 186. | 66 bis < 69 | 12 |
| 151. | 60 bis < 63 | 9 | | 187. | 66 bis < 69 | 13 |
| 152. | 60 bis < 63 | 10 | | 188. | 66 bis < 69 | 14 |
| 153. | 60 bis < 63 | 11 | | 189. | 66 bis < 69 | 15 |
| 154. | 60 bis < 63 | 12 | | 190. | 66 bis < 69 | 16 |
| 155. | 60 bis < 63 | 13 | | 191. | 66 bis < 69 | 17 |
| 156. | 60 bis < 63 | 14 | | 192. | 66 bis < 69 | 18 |
| 157. | 60 bis < 63 | 15 | | 193. | 66 bis < 69 | 19 |
| 158. | 60 bis < 63 | 16 | | 194. | 66 bis < 69 | 20 + |
| 159. | 60 bis < 63 | 17 | | 195. | ≥ 69 | - 5 |
| 160. | 60 bis < 63 | 18 | | 196. | ≥ 69 | 6 |
| 161. | 60 bis < 63 | 19 | | 197. | ≥ 69 | 7 |
| 162. | 60 bis < 63 | 20 + | | 198. | ≥ 69 | 8 |
| 163. | 63 bis < 66 | - 5 | | 199. | ≥ 69 | 9 |
| 164. | 63 bis < 66 | 6 | | 200. | ≥ 69 | 10 |
| 165. | 63 bis < 66 | 7 | | 201. | ≥ 69 | 11 |
| 166. | 63 bis < 66 | 8 | | 202. | ≥ 69 | 12 |
| 167. | 63 bis < 66 | 9 | | 203. | ≥ 69 | 13 |
| 168. | 63 bis < 66 | 10 | | 204. | ≥ 69 | 14 |
| 169. | 63 bis < 66 | 11 | | 205. | ≥ 69 | 15 |
| 170. | 63 bis < 66 | 12 | | 206. | ≥ 69 | 16 |
| 171. | 63 bis < 66 | 13 | | 207. | ≥ 69 | 17 |
| 172. | 63 bis < 66 | 14 | | 208. | ≥ 69 | 18 |
| 173. | 63 bis < 66 | 15 | | 209. | ≥ 69 | 19 |
| 174. | 63 bis < 66 | 16 | | 210 | ≥ 69 | 20 + |

wobei die Bezeichnung -5 weniger als 5 aber mehr als 4 bedeutet und die Bezeichnung 12+ mehr als 12 aber weniger als 13 bedeutet.

**14.** Verfahren nach einem der Ansprüche 1 bis 7, wobei:

Schritt (a) die Zusammenstellung von mindestens einem Bereich eines Farbfaktors, dessen Wert im wesentlichen der des Farbfaktors Hunter L ist, enthält, wobei wenigstens ein Bereich im wesentlichen wie folgt aus den Bereichen ausgewählt ist:
Hunter L = <27, 27 bis <30, 30 bis <33, 33 bis <36, 36 bis <39, 39 bis <42, 42 bis <45, 45 bis <48, 48 bis <51, 51 bis <54, 54 bis <57, 57 bis <60, 60 bis <63, 63 bis <66, 66 bis <69, und >69.

**15.** Verfahren nach einem der vorhergehenden Ansprüche, enthaltend:

(c) Messung eines Wertes des erwähnten Helligkeitsmaßes bei der Hautfärbung der Testperson mit einem Farbmeßinstrument, und

(d) Messung eines Wertes des erwähnten Farbfaktors bei der Hautfärbung der Testperson.

**16.** Verfahren nach Anspruch 15, wobei das erwähnte Helligkeitsmaß ein Helligkeits-Farbfaktor ist, der von der Helligkeit der Färbung der erwähnten Haut der Testperson abhängt, Bereiche von Werten des erwähnten Helligkeits-Farbfaktors die erwähnten Helligkeits-Messungs-Wertebereiche darstellen, der erwähnte Farbfaktor von Schritt (b) vom relativen Gehalt entgegengesetzter Farben bei der Färbung der erwähnten Haut der Testperson abhängig ist, wobei wenigstens einer der Schritte (a) und (b) das Erreichen eines gemessenen Wertes enthält, der mit einem nachgewiesenermaßen nützlichen Maß Maß der Färbung korreliert.

**17.** Verfahren nach einem der Ansprüche 15 oder 16, wobei:

Schritt (a) die Zusammenstellung einer Gruppe von Helligkeits-Meßwertbereichen enthält, die bei der Färbung der Hautfarbe von von den Testpersonen verschiedenen Personen gefunden wurden;

Schritt (b) die Assoziation eines Bereichs von Werten der erwähnten Farbfaktoren in der Färbung von von den Testpersonen verschiedenen Personen mit jedem Helligkeitsmeßwertbereich enthält, das Verfahren weiter den Vergleich der gemessenen Werte des erwähnten Helligkeitsmaßes und des erwähnten Farbfaktors mit den erwähnten Bereichen des Werte des erwähnten Helligkeitsmaßes und dem Farbfaktor, die bei der Färbung von von den besagten Testpersonen verschiedenen Personen gefunden wurden, wie sie in Schritt (a) und (b) zusammengestellt und eingeordnet wurden, enthält, um zu bestimmen, wo innerhalb des erwähnten Bereiches die gemessenen Werte liegen.

**18.** Verfahren nach einem der Ansprüche 15 bis 17, wobei der erwähnte Farbfaktor vom relativen Gehalt von blau und gelb in der erwähnten Färbung abhängig ist.

**19.** Verfahren nach einem der Ansprüche 15 bis 17, wobei der erwähnte Farbfaktor vom relativen Gehalt von rot und grün in der erwähnten Färbung abhängig ist.

**20.** Verfahren nach einem der Ansprüche 15 bis 17, wobei jeder der Schritte (c) und (d) die Messung von Werten des erwähnten Helligkeitsmaßes und des erwähnten Farbfaktors an verschiedenen Stellen der Testperson enthält.

**21.** Verfahren nach einem der Ansprüche 15 bis 19, wobei jeder der Schritte (c) und (d) die Erstellung einer Gruppe von Mehrfachmessungen der Werte von jedem der erwähnten Farbfaktoren des besagten Helligkeitsmaßesund die Mittelung jeder Gruppe der Mehrfachmessungen enthält.

**22.** Vorrichtung zur Bewertung eines medizinischen oder nicht kosmetischen, physiologischen Zustandes einer menschlichen oder tierischen Testperson, wobei der Zustand mit einer symptomatischen nachweisbaren Hautfärbung verbunden ist, enthaltend:

(a) Mittel für die Zusammenstellung einer Gruppe von Helligkeits-Meßwertbereichen, und

(b) Mittel für die Assoziation eines Wertes oder eines Bereiches von Werten eines Farbfaktors mit jeden Helligkeits-Meßwertbereich zur Verwendung im Vergleich mit einer Messung des Wertes des Farbfaktors bei der Färbung einer Testperson, die eine gemessene Helligkeit in dem erwähnten Bereich hat, mit der Vorgabe, daß die Vorrichtung nicht für die Bestimmung eines hautfärbenden Kosmetikums für die Testperson verwendet wird.

**23.** Vorrichtung nach Anspruch 22, wobei der Zustand ein bei menschlichen kindlichen oder erwachsenen Testpersonen Gelbsucht verursachender Zustand ist.

**24.** Vorrichtung nach Anspruch 22, wobei der Zustand einer von Hyperbilirubinemia, Hypertension, Tuberkulose oder Leberstörung ist.

25. Vorrichtung nach einem der Ansprüche 22 bis 24, wobei die Vorrichtung für die Bewertung eines Zustandes eines menschlichen Kleinkindes angepaßt ist.

26. Vorrichtung nach einem der Ansprüche 22 bis 25, wobei die Mittel für die Zusammenstellung Mittel für die Einordnung der Gruppe der Helligkeits-Meßwertbereiche in mit einem Computer bearbeitbarer Form enthält, und die Mittel für die Assoziation Mittel der Farbfaktorwerte mit jedem Helligkeits-Meßwertbereich in der erwähnten mit einem Computer bearbeitbaren Form enthält.

27. Vorrichtung nach einem der Ansprüche 22 bis 26, wobei die Mittel für die Assoziation Mittel für die Assoziation eines Wertes des Farbfaktors Hunter b mit jedem Helligkeits-Meßwertbereich enthält.

28. Vorrichtung nach einem der Ansprüche 22 bis 27, wobei die Mittel für die Zusammenstellung Mittel für die Zusammenstellung einer Gruppe von Werten der Helligkeitsmessung Hunter L enthalten.

29. Vorrichtung nach einem der Ansprüche 22 bis 26, wobei die Mittel für die Zusammenstellung Mittel für die Zusammenstellung einer Gruppe von Helligkeits-Farbfaktor-Meßwerten enthalten, die im wesentlichen die des Farbfaktors Hunter L sind, und die Mittel für die Assoziation Mittel für die Assoziation eines Wertes des Farbfaktors, der im wesentlichen der des Farbfaktors Hunter a ist, mit jedem Helligkeits-Farbfaktor-Meßwertbereich enthalten, wobei das Verhältnis zwischen den erwähnten Helligkeits-Farbfaktor-Meßwertbereichen und den erwähnten assoziierten Farbfaktorwerten im wesentlichen wie folgt ist:

| Hunter L | Hunter a |
|---|---|
| 24 oder weniger 44 | 4 bis 18 |
| 45 bis 54 bis | 18 |
| 55 bis 59 bis | 25 |
| 60 bis 71 oder mehr | 8 bis 30. |

30. Vorrichtung nach einem der Ansprüche 22 bis 26, wobei die Mittel für die Assoziation Mittel für die Assoziation eines Wertes eines vergilbungsabhängigen Farbfaktors mit jedem Helligkeits-Meßwertbereich enthalten.

31. Vorrichtung nach einem der Ansprüche 22 bis 26, wobei der Farbfaktor Hunter b ist und die Helligkeits-Meßwertbereiche Bereiche von Hunter L sind, wobei die Vorrichtung weiter Mittel zur Messung des Hunter L-Wertes der Hautfärbung der Testperson mit einem Instrument enthält.

32. Vorrichtung nach einem der Ansprüche 22 bis 28, wobei die erwähnten Wertebereiche durch Bereiche der Hunter L-Werte bestimmt werden; die erwähnten Bereiche der Hunter L-Werte mit wenigstens einem der Hunter L-Werte im wesentlichen wie folgt verbunden sind:

Hunter L 27, 30, 33, 36, 39, 42, 45, 48, 51, 54, 57, 60,63,66 und 69.

33. Vorrichtung nach einem der Ansprüche 22 bis 28, wobei die Färbung die Hautfärbung und der Zustand Hyperbilirubinemia ist, die Mittel für die Zusammenstellung einer Gruppe von Helligkeits-Meßwertbereichen Mittel für die Zusammenstellung von Bereichen, die im wesentlichen Bereiche der Hunter L-Werte sind, enthalten, und die Mittel für die Assoziation eines Wertes eines Farbfaktors mit jedem Helligkeits-Meßwertbereich Mittel für die Assoziation eines Bereiches von Werten, die im wesentlichen die von Hunter b sind, mit jedem Helligkeits-Meßwertbereich enthalten, und die Vorrichtung weiter enthält:

(c) Mittel zur Messung eines Wertes des ersten Farbfaktors, der im wesentlichen der des Hunter b bei der Hautfärbung der Testperson ist, mit einem Farbmeßinstrument;

(d) Mittel zur Messung eines Wertes eines zweiten Farbfaktors, der im wesentlichen der des Hunter L bei der erwähnten Hautfärbung des Testobjektes ist, mit einem Farbmeßinstrument;

(e) Mittel für den Vergleich des gemessenen zweiten Farbfaktors mit Bereichen, die mit den Mitteln für die Zusammenstellung von Bereichen, die im wesentlichen Bereiche des Hunter L sind, enthalten, um eine vergleichbare Helligkeit bei der Färbung der Testperson und den zusammengestellten Bereichen einzugrenzen;

und

(f) Mittel für den Vergleich des gemessenen ersten Farbfaktorwertes, der im wesentlichen der des Hunter b ist, mit einem Wertebereich des Farbfaktors, der mit den Mitteln zur Assoziation eines Wertes, der im wesentlichen der des Hunters b ist, mit den vergleichbaren zusammengestellten Helligkeits-Meßwertbereichen, um zu bestimmen, ob der gemessene Wert des ersten Farbfaktors innerhalb oder außerhalb des assoziierten Bereichs der ersten Farbfaktorwerte liegt; wobei der erwähnte Bereich der assoziierten Werte im wesentlichen aus Bereichen wie folgt ausgewählt ist:

| Nr. | Hunter L | Hunter b |
|-----|----------|----------|
| 1. | < 27 | - 5 |
| 2. | < 27 | 6 |
| 3. | < 27 | 7 |
| 4. | < 27 | 8 |
| 5. | < 27 | 9 |
| 6. | < 27 | 10 |
| 7. | < 27 | 11 |
| 8. | < 27 | 12 + |
| 9. | 27 bis < 30 | - 5 |
| 10. | 27 bis < 30 | 6 |
| 11. | 27 bis < 30 | 7 |
| 12. | 27 bis < 30 | 8 |
| 13. | 27 bis < 30 | 9 |
| 14. | 27 bis < 30 | 10 |
| 15. | 27 bis < 30 | 11 |
| 16. | 27 bis < 30 | 12 + |
| 17. | 30 bis < 33 | - 5 |
| 18. | 30 bis < 33 | 6 |
| 19. | 30 bis < 33 | 7 |
| 20. | 30 bis < 33 | 8 |
| 21. | 30 bis < 33 | 9 |
| 22. | 30 bis < 33 | 10 |
| 23. | 30 bis < 33 | 11 |
| 24. | 30 bis < 33 | 12 + |
| 25. | 33 bis < 36 | - 5 |
| 26. | 33 bis < 36 | 6 |
| 27. | 33 bis < 36 | 7 |
| 28. | 33 bis < 36 | 8 |
| 29. | 33 bis < 36 | 9 |
| 30. | 33 bis < 36 | 10 |
| 31. | 33 bis < 36 | 11 |
| 32. | 33 bis < 36 | 12 + |

| Nr. | Hunter L | Hunter b |
|-----|----------|----------|
| 33. | 36 bis < 39 | - 5 |
| 34. | 36 bis < 39 | 6 |
| 35. | 36 bis < 39 | 7 |
| 36. | 36 bis < 39 | 8 |
| 37. | 36 bis < 39 | 9 |
| 38. | 36 bis < 39 | 10 |
| 39. | 36 bis < 39 | 11 |
| 40. | 36 bis < 39 | 12 |
| 41. | 36 bis < 39 | 13 |
| 42. | 36 bis < 39 | 14 |
| 43. | 36 bis < 39 | 15 + |
| 44. | 39 bis < 42 | - 5 |
| 45. | 39 bis < 42 | 6 |
| 46. | 39 bis < 42 | 7 |
| 47. | 39 bis < 42 | 8 |
| 48. | 39 bis < 42 | 9 |
| 49. | 39 bis < 42 | 10 |
| 50. | 39 bis < 42 | 11 |
| 51. | 39 bis < 42 | 12 |
| 52. | 39 bis < 42 | 13 |
| 53. | 39 bis < 42 | 14 |
| 54. | 39 bis < 42 | 15 + |
| 55. | 42 bis < 45 | - 5 |
| 56. | 42 bis < 45 | 6 |
| 57. | 42 bis < 45 | 7 |
| 58. | 42 bis < 45 | 8 |
| 59. | 42 bis < 45 | 9 |
| 60. | 42 bis < 45 | 10 |
| 61. | 42 bis < 45 | 11 |
| 62. | 42 bis < 45 | 12 |
| 63. | 42 bis < 45 | 13 |
| 64. | 42 bis < 45 | 14 |

| Nr. | Hunter L | Hunter b |
|------|-------------|--------|
| 65. | 42 bis < 45 | 15 |
| 66. | 42 bis < 45 | 16 |
| 67. | 42 bis < 45 | 17 |
| 68. | 42 bis < 45 | 18 + |
| 69. | 45 bis < 48 | - 5 |
| 70. | 45 bis < 48 | 6 |
| 71. | 45 bis < 48 | 7 |
| 72. | 45 bis < 48 | 8 |
| 73. | 45 bis < 48 | 9 |
| 74. | 45 bis < 48 | 10 |
| 75. | 45 bis < 48 | 11 |
| 76. | 45 bis < 48 | 12 |
| 77. | 45 bis < 48 | 13 |
| 78. | 45 bis < 48 | 14 |
| 79. | 45 bis < 48 | 15 |
| 80. | 45 bis < 48 | 16 |
| 81. | 45 bis < 48 | 17 |
| 82. | 45 bis < 48 | 18 + |
| 83. | 48 bis < 51 | - 5 |
| 84. | 48 bis < 51 | 6 |
| 85. | 48 bis < 51 | 7 |
| 86. | 48 bis < 51 | 8 |
| 87. | 48 bis < 51 | 9 |
| 88. | 48 bis < 51 | 10 |
| 89. | 48 bis < 51 | 11 |
| 90. | 48 bis < 51 | 12 |
| 91. | 48 bis < 51 | 13 |
| 92. | 48 bis < 51 | 14 |
| 93. | 48 bis < 51 | 15 |
| 94. | 48 bis < 51 | 16 |
| 95. | 48 bis < 51 | 17 |
| 96. | 48 bis < 51 | 18 |
| 97. | 48 bis < 51 | 19 |
| 98. | 48 bis < 51 | 20 + |
| 99. | 51 bis < 54 | - 5 |
| 100. | 51 bis < 54 | 6 |
| 101. | 51 bis < 54 | 7 |

| Nr. | Hunter L | Hunter b |
|------|-------------|--------|
| 102. | 51 bis < 54 | 8 |
| 103. | 51 bis < 54 | 9 |
| 104. | 51 bis < 54 | 10 |
| 105. | 51 bis < 54 | 11 |
| 106. | 51 bis < 54 | 12 |
| 107. | 51 bis < 54 | 13 |
| 108. | 51 bis < 54 | 14 |
| 109. | 51 bis < 54 | 15 |
| 110. | 51 bis < 54 | 16 |
| 111. | 51 bis < 54 | 17 |
| 112. | 51 bis < 54 | 18 |
| 113. | 51 bis < 54 | 19 |
| 114. | 51 bis < 54 | 20 + |
| 115. | 51 bis < 54 | - 5 |
| 116. | 54 bis < 57 | 6 |
| 117. | 54 bis < 57 | 7 |
| 118. | 54 bis < 57 | 8 |
| 119. | 54 bis < 57 | 9 |
| 120. | 54 bis < 57 | 10 |
| 121. | 54 bis < 57 | 11 |
| 122. | 54 bis < 57 | 12 |
| 123. | 54 bis < 57 | 13 |
| 124. | 54 bis < 57 | 14 |
| 125. | 54 bis < 57 | 15 |
| 126. | 54 bis < 57 | 16 |
| 127. | 54 bis < 57 | 17 |
| 128. | 54 bis < 57 | 18 |
| 129. | 54 bis < 57 | 19 |
| 130. | 54 bis < 57 | 20 + |
| 131. | 57 bis < 60 | - 5 |
| 132. | 57 bis < 60 | 6 |
| 133. | 57 bis < 60 | 7 |
| 134. | 57 bis < 60 | 8 |
| 135. | 57 bis < 60 | 9 |
| 136. | 57 bis < 60 | 10 |
| 137. | 57 bis < 60 | 11 |
| 138. | 57 bis < 60 | 12 |

| Nr. | Hunter L | Hunter b | | Nr. | Hunter L | Hunter b |
|-----|----------|----------|--|-----|----------|----------|
| 139. | 57 bis < 60 | 13 | | 175. | 63 bis < 66 | 17 |
| 140. | 57 bis < 60 | 14 | | 176. | 63 bis < 66 | 18 |
| 141. | 57 bis < 60 | 15 | | 177. | 63 bis < 66 | 19 |
| 142. | 57 bis < 60 | 16 | | 178. | 63 bis < 66 | 20 + |
| 143. | 57 bis < 60 | 17 | | 179. | 66 bis < 69 | - 5 |
| 144. | 57 bis < 60 | 18 | | 180. | 66 bis < 69 | 6 |
| 145. | 57 bis < 60 | 19 | | 181. | 66 bis < 69 | 7 |
| 146. | 57 bis < 60 | 20 + | | 182. | 66 bis < 69 | 8 |
| 147. | 60 bis < 63 | - 5 | | 183. | 66 bis < 69 | 9 |
| 148. | 60 bis < 63 | 6 | | 184. | 66 bis < 69 | 10 |
| 149. | 60 bis < 63 | 7 | | 185. | 66 bis < 69 | 11 |
| 150. | 60 bis < 63 | 8 | | 186. | 66 bis < 69 | 12 |
| 151. | 60 bis < 63 | 9 | | 187. | 66 bis < 69 | 13 |
| 152. | 60 bis < 63 | 10 | | 188. | 66 bis < 69 | 14 |
| 153. | 60 bis < 63 | 11 | | 189. | 66 bis < 69 | 15 |
| 154. | 60 bis < 63 | 12 | | 190. | 66 bis < 69 | 16 |
| 155. | 60 bis < 63 | 13 | | 191. | 66 bis < 69 | 17 |
| 156. | 60 bis < 63 | 14 | | 192. | 66 bis < 69 | 18 |
| 157. | 60 bis < 63 | 15 | | 193. | 66 bis < 69 | 19 |
| 158. | 60 bis < 63 | 16 | | 194. | 66 bis < 69 | 20 + |
| 159. | 60 bis < 63 | 17 | | 195. | ≥ 69 | - 5 |
| 160. | 60 bis < 63 | 18 | | 196. | ≥ 69 | 6 |
| 161. | 60 bis < 63 | 19 | | 197. | ≥ 69 | 7 |
| 162. | 60 bis < 63 | 20 + | | 198. | ≥ 69 | 8 |
| 163. | 63 bis < 66 | - 5 | | 199. | ≥ 69 | 9 |
| 164. | 63 bis < 66 | 6 | | 200. | ≥ 69 | 10 |
| 165. | 63 bis < 66 | 7 | | 201. | ≥ 69 | 11 |
| 166. | 63 bis < 66 | 8 | | 202. | ≥ 69 | 12 |
| 167. | 63 bis < 66 | 9 | | 203. | ≥ 69 | 13 |
| 168. | 63 bis < 66 | 10 | | 204. | ≥ 69 | 14 |
| 169. | 63 bis < 66 | 11 | | 205. | ≥ 69 | 15 |
| 170. | 63 bis < 66 | 12 | | 206. | ≥ 69 | 16 |
| 171. | 63 bis < 66 | 13 | | 207. | ≥ 69 | 17 |
| 172. | 63 bis < 66 | 14 | | 208. | ≥ 69 | 18 |
| 173. | 63 bis < 66 | 15 | | 209. | ≥ 69 | 19 |
| 174. | 63 bis < 66 | 16 | | 210. | ≥ 69 | 20 + |

wobei die Bezeichnung -5 weniger als 5 aber mehr als 4 bedeutet und die Bezeichnung 12+ mehr als 12 aber weniger als 13 bedeutet.

**34.** Vorrichtung nach einem der Ansprüche 22 bis 28, wobei:

die Mittel für die Zusammenstellung einer Gruppe von Helligkeits-Meßwertbereichen Mittel für die Zusammenstellung von Bereichen eines Farbfaktors, dessen Wert im wesentlichen dem des Farbfaktors Hunter L entspricht, enthält,

die Mittel für die Assoziation eines Wertes eines Farbfaktors mit jedem Helligkeits-Meßwertbereich zur Verwendung für den Vergleich mit einer Messung des Wertes des Farbfaktors bei der Färbung der Testperson, die eine gemessene Helligkeit innerhalb des erwähnten Bereiches besitzt, Mittel für die Assoziation von Werten eines Farbfaktors, dessen Wert im wesentlichen der des Farbfaktors Hunter b ist, mit jedem Helligkeits-Meßwertbereich enthält, und das Verhältnis zwischen den erwähnten Helligkeits-Farbfaktor-Meßwertbereichen und den erwähnten assoziierten Farbfaktorwerten im wesentlichen wie folgt ist:

| Nr. | Hunter L | Hunter b |
|-----|----------|----------|
| 1. | < 27 | - 5 |
| 2. | < 27 | 6 |
| 3. | < 27 | 7 |
| 4. | < 27 | 8 |
| 5. | < 27 | 9 |
| 6. | < 27 | 10 |
| 7. | < 27 | 11 |
| 8. | < 27 | 12 + |
| 9. | 27 bis < 30 | - 5 |
| 10. | 27 bis < 30 | 6 |
| 11. | 27 bis < 30 | 7 |
| 12. | 27 bis < 30 | 8 |
| 13. | 27 bis < 30 | 9 |
| 14. | 27 bis < 30 | 10 |
| 15. | 27 bis < 30 | 11 |
| 16. | 27 bis < 30 | 12 + |
| 17. | 30 bis < 33 | - 5 |
| 18. | 30 bis < 33 | 6 |
| 19. | 30 bis < 33 | 7 |
| 20. | 30 bis < 33 | 8 |
| 21. | 30 bis < 33 | 9 |
| 22. | 30 bis < 33 | 10 |
| 23. | 30 bis < 33 | 11 |
| 24. | 30 bis < 33 | 12 + |
| 25. | 33 bis < 36 | - 5 |
| 26. | 33 bis < 36 | 6 |
| 27. | 33 bis < 36 | 7 |
| 28. | 33 bis < 36 | 8 |
| 29. | 33 bis < 36 | 9 |
| 30. | 33 bis < 36 | 10 |
| 31. | 33 bis < 36 | 11 |
| 32. | 33 bis < 36 | 12 + |

| Nr. | Hunter L | Hunter b |
|-----|----------|----------|
| 33. | 36 bis < 39 | - 5 |
| 34. | 36 bis < 39 | 6 |
| 35. | 36 bis < 39 | 7 |
| 36. | 36 bis < 39 | 8 |
| 37. | 36 bis < 39 | 9 |
| 38. | 36 bis < 39 | 10 |
| 39. | 36 bis < 39 | 11 |
| 40. | 36 bis < 39 | 12 |
| 41. | 36 bis < 39 | 13 |
| 42. | 36 bis < 39 | 14 |
| 43. | 36 bis < 39 | 15 + |
| 44. | 39 bis < 42 | - 5 |
| 45. | 39 bis < 42 | 6 |
| 46. | 39 bis < 42 | 7 |
| 47. | 39 bis < 42 | 8 |
| 48. | 39 bis < 42 | 9 |
| 49. | 39 bis < 42 | 10 |
| 50. | 39 bis < 42 | 11 |
| 51. | 39 bis < 42 | 12 |
| 52. | 39 bis < 42 | 13 |
| 53. | 39 bis < 42 | 14 |
| 54. | 39 bis < 42 | 15 + |
| 55. | 42 bis < 45 | - 5 |
| 56. | 42 bis < 45 | 6 |
| 57. | 42 bis < 45 | 7 |
| 58. | 42 bis < 45 | 8 |
| 59. | 42 bis < 45 | 9 |
| 60. | 42 bis < 45 | 10 |
| 61. | 42 bis < 45 | 11 |
| 62. | 42 bis < 45 | 12 |
| 63. | 42 bis < 45 | 13 |
| 64. | 42 bis < 45 | 14 |

| Nr. | Hunter L | Hunter b |
|------|------------|----------|
| 65. | 42 bis < 45 | 15 |
| 66. | 42 bis < 45 | 16 |
| 67. | 42 bis < 45 | 17 |
| 68. | 42 bis < 45 | 18 + |
| 69. | 45 bis < 48 | - 5 |
| 70. | 45 bis < 48 | 6 |
| 71. | 45 bis < 48 | 7 |
| 72. | 45 bis < 48 | 8 |
| 73. | 45 bis < 48 | 9 |
| 74. | 45 bis < 48 | 10 |
| 75. | 45 bis < 48 | 11 |
| 76. | 45 bis < 48 | 12 |
| 77. | 45 bis < 48 | 13 |
| 78. | 45 bis < 48 | 14 |
| 79. | 45 bis < 48 | 15 |
| 80. | 45 bis < 48 | 16 |
| 81. | 45 bis < 48 | 17 |
| 82. | 45 bis < 48 | 18 + |
| 83. | 48 bis < 51 | - 5 |
| 84. | 48 bis < 51 | 6 |
| 85. | 48 bis < 51 | 7 |
| 86. | 48 bis < 51 | 8 |
| 87. | 48 bis < 51 | 9 |
| 88. | 48 bis < 51 | 10 |
| 89. | 48 bis < 51 | 11 |
| 90. | 48 bis < 51 | 12 |
| 91. | 48 bis < 51 | 13 |
| 92. | 48 bis < 51 | 14 |
| 93. | 48 bis < 51 | 15 |
| 94. | 48 bis < 51 | 16 |
| 95. | 48 bis < 51 | 17 |
| 96. | 48 bis < 51 | 18 |
| 97. | 48 bis < 51 | 19 |
| 98. | 48 bis < 51 | 20 + |
| 99. | 51 bis < 54 | - 5 |
| 100. | 51 bis < 54 | 6 |
| 101. | 51 bis < 54 | 7 |

| Nr. | Hunter L | Hunter b |
|------|------------|----------|
| 102. | 51 bis < 54 | 8 |
| 103. | 51 bis < 54 | 9 |
| 104. | 51 bis < 54 | 10 |
| 105. | 51 bis < 54 | 11 |
| 106. | 51 bis < 54 | 12 |
| 107. | 51 bis < 54 | 13 |
| 108. | 51 bis < 54 | 14 |
| 109. | 51 bis < 54 | 15 |
| 110. | 51 bis < 54 | 16 |
| 111. | 51 bis < 54 | 17 |
| 112. | 51 bis < 54 | 18. |
| 113. | 51 bis < 54 | 19 |
| 114. | 51 bis < 54 | 20 + |
| 115. | 54 bis < 57 | - 5 |
| 116. | 54 bis < 57 | 6 |
| 117. | 54 bis < 57 | 7 |
| 118. | 54 bis < 57 | 8 |
| 119. | 54 bis < 57 | 9 |
| 120. | 54 bis < 57 | 10 |
| 121. | 54 bis < 57 | 11 |
| 122. | 54 bis < 57 | 12 |
| 123. | 54 bis < 57 | 13 |
| 124. | 54 bis < 57 | 14 |
| 125. | 54 bis < 57 | 15 |
| 126. | 54 bis < 57 | 16 |
| 127. | 54 bis < 57 | 17 |
| 128. | 54 bis < 57 | 18 |
| 129. | 54 bis < 57 | 19 |
| 130. | 54 bis < 57 | 20 + |
| 131. | 57 bis < 60 | - 5 |
| 132. | 57 bis < 60 | 6 |
| 133. | 57 bis < 60 | 7 |
| 134. | 57 bis < 60 | 8 |
| 135. | 57 bis < 60 | 9 |
| 136. | 57 bis < 60 | 10 |
| 137. | 57 bis < 60 | 11 |
| 138. | 57 bis < 60 | 12 |

| Nr | Hunter L | Hunter b |
|---|---|---|
| 139. | 57 bis < 60 | 13 |
| 140. | 57 bis < 60 | 14 |
| 141. | 57 bis < 60 | 15 |
| 142. | 57 bis < 60 | 16 |
| 143. | 57 bis < 60 | 17 |
| 144. | 57 bis < 60 | 18 |
| 145. | 57 bis < 60 | 19 |
| 146. | 57 bis < 60 | 20 + |
| 147. | 60 bis < 63 | - 5 |
| 148. | 60 bis < 63 | 6 |
| 149. | 60 bis < 63 | 7 |
| 150. | 60 bis < 63 | 8 |
| 151. | 60 bis < 63 | 9 |
| 152. | 60 bis < 63 | 10 |
| 153. | 60 bis < 63 | 11 |
| 154. | 60 bis < 63 | 12 |
| 155. | 60 bis < 63 | 13 |
| 156. | 60 bis < 63 | 14 |
| 157. | 60 bis < 63 | 15 |
| 158. | 60 bis < 63 | 16 |
| 159. | 60 bis < 63 | 17 |
| 160. | 60 bis < 63 | 18 |
| 161. | 60 bis < 63 | 19 |
| 162. | 60 bis < 63 | 20 + |
| 163. | 63 bis < 66 | - 5 |
| 164. | 63 bis < 66 | 6 |
| 165. | 63 bis < 66 | 7 |
| 166. | 63 bis < 66 | 8 |
| 167. | 63 bis < 66 | 9 |
| 168. | 63 bis < 66 | 10 |
| 169. | 63 bis < 66 | 11 |
| 170. | 63 bis < 66 | 12 |
| 171. | 63 bis < 66 | 13 |
| 172. | 63 bis < 66 | 14 |
| 173. | 63 bis < 66 | 15 |
| 174. | 63 bis < 66 | 16 |

| Nr. | Hunter L | Hunter b |
|---|---|---|
| 175. | 63 bis < 66 | 17 |
| 176. | 63 bis < 66 | 18 |
| 177. | 63 bis < 66 | 19 |
| 178. | 63 bis < 66 | 20 + |
| 179. | 66 bis < 69 | - 5 |
| 180. | 66 bis < 69 | 6 |
| 181. | 66 bis < 69 | 7 |
| 182. | 66 bis < 69 | 8 |
| 183. | 66 bis < 69 | 9 |
| 184. | 66 bis < 69 | 10 |
| 185. | 66 bis < 69 | 11 |
| 186. | 66 bis < 69 | 12 |
| 187. | 66 bis < 69 | 13 |
| 188. | 66 bis < 69 | 14 |
| 189. | 66 bis < 69 | 15 |
| 190. | 66 bis < 69 | 16 |
| 191. | 66 bis < 69 | 17 |
| 192. | 66 bis < 69 | 18 |
| 193. | 66 bis < 69 | 19 |
| 194. | 66 bis < 69 | 20 + |
| 195. | ≥ 69 | - 5 |
| 196. | ≥ 69 | 6 |
| 197. | ≥ 69 | 7 |
| 198. | ≥ 69 | 8 |
| 199. | ≥ 69 | 9 |
| 200. | ≥ 69 | 10 |
| 201. | ≥ 69 | 11 |
| 202. | ≥ 69 | 12 |
| 203. | ≥ 69 | 13 |
| 204. | ≥ 69 | 14 |
| 205. | ≥ 69 | 15 |
| 206. | ≥ 69 | 16 |
| 207. | ≥ 69 | 17 |
| 208. | ≥ 69 | 18 |
| 209. | ≥ 69 | 19 |
| 210. | ≥ 69 | 20 + |

wobei die Bezeichnung -5 weniger als 5 aber mehr als 4 bedeutet und die Bezeichnung 12+ mehr als 12 aber weniger als 13 bedeutet.

**35.** Vorrichtung nach einem der Ansprüche 22 bis 28, wobei:

die Mittel für die Zusammenstellung Mittel für die Zusammenstellung von mindestens einem Bereich eine Farbfaktors, dessen Wert im wesentlichen der des Farbfaktors Hunter L ist, enthält, wobei der erwähnte mindestens eine Bereich im wesentlichen wie folgt aus den Bereichen ausgewählt ist:
Hunter L=<27, 27 bis <30, 30 bis <33, 33 bis <36, 36 bis <39, 39 bis <42, 42 bis <45, 45 bis <48, 48 bis <51, 51 bis <54, 54 bis <57, 57 bis <60, 60 bis <63, 63 bis <66, 66 bis <69, und >69.

**36.** Vorrichtung nach einem der Ansprüche 22 bis 35, weiter enthaltend:

(c) Mittel zur Messung (i) eines Wertes des erwähnten Helligkeitsmaßes bei der Färbung einer Testperson mit einem Farbmeßinstrument, und (ii) einen Wert des erwähnten Farbfaktors von (b) bei der erwähnten Färbung der Testperson.

**37.** Vorrichtung nach Anspruch 36, wobei das erwähnte Helligkeitsmaß ein Helligkeits-Farbfaktor ist, der von der Helligkeit der Färbung der erwähnten Testperson abhängig ist, die Bereiche der Werte des erwähnten Helligkeits-Farbfaktors die erwähnten Helligkeits-Messungswertebereiche darstellen, der erwähnte Farbfaktor von (b) vom relativen Gehalt der entgegengesetzten Farben bei der Färbung der erwähnten Testperson abhängig ist, wobei wenigstens eine der Farbfaktoren einen gemessenen Wert enthält, der mit einer nachgewiesenermaßen nützlichen Messung der Färbung korreliert.

**38.** Vorrichtung nach Anspruch 36 oder 37, weiter enthaltend Mittel zum Vergleich der gemessenen Werte des erwähnten ersten Farbfaktors und des erwähnten zweiten Farbfaktors mit Wertebereichen der erwähnten Farbfaktoren, die bei der Färbung von von den erwähnten Testpersonen verschiedenen Personen gefunden wurden, wie sie durch die Mittel für die Zusammenstellung und die Mittel für die Assoziation zusammengestellt und eingeordnet wurden, um zu bestimmen, ob die gemessenen Werte der erwähnten Farbfaktoren in den erwähnten Bereichen liegen.

**39.** Vorrichtung nach einem der Ansprüche 36 bis 38, wobei die Mittel zur Messung Mittel zur Messung des Wertes eines Farbfaktors, der vom relativen Gehalt von Blau und Gelb bei der erwähnten Färbung abhängig ist, enthalten.

**40.** Vorrichtung nach einem der Ansprüche 36 bis 38, wobei die Mittel zur Messung Mittel zur Messung des Wertes eines Farbfaktors, der vom relativen Gehalt von Rot und Grün bei der erwähnten Färbung abhängig ist, enthalten.

**41.** Vorrichtung nach einem der Ansprüche 36 bis 40, wobei die Mittel zur Messung Mittel zur Messung der Werte des erwähnten ersten Farbfaktors und des erwähnten zweiten Farbfaktors an verschiedenen Stellen der Testperson, enthalten.

**42.** Vorrichtung nach einem der Ansprüche 36 bis 41, wobei die Mittel zur Messung Mittel zur Erstellung einer Gruppe von Mehrfachmessungen der Werte von jeden der erwähnten ersten Farbfaktoren und der erwähnten zweiten Farbfaktoren und zur Mitteilung jeder Gruppe der Mehrfachmessungen enthält.

**Revendications**

**1.** Processus de formation d'une compilation de valeurs de facteurs de couleur indicatives de l'état physiologique médical ou non cosmétique d'un sujet d'essai humain ou animal, lequel état est associé à une coloration de la peau symptomatique pouvant être détectée, le procédé comprenant les opérations suivantes :

(a) compiler un groupe d'intervalles de valeurs de mesures de luminance et
(b) associer à chaque intervalle de valeurs de mesures de luminance une valeur ou un intervalle de valeurs d'un facteur de couleur qui indique l'état d'un sujet d'essai dont la luminance a été mesurée à l'intérieur de l'intervalle de valeurs de mesures de luminance pour l'utiliser dans la comparaison avec la mesure de la valeur de ce facteur de couleur dans la coloration d'un sujet d'essai.

**2.** Procédé selon la revendication 1, où l'état est un état qui provoque la jaunisse chez un sujet d'essai humain jeune ou adulte.

**3.** Procédé selon la revendication 1, où l'état est l'un des suivants, à savoir l'hyperbilirubinémie, l'hypertension, la tuberculose ou une affection du foie.

**4.** Procédé selon l'une quelconque des revendications précédentes, où le sujet d'essai est un nourrisson humain.

**5.** Procédé selon l'une quelconque des revendications précédentes, où l'opération (a) comprend l'assemblage du groupe d'intervalles de mesures de luminance sous une forme pouvant être traitée par ordinateur, et l'opération (b) comprend l'association de la valeur de facteur de couleur avec chaque intervalle de valeurs de mesures de luminance sous ladite forme pouvant être traitée par ordinateur.

**6.** Procédé selon l'une quelconque des revendications précédentes, où l'opération (b) comprend l'association d'une valeur du facteur de couleur Hunter b avec chaque intervalle de valeurs de mesures de luminance.

**7.** Procédé selon l'une quelconque des revendications précédentes, où l'opération (a) comprend la compilation d'un groupe de valeurs de la mesure de luminance Hunter L.

**8.** Procédé selon l'une quelconque des revendications 1 à 5, où l'opération (a) comprend la compilation d'un groupe d'intervalles de valeurs de facteurs de couleur de mesures de luminance qui sont sensiblement ceux du facteur de couleur Hunter L, et l'opération (b) comprend l'association d'une valeur d'un facteur de couleur dont la valeur est sensiblement celle du facteur de couleur Hunter a avec chaque intervalle de valeurs de facteurs de couleur de mesures de luminance, la relation entre lesdits intervalles de valeurs de facteurs de couleur de mesures de luminance et lesdites valeurs de facteurs de couleur associées étant sensiblement la suivante :

| Hunter L | Hunter a |
| --- | --- |
| de 24 (ou moins) à 44 | de 4 à 16 |
| 45 à 54 à | 18 |
| 55 à 59 à | 25 |
| 60 à 71 (ou plus) | 6 à 30. |

**9.** Procédé selon l'une quelconque des revendications 1 à 5, où l'opération (b) comprend l'association, avec chaque intervalle de valeurs de mesures de luminance, d'une valeur d'un facteur de couleur dépendant du jaune.

**10.** Procédé selon l'une quelconque des revendications 1 à 7, où le facteur de couleur de l'opération (b) est Hunter b et les intervalles de valeurs de mesures de luminance de l'opération (a) sont les intervalles de Hunter L, et dans lequel la détermination de l'état peut comporter l'opération consistant à mesurer, au moyen d'un instrument, une valeur de Hunter L pour la coloration de la peau du sujet d'essai.

**11.** Procédé selon l'une quelconque des revendications 1 à 7, où lesdits intervalles de valeurs de l'opération (a) sont déterminés par les Intervalles de valeurs de Hunter L ; les intervalles de valeurs de Hunter L étant limités par au moins l'une des valeurs de Hunter L sensiblement suivantes
Hunter L = 27, 30, 33, 36, 39, 42, 45, 48, 51, 54, 57, 60, 63, 66 et 69

**12.** Procédé selon l'une quelconque des revendications 1 à 7, où l'état est l'hyperbilirubinémie, l'opération (a) comprend la compilation d'intervalles qui sont sensiblement des intervalles de valeurs de Hunter L, et l'opération (b) comprend l'association d'un intervalle de valeurs qui sont sensiblement celles de Hunter b avec chaque intervalle de valeurs de mesures de luminance, l'état du sujet d'essai étant déterminé par les opérations suivantes :

(c) mesurer, avec un instrument de mesure de couleur, la valeur d'un premier facteur de couleur qui est sensiblement celui de Hunter b dans la coloration de la peau du sujet d'essai ;
(d) mesurer, avec un instrument de mesure de couleur, la valeur d'un deuxième facteur de couleur qui est sensiblement celui de Hunter L dans ladite coloration de la peau du sujet d'essai ;
(e) comparer la valeur mesurée du deuxième facteur de couleur avec les intervalles compilés de l'opération (a) afin de localiser une luminance comparable dans la coloration du sujet d'essai et les intervalles compilés ; et
(f) comparer la valeur mesurée du premier facteur de couleur avec un intervalle de valeurs de premiers facteurs de couleur associé dans l'opération (b) avec l'intervalle de valeurs de mesures de luminance compilées comparables afin de déterminer si la valeur mesurée du premier facteur de couleur se trouve à l'intérieur ou à l'extérieur de l'intervalle associé des valeurs de premiers facteurs de couleur ; où on choisit l'intervalle associé de valeurs de premiers facteurs de couleur dans les intervalles sensiblement suivants :

| n° | Hunter L | Hunter b |
|----|----------|----------|
| 1. | <27 | -5 |
| 2. | <27 | 6 |
| 3. | <27 | 7 |
| 4. | <27 | 8 |
| 5. | <27 | 9 |
| 6. | <27 | 10 |
| 7. | <27 | 11 |
| 8. | <27 | 12+ |
| 9. | 27 à <30 | -5 |
| 10. | 27 à <30 | 6 |
| 11. | 27 à <30 | 7 |
| 12. | 27 à <30 | 8 |
| 13. | 27 à <30 | 9 |
| 14. | 27 à <30 | 10 |
| 15. | 27 à <30 | 11 |
| 16. | 27 à <30 | 12+ |
| 17. | 30 à <33 | -5 |
| 18. | 30 à <33 | 6 |
| 19. | 30 à <33 | 7 |
| 20. | 30 à <33 | 8 |
| 21. | 30 à <33 | 9 |
| 22. | 30 à <33 | 10 |
| 23. | 30 à <33 | 11 |
| 24. | 30 à <33 | 12+ |
| 25. | 33 à <36 | -5 |
| 26. | 33 à <36 | 6 |
| 27. | 33 à <36 | 7 |
| 28. | 33 à <36 | 8 |
| 29. | 33 à <36 | 9 |
| 30. | 33 à <36 | 10 |
| 31. | 33 à <36 | 11 |
| 32. | 33 à <36 | 12+ |

| n° | Hunter L | Hunter b |
|----|----------|----------|
| 33. | 36 à <39 | -5 |
| 34. | 36 à <39 | 6 |
| 35. | 36 à <39 | 7 |
| 36. | 36 à <39 | 8 |
| 37. | 36 à <39 | 9 |
| 38. | 36 à <39 | 10 |
| 39. | 36 à <39 | 11 |
| 40. | 36 à <39 | 12 |
| 41. | 36 à <39 | 13 |
| 42. | 36 à <39 | 14 |
| 43. | 36 à <39 | 15+ |
| 44. | 39 à <42 | -5 |
| 45. | 39 à <42 | 6 |
| 46. | 39 à <42 | 7 |
| 47. | 39 à <42 | 8 |
| 48. | 39 à <42 | 9 |
| 49. | 39 à <42 | 10 |
| 50. | 39 à <42 | 11 |
| 51. | 39 à <42 | 12 |
| 52. | 39 à <42 | 13 |
| 53. | 39 à <42 | 14 |
| 54. | 39 à <42 | 15+ |
| 55. | 42 à <45 | -5 |
| 56. | 42 à <45 | 6 |
| 57. | 42 à <45 | 7 |
| 58. | 42 à <45 | 8 |
| 59. | 42 à <45 | 9 |
| 60. | 42 à <45 | 10 |
| 61. | 42 à <45 | 11 |
| 62. | 42 à <45 | 12 |
| 63. | 42 à <45 | 13 |
| 64. | 42 à <45 | 14 |

| n° | Hunter L | Hunter b | | n° | Hunter L | Hunter b |
|---|---|---|---|---|---|---|
| 65. | 42 à <45 | 15 | | 98. | 48 à <51 | 20+ |
| 66. | 42 à <45 | 16 | | 99. | 51 à <54 | -5 |
| 67. | 42 à <45 | 17 | | 100. | 51 à <54 | 6 |
| 68. | 42 à <45 | 18+ | | 101. | 51 à <54 | 7 |
| 69. | 45 à <48 | -5 | | 102. | 51 à <54 | 8 |
| 70. | 45 à <48 | 6 | | 103. | 51 à <54 | 9 |
| 71. | 45 à <48 | 7 | | 104. | 51 à <54 | 10 |
| 72. | 45 à <48 | 8 | | 105. | 51 à <54 | 11 |
| 73. | 45 à <48 | 9 | | 106. | 51 à <54 | 12 |
| 74. | 45 à <48 | 10 | | 107. | 51 à <54 | 13 |
| 75. | 45 à <48 | 11 | | 108. | 51 à <54 | 14 |
| 76. | 45 à <48 | 12 | | 109. | 51 à <54 | 15 |
| 77. | 45 à <48 | 13 | | 110. | 51 à <54 | 16 |
| 78. | 45 à <48 | 14 | | 111. | 51 à <54 | 17 |
| 79. | 45 à <48 | 15 | | 112. | 51 à <54 | 18 |
| 80. | 45 à <48 | 16 | | 113. | 51 à <54 | 19 |
| 81. | 45 à <48 | 17 | | 114. | 51 à <54 | 20+ |
| 82. | 45 à <48 | 18+ | | 115. | 54 à <57 | -5 |
| 83. | 48 à <51 | -5 | | 116. | 54 à <57 | 6 |
| 84. | 48 à <51 | 6 | | 117. | 54 à <57 | 7 |
| 85. | 48 à <51 | 7 | | 118. | 54 à <57 | 8 |
| 86. | 48 à <51 | 8 | | 119. | 54 à <57 | 9 |
| 87. | 48 à <51 | 9 | | 120. | 54 à <57 | 10 |
| 88. | 48 à <51 | 10 | | 121. | 54 à <57 | 11 |
| 89. | 48 à <51 | 11 | | 122. | 54 à <57 | 12 |
| 90. | 48 à <51 | 12 | | 123. | 54 à <57 | 13 |
| 91. | 48 à <51 | 13 | | 124. | 54 à <57 | 14 |
| 92. | 48 à <51 | 14 | | 125. | 54 à <57 | 15 |
| 93. | 48 à <51 | 15 | | 126. | 54 à <57 | 16 |
| 94. | 48 à <51 | 16 | | 127. | 54 à <57 | 17 |
| 95. | 48 à <51 | 17 | | 128. | 54 à <57 | 18 |
| 96. | 48 à <51 | 18 | | 129. | 54 à <57 | 19 |
| 97. | 48 à <51 | 19 | | 130. | 54 à <57 | 20+ |

| n° | Hunter L | Hunter b |
|---|---|---|
| 131. | 57 à <60 | -5 |
| 132. | 57 à <60 | 6 |
| 133. | 57 à <60 | 7 |
| 134. | 57 à <60 | 8 |
| 135. | 57 à <60 | 9 |
| 136. | 57 à <60 | 10 |
| 137. | 57 à <60 | 11 |
| 138. | 57 à <60 | 12 |
| 139. | 57 à <60 | 13 |
| 140. | 57 à <60 | 14 |
| 141. | 57 à <60 | 15 |
| 142. | 57 à <60 | 16 |
| 143. | 57 à <60 | 17 |
| 144. | 57 à <60 | 18 |
| 145. | 57 à <60 | 19 |
| 146. | 57 à <60 | 20+ |
| 147. | 60 à <63 | -5 |
| 148. | 60 à <63 | 6 |
| 149. | 60 à <63 | 7 |
| 150. | 60 à <63 | 8 |
| 151. | 60 à <63 | 9 |
| 152. | 60 à <63 | 10 |
| 153. | 60 à <63 | 11 |
| 154. | 60 à <63 | 12 |
| 155. | 60 à <63 | 13 |
| 156. | 60 à <63 | 14 |
| 157. | 60 à <63 | 15 |
| 158. | 60 à <63 | 16 |
| 159. | 60 à <63 | 17 |
| 160. | 60 à <63 | 18 |
| 161. | 60 à <63 | 19 |
| 162. | 60 à <63 | 20+ |
| 163. | 63 à <66 | -5 |
| 164. | 63 à <66 | 6 |

| n° | Hunter L | Hunter b |
|---|---|---|
| 165. | 63 à <66 | 7 |
| 166. | 63 à <66 | 8 |
| 167. | 63 à <66 | 9 |
| 168. | 63 à <66 | 10 |
| 169. | 63 à <66 | 11 |
| 170. | 63 à <66 | 12 |
| 171. | 63 à <66 | 13 |
| 172. | 63 à <66 | 14 |
| 173. | 63 à <66 | 15 |
| 174. | 63 à <66 | 16 |
| 175. | 63 à <66 | 17 |
| 176. | 63 à <66 | 18 |
| 177. | 63 à <66 | 19 |
| 178. | 63 à <66 | 20+ |
| 179. | 66 à <69 | -5 |
| 180. | 66 à <69 | 6 |
| 181. | 66 à <69 | 7 |
| 182. | 66 à <69 | 8 |
| 183. | 66 à <69 | 9 |
| 184. | 66 à <69 | 10 |
| 185. | 66 à <69 | 11 |
| 186. | 66 à <69 | 12 |
| 187. | 66 à <69 | 13 |
| 188. | 66 à <69 | 14 |
| 189. | 66 à <69 | 15 |
| 190. | 66 à <69 | 16 |
| 191. | 66 à <69 | 17 |
| 192. | 66 à <69 | 18 |
| 193. | 66 à <69 | 19 |
| 194. | 66 à <69 | 20+ |
| 195. | $\geq$69 | -5 |
| 196. | $\geq$69 | 6 |
| 197. | $\geq$69 | 7 |
| 198. | $\geq$69 | 8 |

| n° | Hunter L | Hunter b |
|---|---|---|
| 199. | $\geq$69 | 9 |
| 200. | $\geq$69 | 10 |
| 201. | $\geq$69 | 11 |
| 202. | $\geq$69 | 12 |
| 203. | $\geq$69 | 13 |
| 204. | $\geq$69 | 14 |

| n° | Hunter L | Hunter b |
|---|---|---|
| 205. | $\geq$69 | 15 |
| 206. | $\geq$69 | 16 |
| 207. | $\geq$69 | 17 |
| 208. | $\geq$69 | 18 |
| 209. | $\geq$69 | 19 |
| 210. | $\geq$69 | 20+ |

où l'indication -5 signifie moins de 5, mais plus de 4, et l'indication +12 signifie plus de 12, mais moins de 13.

13. Procédé selon l'une quelconque des revendications 1 à 7, où:

(a) l'opération de compilation d'un groupe d'intervalles de valeurs de mesures de luminance comprend la compilation d'intervalles d'un facteur de couleur dont la valeur est sensiblement celle du facteur de couleur Hunter L, et

(b) l'opération d'association, avec chaque intervalle de valeurs de mesures de luminance, d'une valeur d'un facteur de couleur à utiliser dans la comparaison avec une mesure de la valeur de ce facteur de couleur dans la coloration d'un sujet d'essai ayant une clarté mesurée comprise à l'intérieur dudit intervalle comprend l'association, avec chaque intervalle de valeurs de mesures de luminance, de valeurs d'un facteur de couleur dont la valeur est sensiblement celle du facteur de couleur Hunter b, et la relation entre lesdits intervalles de valeurs de facteurs de couleur de mesures de luminance et lesdites valeurs de facteurs de couleur associées étant sensiblement la suivante:

| n° | Hunter L | Hunter b |
|---|---|---|
| 1. | <27 | -5 |
| 2. | <27 | 6 |
| 3. | <27 | 7 |
| 4. | <27 | 8 |
| 5. | <27 | 9 |
| 6. | <27 | 10 |
| 7. | <27 | 11 |
| 8. | <27 | 12+ |
| 9. | 27 à <30 | -5 |
| 10. | 27 à <30 | 6 |
| 11. | 27 à <30 | 7 |
| 12. | 27 à <30 | 8 |
| 13. | 27 à <30 | 9 |
| 14. | 27 à <30 | 10 |
| 15. | 27 à <30 | 11 |
| 16. | 27 à <30 | 12+ |
| 17. | 30 à <33 | -5 |
| 18. | 30 à <33 | 6 |
| 19. | 30 à <33 | 7 |
| 20. | 30 à <33 | 8 |
| 21. | 30 à <33 | 9 |
| 22. | 30 à <33 | 10 |
| 23. | 30 à <33 | 11 |
| 24. | 30 à <33 | 12+ |
| 25. | 33 à <36 | -5 |
| 26. | 33 à <36 | 6 |
| 27. | 33 à <36 | 7 |
| 28. | 33 à <36 | 8 |
| 29. | 33 à <36 | 9 |
| 30. | 33 à <36 | 10 |
| 31. | 33 à <36 | 11 |
| 32. | 33 à <36 | 12+ |

| n° | Hunter L | Hunter b |
|---|---|---|
| 33. | 36 à <39 | -5 |
| 34. | 36 à <39 | 6 |
| 35. | 36 à <39 | 7 |
| 36. | 36 à <39 | 8 |
| 37. | 36 à <39 | 9 |
| 38. | 36 à <39 | 10 |
| 39. | 36 à <39 | 11 |
| 40. | 36 à <39 | 12 |
| 41. | 36 à <39 | 13 |
| 42. | 36 à <39 | 14 |
| 43. | 36 à <39 | 15+ |
| 44. | 39 à <42 | -5 |
| 45. | 39 à <42 | 6 |
| 46. | 39 à <42 | 7 |
| 47. | 39 à <42 | 8 |
| 48. | 39 à <42 | 9 |
| 49. | 39 à <42 | 10 |
| 50. | 39 à <42 | 11 |
| 51. | 39 à <42 | 12 |
| 52. | 39 à <42 | 13 |
| 53. | 39 à <42 | 14 |
| 54. | 39 à <42 | 15+ |
| 55. | 42 à <45 | -5 |
| 56. | 42 à <45 | 6 |
| 57. | 42 à <45 | 7 |
| 58. | 42 à <45 | 8 |
| 59. | 42 à <45 | 9 |
| 60. | 42 à <45 | 10 |
| 61. | 42 à <45 | 11 |
| 62. | 42 à <45 | 12 |
| 63. | 42 à <45 | 13 |
| 64. | 42 à <45 | 14 |

| n° | Hunter L | Hunter b |
|-----|----------|----------|
| 65. | 42 à <45 | 15 |
| 66. | 42 à <45 | 16 |
| 67. | 42 à <45 | 17 |
| 68. | 42 à <45 | 18+ |
| 69. | 45 à <48 | -5 |
| 70. | 45 à <48 | 6 |
| 71. | 45 à <48 | 7 |
| 72. | 45 à <48 | 8 |
| 73. | 45 à <48 | 9 |
| 74. | 45 à <48 | 10 |
| 75. | 45 à <48 | 11 |
| 76. | 45 à <48 | 12 |
| 77. | 45 à <48 | 13 |
| 78. | 45 à <48 | 14 |
| 79. | 45 à <48 | 15 |
| 80. | 45 à <48 | 16 |
| 81. | 45 à <48 | 17 |
| 82. | 45 à <48 | 18+ |
| 83. | 48 à <51 | -5 |
| 84. | 48 à <51 | 6 |
| 85. | 48 à <51 | 7 |
| 86. | 48 à <51 | 8 |
| 87. | 48 à <51 | 9 |
| 88. | 48 à <51 | 10 |
| 89. | 48 à <51 | 11 |
| 90. | 48 à <51 | 12 |
| 91. | 48 à <51 | 13 |
| 92. | 48 à <51 | 14 |
| 93. | 48 à <51 | 15 |
| 94. | 48 à <51 | 16 |
| 95. | 48 à <51 | 17 |
| 96. | 48 à <51 | 18 |
| 97. | 48 à <51 | 19 |

| n° | Hunter L | Hunter b |
|-----|----------|----------|
| 98. | 48 à <51 | 20+ |
| 99. | 51 à <54 | -5 |
| 100. | 51 à <54 | 6 |
| 101. | 51 à <54 | 7 |
| 102. | 51 à <54 | 8 |
| 103. | 51 à <54 | 9 |
| 104. | 51 à <54 | 10 |
| 105. | 51 à <54 | 11 |
| 106. | 51 à <54 | 12 |
| 107. | 51 à <54 | 13 |
| 108. | 51 à <54 | 14 |
| 109. | 51 à <54 | 15 |
| 110. | 51 à <54 | 16 |
| 111. | 51 à <54 | 17 |
| 112. | 51 à <54 | 18 |
| 113. | 51 à <54 | 19 |
| 114. | 51 à <54 | 20+ |
| 115. | 54 à <57 | -5 |
| 116. | 54 à <57 | 6 |
| 117. | 54 à <57 | 7 |
| 118. | 54 à <57 | 8 |
| 119. | 54 à <57 | 9 |
| 120. | 54 à <57 | 10 |
| 121. | 54 à <57 | 11 |
| 122. | 54 à <57 | 12 |
| 123. | 54 à <57 | 13 |
| 124. | 54 à <57 | 14 |
| 125. | 54 à <57 | 15 |
| 126. | 54 à <57 | 16 |
| 127. | 54 à <57 | 17 |
| 128. | 54 à <57 | 18 |
| 129. | 54 à <57 | 19 |
| 130. | 54 à <57 | 20+ |

| n° | Hunter L | Hunter b | n° | Hunter L | Hunter b |
|---|---|---|---|---|---|
| 131. | 57 à <60 | -5 | 165. | 63 à <66 | 7 |
| 132. | 57 à <60 | 6 | 166. | 63 à <66 | 8 |
| 133. | 57 à <60 | 7 | 167. | 63 à <66 | 9 |
| 134. | 57 à <60 | 8 | 168. | 63 à <66 | 10 |
| 135. | 57 à <60 | 9 | 169. | 63 à <66 | 11 |
| 136. | 57 à <60 | 10 | 170. | 63 à <66 | 12 |
| 137. | 57 à <60 | 11 | 171. | 63 à <66 | 13 |
| 138. | 57 à <60 | 12 | 172. | 63 à <66 | 14 |
| 139. | 57 à <60 | 13 | 173. | 63 à <66 | 15 |
| 140. | 57 à <60 | 14 | 174. | 63 à <66 | 16 |
| 141. | 57 à <60 | 15 | 175. | 63 à <66 | 17 |
| 142. | 57 à <60 | 16 | 176. | 63 à <66 | 18 |
| 143. | 57 à <60 | 17 | 177. | 63 à <66 | 19 |
| 144. | 57 à <60 | 18 | 178. | 63 à <66 | 20+ |
| 145. | 57 à <60 | 19 | 179. | 66 à <69 | -5 |
| 146. | 57 à <60 | 20+ | 180. | 66 à <69 | 6 |
| 147. | 60 à <63 | -5 | 181. | 66 à <69 | 7 |
| 148. | 60 à <63 | 6 | 182. | 66 à <69 | 8 |
| 149. | 60 à <63 | 7 | 183. | 66 à <69 | 9 |
| 150. | 60 à <63 | 8 | 184. | 66 à <69 | 10 |
| 151. | 60 à <63 | 9 | 185. | 66 à <69 | 11 |
| 152. | 60 à <63 | 10 | 186. | 66 à <69 | 12 |
| 153. | 60 à <63 | 11 | 187. | 66 à <69 | 13 |
| 154. | 60 à <63 | 12 | 188. | 66 à <69 | 14 |
| 155. | 60 à <63 | 13 | 189. | 66 à <69 | 15 |
| 156. | 60 à <63 | 14 | 190. | 66 à <69 | 16 |
| 157. | 60 à <63 | 15 | 191. | 66 à <69 | 17 |
| 158. | 60 à <63 | 16 | 192. | 66 à <69 | 18 |
| 159. | 60 à <63 | 17 | 193. | 66 à <69 | 19 |
| 160. | 60 à <63 | 18 | 194. | 66 à <69 | 20+ |
| 161. | 60 à <63 | 19 | 195. | ≥69 | -5 |
| 162. | 60 à <63 | 20+ | 196. | ≥69 | 6 |
| 163. | 63 à <66 | -5 | 197. | ≥69 | 7 |
| 164. | 63 à <66 | 6 | 198. | ≥69 | 8 |

| n° | Hunter L | Hunter b |
|---|---|---|
| 199. | $\geq 69$ | 9 |
| 200. | $\geq 69$ | 10 |
| 201. | $\geq 69$ | 11 |
| 202. | $\geq 69$ | 12 |
| 203. | $\geq 69$ | 13 |
| 204. | $\geq 69$ | 14 |

| n° | Hunter L | Hunter b |
|---|---|---|
| 205. | $\geq 69$ | 15 |
| 206. | $\geq 69$ | 16 |
| 207. | $\geq 69$ | 17 |
| 208. | $\geq 69$ | 18 |
| 209. | $\geq 69$ | 19 |
| 210. | $\geq 69$ | 20+ |

ou l'indication -5 signifie moins de 5, mais plus de 4, et l'indication +12 signifie plus de 12, mais moins de 13.

**14.** Procédé selon l'une quelconque des revendications 1 à 7, où :

l'opération (a) comprend la compilation d'au moins un intervalle d'un facteur de couleur dont la valeur est sensiblement celle du facteur de couleur Hunter L, ledit ou lesdits intervalles étant sélectionnés dans les intervalles sensiblement tels que les suivants :
Hunter L = 27, 27 à <30, 30 à <33, 33 à <36, 36 à <39, 39 à <42, 42 à <45, 45 à <48, 48 à <51, 51 à <54, 54 à <57, 57 à <60, 60 à <63, 63 à <66, 66 à <69 et $\geq$ 69.

**15.** Procédé selon l'une quelconque des revendications précédentes, comprenant les opérations suivantes :

(c) mesurer, avec un instrument de mesure de couleur, la valeur de ladite mesure de luminance dans la coloration de la peau du sujet d'essai, et
(d) mesurer, avec un instrument de mesure de couleur, la valeur dudit facteur de couleur dans la coloration de la peau du sujet d'essai.

**16.** Procédé selon la revendication 15, où la mesure de luminance est un facteur de couleur de luminance dépendant de la luminance de la coloration de ladite peau du sujet d'essai, des intervalles de valeurs dudit facteur de couleur de luminance constituant lesdits intervalles de valeurs de mesures de luminance, ledit facteur de couleur de l'opération (b) dépendant de la teneur relative en couleurs opposée dans la coloration de la peau dudit sujet d'essai, où au moins l'une des opérations (a) et (b) comprend l'opération consistant à arriver à une valeur mesurée qui se corrèle à une mesure de coloration ayant une utilité établie.

**17.** Procédé selon la revendication 15 ou 16, où :

l'opération (a) comprend la compilation d'un groupe d'intervalles de valeurs de mesures de luminance que l'on trouve dans la coloration de la couleur de la peau de sujets autres que le sujet d'essai ;
l'opération (b) comprend l'association, à chaque intervalle de valeurs de mesures de luminance, un intervalle de valeurs dudit facteur de couleur dans la coloration de sujets autres que le sujet d'essai, le procédé comprenant en outre l'opération qui consiste à comparer les valeurs mesurées de ladite mesure de luminance et dudit facteur de couleur avec lesdits intervalles de valeurs de ladite mesure d'éclairement et dudit facteur de couleur que l'on trouve dans les colorations de sujets autres que ledit sujet d'essai, comme compilé et assemblé dans les opérations (a) et (b), afin de déterminer où se situent les valeurs mesurées à l'intérieur dudit intervalle.

**18.** Procédé selon l'une quelconque des revendications 15 à 17, où ledit facteur de couleur dépend de la teneur relative en bleu et en jaune de ladite coloration.

**19.** Procédé selon l'une quelconque des revendications 15 à 17, où ledit facteur de couleur dépend de la teneur relative en rouge et en vert dans ladite coloration.

**20.** Procédé selon l'une quelconque des revendications 15 à 19, où chacune des opérations (c) et (d) comprend la mesure des valeurs de ladite mesure de luminance et dudit facteur de couleur en des emplacements différents du sujet d'essai.

**21.** Procédé selon l'une quelconque des revendications 15 à 19, où chacune des opérations (c) et (d) réalise un ensemble de plusieurs mesures des valeurs de chacun des suivants, à savoir la mesure de luminance, le facteur de couleur, et la prise de la moyenne pour chaque ensemble de plusieurs mesures.

**22.** Appareil servant à évaluer l'état physiologique médical ou non cosmétique d'un sujet d'essai humain ou animal, lequel état est associé à une coloration symptomatique pouvant être détectée, l'appareil comprenant :

(a) un moyen servant à compiler un groupe d'intervalles de valeurs de mesures de luminance, et
(b) un moyen servant à associer, à chaque intervalle de valeurs de mesures de luminance, une valeur ou un intervalle de valeurs d'un facteur de couleur destiné à être utilisé dans la comparaison avec une mesure de la valeur de ce facteur de couleur dans la coloration d'un sujet d'essai ayant une luminance mesurée comprise à l'intérieur dudit intervalle, pour autant que l'appareil ne soit pas utilisé pour déterminer une coloration de la peau par un cosmétique pour le sujet d'essai.

**23.** Appareil selon la revendication 22, où l'état est un état qui provoque la jaunisse chez un sujet d'essai humain jeune ou adulte.

**24.** Appareil selon la revendication 22, où l'état est l'un des suivants, à savoir l'hyperbilirubinémie, l'hypertension, la tuberculose ou une affection du foie.

**25.** Appareil selon l'une quelconque des revendications 22 à 24, où le sujet d'essai est un nourrisson humain.

**26.** Appareil selon l'une quelconque des revendications 22 à 25, où le moyen de compilation comprend un moyen servant à assembler le groupe d'intervalles de valeurs de mesures de luminance sous une forme pouvant être traitée par ordinateur, et le moyen d'association comprend un moyen servant à associer la valeur du facteur de couleur avec chaque intervalle de valeurs de mesures de luminance sous ladite forme pouvant être traitée par ordinateur.

**27.** Appareil selon l'une quelconque des revendications 22 à 26, où le moyen d'association comprend un moyen servant à associer une valeur du facteur de couleur Hunter b avec chaque intervalle de valeurs de mesures de luminance.

**28.** Appareil selon l'une quelconque des revendications 22 à 27, où le moyen de compilation comprend un moyen permettant de compiler un groupe de valeurs de mesures de luminance Hunter L

**29.** Appareil selon l'une quelconque des revendications 22 à 26, où le moyen de compilation comprend un moyen permettant de compiler un groupe de valeurs de facteurs de couleur de mesures de luminance qui sont sensiblement celles du facteur de couleur Hunter L, et le moyen d'association comprend un moyen permettant d'associer une valeur d'un facteur de couleur dont la valeur est sensiblement celle du facteur de couleur Hunter a avec chaque intervalle de valeurs de facteurs de couleur de mesures de luminance, la relation entre lesdits intervalles de valeurs de facteurs de couleur de mesures de luminance et lesdites valeurs de facteurs de couleur associées étant sensiblement la suivante :

| Hunter L | Hunter a |
|---|---|
| de 24 (ou moins) à 44 | de 4 à 16 |
| 45 à 54 à | 18 |
| 55 à 59 à | 25 |
| 60 à 71 (ou plus) | 6 à 30. |

**30.** Appareil selon l'une quelconque des revendications 22 à 26, où le moyen d'association comprend un moyen permettant d'associer, avec chaque intervalle de valeurs de mesures de luminance, une valeur d'un facteur de couleur dépendant du jaune.

**31.** Appareil selon l'une quelconque des revendications 22 à 26, où le facteur de couleur de luminance est Hunter b

**EP 0 832 422 B1**

et les intervalles de valeurs de mesures de luminance sont les intervalles de Hunter L, l'appareil comprenant en outre un moyen permettant de mesurer, au moyen d'un instrument, une valeur de Hunter L pour la coloration de la peau du sujet d'essai.

**32.** Appareil selon l'une quelconque des revendications 22 à 28, où lesdits intervalles de valeurs sont déterminés par les intervalles de valeurs Hunter L ; les intervalles de valeurs de Hunter L étant limités par au moins l'une des valeurs Hunter L sensiblement suivantes :

Hunter L = 27, 30, 33, 36, 39, 42, 45, 48, 51, 54, 57, 60, 63, 66 et 69

**33.** Appareil selon l'une quelconque des revendications 22 à 28, où la coloration est la coloration de la peau, et l'état est l'hyperbilirubinémie, le moyen de compilation comprend un moyen permettant de compiler des intervalles qui sont sensiblement des intervalles de valeurs de Hunter L, et le moyen d'association comprend un moyen permettant d'associer un intervalle de valeurs qui sont sensiblement celles de Hunter b avec chaque intervalle de valeurs de mesures de luminance, l'appareil comprenant en outre :

(c) un moyen servant à mesurer, avec un instrument de mesure de couleur, la valeur d'un premier facteur de couleur qui est sensiblement celui de Hunter b dans la coloration de la peau du sujet d'essai ;
(d) un moyen servant à mesurer, avec un instrument de mesure de couleur, la valeur d'un deuxième facteur de couleur qui est sensiblement celui de Hunter L dans ladite coloration de la peau du sujet d'essai ;
(e) un moyen servant à comparer la valeur mesurée du deuxième facteur de couleur avec les intervalles compilés par le moyen de compilation d'intervalles qui sont sensiblement des intervalles de Hunter L afin de localiser une luminance comparable dans la coloration du sujet d'essai et les intervalles compilés ; et
(f) un moyen servant à comparer la valeur mesurée du premier facteur de couleur avec un intervalle de valeurs de premiers facteurs de couleur associé par le moyen d'association d'une valeur qui est sensiblement celle de Hunter b avec l'intervalle de valeurs de mesures de luminance compilées comparables afin de déterminer si la valeur mesurée de premiers facteurs de couleur se trouve à l'intérieur ou à l'extérieur de l'intervalle associé des valeurs de premiers facteurs de couleur ; où on choisit l'intervalle associé de valeurs de premiers facteurs de couleur dans les intervalles sensiblement suivants :

| n° | Hunter L | Hunter b |
|-----|-----------|-----------|
| 1. | <27 | -5 |
| 2. | <27 | 6 |
| 3. | <27 | 7 |
| 4. | <27 | 8 |
| 5. | <27 | 9 |
| 6. | <27 | 10 |
| 7. | <27 | 11 |
| 8. | <27 | 12+ |
| 9. | 27 à <30 | -5 |
| 10. | 27 à <30 | 6 |
| 11. | 27 à <30 | 7 |
| 12. | 27 à <30 | 8 |
| 13. | 27 à <30 | 9 |
| 14. | 27 à <30 | 10 |
| 15. | 27 à <30 | 11 |
| 16. | 27 à <30 | 12+ |
| 17. | 30 à <33 | -5 |
| 18. | 30 à <33 | 6 |
| 19. | 30 à <33 | 7 |
| 20. | 30 à <33 | 8 |
| 21. | 30 à <33 | 9 |
| 22. | 30 à <33 | 10 |
| 23. | 30 à <33 | 11 |
| 24. | 30 à <33 | 12+ |
| 25. | 33 à <36 | -5 |
| 26. | 33 à <36 | 6 |
| 27. | 33 à <36 | 7 |
| 28. | 33 à <36 | 8 |
| 29. | 33 à <36 | 9 |
| 30. | 33 à <36 | 10 |
| 31. | 33 à <36 | 11 |
| 32. | 33 à <36 | 12+ |

| n° | Hunter L | Hunter b |
|-----|-----------|-----------|
| 33. | 36 à <39 | -5 |
| 34. | 36 à <39 | 6 |
| 35. | 36 à <39 | 7 |
| 36. | 36 à <39 | 8 |
| 37. | 36 à <39 | 9 |
| 38. | 36 à <39 | 10 |
| 39. | 36 à <39 | 11 |
| 40. | 36 à <39 | 12 |
| 41. | 36 à <39 | 13 |
| 42. | 36 à <39 | 14 |
| 43. | 36 à <39 | 15+ |
| 44. | 39 à <42 | -5 |
| 45. | 39 à <42 | 6 |
| 46. | 39 à <42 | 7 |
| 47. | 39 à <42 | 8 |
| 48. | 39 à <42 | 9 |
| 49. | 39 à <42 | 10 |
| 50. | 39 à <42 | 11 |
| 51. | 39 à <42 | 12 |
| 52. | 39 à <42 | 13 |
| 53. | 39 à <42 | 14 |
| 54. | 39 à <42 | 15+ |
| 55. | 42 à <45 | -5 |
| 56. | 42 à <45 | 6 |
| 57. | 42 à <45 | 7 |
| 58. | 42 à <45 | 8 |
| 59. | 42 à <45 | 9 |
| 60. | 42 à <45 | 10 |
| 61. | 42 à <45 | 11 |
| 62. | 42 à <45 | 12 |
| 63. | 42 à <45 | 13 |
| 64. | 42 à <45 | 14 |

| n° | Hunter L | Hunter b |
|---|---|---|
| 65. | 42 à <45 | 15 |
| 66. | 42 à <45 | 16 |
| 67. | 42 à <45 | 17 |
| 68. | 42 à <45 | 18+ |
| 69. | 45 à <48 | -5 |
| 70. | 45 à <48 | 6 |
| 71. | 45 à <48 | 7 |
| 72. | 45 à <48 | 8 |
| 73. | 45 à <48 | 9 |
| 74. | 45 à <48 | 10 |
| 75. | 45 à <48 | 11 |
| 76. | 45 à <48 | 12 |
| 77. | 45 à <48 | 13 |
| 78. | 45 à <48 | 14 |
| 79. | 45 à <48 | 15 |
| 80. | 45 à <48 | 16 |
| 81. | 45 à <48 | 17 |
| 82. | 45 à <48 | 18+ |
| 83. | 48 à <51 | -5 |
| 84. | 48 à <51 | 6 |
| 85. | 48 à <51 | 7 |
| 86. | 48 à <51 | 8 |
| 87. | 48 à <51 | 9 |
| 88. | 48 à <51 | 10 |
| 89. | 48 à <51 | 11 |
| 90. | 48 à <51 | 12 |
| 91. | 48 à <51 | 13 |
| 92. | 48 à <51 | 14 |
| 93. | 48 à <51 | 15 |
| 94. | 48 à <51 | 16 |
| 95. | 48 à <51 | 17 |
| 96. | 48 à <51 | 18 |
| 97. | 48 à <51 | 19 |

| n° | Hunter L | Hunter b |
|---|---|---|
| 98. | 48 à <51 | 20+ |
| 99. | 51 à <54 | -5 |
| 100. | 51 à <54 | 6 |
| 101. | 51 à <54 | 7 |
| 102. | 51 à <54 | 8 |
| 103. | 51 à <54 | 9 |
| 104. | 51 à <54 | 10 |
| 105. | 51 à <54 | 11 |
| 106. | 51 à <54 | 12 |
| 107. | 51 à <54 | 13 |
| 108. | 51 à <54 | 14 |
| 109. | 51 à <54 | 15 |
| 110. | 51 à <54 | 16 |
| 111. | 51 à <54 | 17 |
| 112. | 51 à <54 | 18 |
| 113. | 51 à <54 | 19 |
| 114. | 51 à <54 | 20+ |
| 115. | 54 à <57 | -5 |
| 116. | 54 à <57 | 6 |
| 117. | 54 à <57 | 7 |
| 118. | 54 à <57 | 8 |
| 119. | 54 à <57 | 9 |
| 120. | 54 à <57 | 10 |
| 121. | 54 à <57 | 11 |
| 122. | 54 à <57 | 12 |
| 123. | 54 à <57 | 13 |
| 124. | 54 à <57 | 14 |
| 125. | 54 à <57 | 15 |
| 126. | 54 à <57 | 16 |
| 127. | 54 à <57 | 17 |
| 128. | 54 à <57 | 18 |
| 129. | 54 à <57 | 19 |
| 130. | 54 à <57 | 20+ |

| n° | Hunter L | Hunter b | | n° | Hunter L | Hunter b |
|---|---|---|---|---|---|---|
| 131. | 57 à <60 | -5 | | 165. | 63 à <66 | 7 |
| 132. | 57 à <60 | 6 | | 166. | 63 à <66 | 8 |
| 133. | 57 à <60 | 7 | | 167. | 63 à <66 | 9 |
| 134. | 57 à <60 | 8 | | 168. | 63 à <66 | 10 |
| 135. | 57 à <60 | 9 | | 169. | 63 à <66 | 11 |
| 136. | 57 à <60 | 10 | | 170. | 63 à <66 | 12 |
| 137. | 57 à <60 | 11 | | 171. | 63 à <66 | 13 |
| 138. | 57 à <60 | 12 | | 172. | 63 à <66 | 14 |
| 139. | 57 à <60 | 13 | | 173. | 63 à <66 | 15 |
| 140. | 57 à <60 | 14 | | 174. | 63 à <66 | 16 |
| 141. | 57 à <60 | 15 | | 175. | 63 à <66 | 17 |
| 142. | 57 à <60 | 16 | | 176. | 63 à <66 | 18 |
| 143. | 57 à <60 | 17 | | 177. | 63 à <66 | 19 |
| 144. | 57 à <60 | 18 | | 178. | 63 à <66 | 20+ |
| 145. | 57 à <60 | 19 | | 179. | 66 à <69 | -5 |
| 146. | 57 à <60 | 20+ | | 180. | 66 à <69 | 6 |
| 147. | 60 à <63 | -5 | | 181. | 66 à <69 | 7 |
| 148. | 60 à <63 | 6 | | 182. | 66 à <69 | 8 |
| 149. | 60 à <63 | 7 | | 183. | 66 à <69 | 9 |
| 150. | 60 à <63 | 8 | | 184. | 66 à <69 | 10 |
| 151. | 60 à <63 | 9 | | 185. | 66 à <69 | 11 |
| 152. | 60 à <63 | 10 | | 186. | 66 à <69 | 12 |
| 153. | 60 à <63 | 11 | | 187. | 66 à <69 | 13 |
| 154. | 60 à <63 | 12 | | 188. | 66 à <69 | 14 |
| 155. | 60 à <63 | 13 | | 189. | 66 à <69 | 15 |
| 156. | 60 à <63 | 14 | | 190. | 66 à <69 | 16 |
| 157. | 60 à <63 | 15 | | 191. | 66 à <69 | 17 |
| 158. | 60 à <63 | 16 | | 192. | 66 à <69 | 18 |
| 159. | 60 à <63 | 17 | | 193. | 66 à <69 | 19 |
| 160. | 60 à <63 | 18 | | 194. | 66 à <69 | 20+ |
| 161. | 60 à <63 | 19 | | 195. | ≥69 | -5 |
| 162. | 60 à <63 | 20+ | | 196. | ≥69 | 6 |
| 163. | 63 à <66 | -5 | | 197. | ≥69 | 7 |
| 164. | 63 à <66 | 6 | | 198. | ≥69 | 8 |

| n° | Hunter L | Hunter b |
|---|---|---|
| 199. | $\geq$69 | 9 |
| 200. | $\geq$69 | 10 |
| 201. | $\geq$69 | 11 |
| 202. | $\geq$69 | 12 |
| 203. | $\geq$69 | 13 |
| 204. | $\geq$69 | 14 |

| n° | Hunter L | Hunter b |
|---|---|---|
| 205. | $\geq$69 | 15 |
| 206. | $\geq$69 | 16 |
| 207. | $\geq$69 | 17 |
| 208. | $\geq$69 | 18 |
| 209. | $\geq$69 | 19 |
| 210. | $\geq$69 | 20+ |

où l'indication -5 signifie moins de 5, mais plus de 4, et l'indication +12 signifie plus de 12, mais moins de 13.

**34.** Appareil selon l'une quelconque des revendications 22 à 28, où :

(a) le moyen de compilation d'un groupe d'intervalles de valeurs de mesures de luminance comprend un moyen permrrettant de compiler des intervalles d'un facteur de couleur dont la valeur est sensiblement celle du facteur de couleur Hunter L, et
(b) le moyen d'association, avec chaque intervalle de valeurs de mesures de luminance, d'une valeur d'un facteur de couleur à utiliser dans la comparaison avec une mesure de la valeur de ce facteur de couleur dans la coloration d'un sujet d'essai ayant une luminance mesurée comprise à l'intérieur dudit intervalle comprend un moyen permettant d'associer, avec chaque intervalle de valeurs de mesures de luminance, des valeurs d'un facteur de couleur dont la valeur est sensiblement celle du facteur de couleur Hunter b, et la relation entre lesdits intervalles de valeurs de facteurs de couleur de mesures d'éclairement et lesdites valeurs de facteurs de couleur associées étant sensiblement la suivante :

| n° | Hunter L | Hunter b |
|----|----------|----------|
| 1. | <27 | -5 |
| 2. | <27 | 6 |
| 3. | <27 | 7 |
| 4. | <27 | 8 |
| 5. | <27 | 9 |
| 6. | <27 | 10 |
| 7. | <27 | 11 |
| 8. | <27 | 12+ |
| 9. | 27 à <30 | -5 |
| 10. | 27 à <30 | 6 |
| 11. | 27 à <30 | 7 |
| 12. | 27 à <30 | 8 |
| 13. | 27 à <30 | 9 |
| 14. | 27 à <30 | 10 |
| 15. | 27 à <30 | 11 |
| 16. | 27 à <30 | 12+ |
| 17. | 30 à <33 | -5 |
| 18. | 30 à <33 | 6 |
| 19. | 30 à <33 | 7 |
| 20. | 30 à <33 | 8 |
| 21. | 30 à <33 | 9 |
| 22. | 30 à <33 | 10 |
| 23. | 30 à <33 | 11 |
| 24. | 30 à <33 | 12+ |
| 25. | 33 à <36 | -5 |
| 26. | 33 à <36 | 6 |
| 27. | 33 à <36 | 7 |
| 28. | 33 à <36 | 8 |
| 29. | 33 à <36 | 9 |
| 30. | 33 à <36 | 10 |
| 31. | 33 à <36 | 11 |
| 32. | 33 à <36 | 12+ |

| n° | Hunter L | Hunter b |
|----|----------|----------|
| 33. | 36 à <39 | -5 |
| 34. | 36 à <39 | 6 |
| 35. | 36 à <39 | 7 |
| 36. | 36 à <39 | 8 |
| 37. | 36 à <39 | 9 |
| 38. | 36 à <39 | 10 |
| 39. | 36 à <39 | 11 |
| 40. | 36 à <39 | 12 |
| 41. | 36 à <39 | 13 |
| 42. | 36 à <39 | 14 |
| 43. | 36 à <39 | 15+ |
| 44. | 39 à <42 | -5 |
| 45. | 39 à <42 | 6 |
| 46. | 39 à <42 | 7 |
| 47. | 39 à <42 | 8 |
| 48. | 39 à <42 | 9 |
| 49. | 39 à <42 | 10 |
| 50. | 39 à <42 | 11 |
| 51. | 39 à <42 | 12 |
| 52. | 39 à <42 | 13 |
| 53. | 39 à <42 | 14 |
| 54. | 39 à <42 | 15+ |
| 55. | 42 à <45 | -5 |
| 56. | 42 à <45 | 6 |
| 57. | 42 à <45 | 7 |
| 58. | 42 à <45 | 8 |
| 59. | 42 à <45 | 9 |
| 60. | 42 à <45 | 10 |
| 61. | 42 à <45 | 11 |
| 62. | 42 à <45 | 12 |
| 63. | 42 à <45 | 13 |
| 64. | 42 à <45 | 14 |

| n° | Hunter L | Hunter b |
|---|---|---|
| 65. | 42 à <45 | 15 |
| 66. | 42 à <45 | 16 |
| 67. | 42 à <45 | 17 |
| 68. | 42 à <45 | 18+ |
| 69. | 45 à <48 | -5 |
| 70. | 45 à <48 | 6 |
| 71. | 45 à <48 | 7 |
| 72. | 45 à <48 | 8 |
| 73. | 45 à <48 | 9 |
| 74. | 45 à <48 | 10 |
| 75. | 45 à <48 | 11 |
| 76. | 45 à <48 | 12 |
| 77. | 45 à <48 | 13 |
| 78. | 45 à <48 | 14 |
| 79. | 45 à <48 | 15 |
| 80. | 45 à <48 | 16 |
| 81. | 45 à <48 | 17 |
| 82. | 45 à <48 | 18+ |
| 83. | 48 à <51 | -5 |
| 84. | 48 à <51 | 6 |
| 85. | 48 à <51 | 7 |
| 86. | 48 à <51 | 8 |
| 87. | 48 à <51 | 9 |
| 88. | 48 à <51 | 10 |
| 89. | 48 à <51 | 11 |
| 90. | 48 à <51 | 12 |
| 91. | 48 à <51 | 13 |
| 92. | 48 à <51 | 14 |
| 93. | 48 à <51 | 15 |
| 94. | 48 à <51 | 16 |
| 95. | 48 à <51 | 17 |
| 96. | 48 à <51 | 18 |
| 97. | 48 à <51 | 19 |

| n° | Hunter L | Hunter b |
|---|---|---|
| 98. | 48 à <51 | 20+ |
| 99. | 51 à <54 | -5 |
| 100. | 51 à <54 | 6 |
| 101. | 51 à <54 | 7 |
| 102. | 51 à <54 | 8 |
| 103. | 51 à <54 | 9 |
| 104. | 51 à <54 | 10 |
| 105. | 51 à <54 | 11 |
| 106. | 51 à <54 | 12 |
| 107. | 51 à <54 | 13 |
| 108. | 51 à <54 | 14 |
| 109. | 51 à <54 | 15 |
| 110. | 51 à <54 | 16 |
| 111. | 51 à <54 | 17 |
| 112. | 51 à <54 | 18 |
| 113. | 51 à <54 | 19 |
| 114. | 51 à <54 | 20+ |
| 115. | 54 à <57 | -5 |
| 116. | 54 à <57 | 6 |
| 117. | 54 à <57 | 7 |
| 118. | 54 à <57 | 8 |
| 119. | 54 à <57 | 9 |
| 120. | 54 à <57 | 10 |
| 121. | 54 à <57 | 11 |
| 122. | 54 à <57 | 12 |
| 123. | 54 à <57 | 13 |
| 124. | 54 à <57 | 14 |
| 125. | 54 à <57 | 15 |
| 126. | 54 à <57 | 16 |
| 127. | 54 à <57 | 17 |
| 128. | 54 à <57 | 18 |
| 129. | 54 à <57 | 19 |
| 130. | 54 à <57 | 20+ |

| n° | Hunter L | Hunter b |
|---|---|---|
| 131. | 57 à <60 | -5 |
| 132. | 57 à <60 | 6 |
| 133. | 57 à <60 | 7 |
| 134. | 57 à <60 | 8 |
| 135. | 57 à <60 | 9 |
| 136. | 57 à <60 | 10 |
| 137. | 57 à <60 | 11 |
| 138. | 57 à <60 | 12 |
| 139. | 57 à <60 | 13 |
| 140. | 57 à <60 | 14 |
| 141. | 57 à <60 | 15 |
| 142. | 57 à <60 | 16 |
| 143. | 57 à <60 | 17 |
| 144. | 57 à <60 | 18 |
| 145. | 57 à <60 | 19 |
| 146. | 57 à <60 | 20+ |
| 147. | 60 à <63 | -5 |
| 148. | 60 à <63 | 6 |
| 149. | 60 à <63 | 7 |
| 150. | 60 à <63 | 8 |
| 151. | 60 à <63 | 9 |
| 152. | 60 à <63 | 10 |
| 153. | 60 à <63 | 11 |
| 154. | 60 à <63 | 12 |
| 155. | 60 à <63 | 13 |
| 156. | 60 à <63 | 14 |
| 157. | 60 à <63 | 15 |
| 158. | 60 à <63 | 16 |
| 159. | 60 à <63 | 17 |
| 160. | 60 à <63 | 18 |
| 161. | 60 à <63 | 19 |
| 162. | 60 à <63 | 20+ |
| 163. | 63 à <66 | -5 |
| 164. | 63 à <66 | 6 |

| n° | Hunter L | Hunter b |
|---|---|---|
| 165. | 63 à <66 | 7 |
| 166. | 63 à <66 | 8 |
| 167. | 63 à <66 | 9 |
| 168. | 63 à <66 | 10 |
| 169. | 63 à <66 | 11 |
| 170. | 63 à <66 | 12 |
| 171. | 63 à <66 | 13 |
| 172. | 63 à <66 | 14 |
| 173. | 63 à <66 | 15 |
| 174. | 63 à <66 | 16 |
| 175. | 63 à <66 | 17 |
| 176. | 63 à <66 | 18 |
| 177. | 63 à <66 | 19 |
| 178. | 63 à <66 | 20+ |
| 179. | 66 à <69 | -5 |
| 180. | 66 à <69 | 6 |
| 181. | 66 à <69 | 7 |
| 182. | 66 à <69 | 8 |
| 183. | 66 à <69 | 9 |
| 184. | 66 à <69 | 10 |
| 185. | 66 à <69 | 11 |
| 186. | 66 à <69 | 12 |
| 187. | 66 à <69 | 13 |
| 188. | 66 à <69 | 14 |
| 189. | 66 à <69 | 15 |
| 190. | 66 à <69 | 16 |
| 191. | 66 à <69 | 17 |
| 192. | 66 à <69 | 18 |
| 193. | 66 à <69 | 19 |
| 194. | 66 à <69 | 20+ |
| 195. | $\geq 69$ | -5 |
| 196. | $\geq 69$ | 6 |
| 197. | $\geq 69$ | 7 |
| 198. | $\geq 69$ | 8 |

| n° | Hunter L | Hunter b |
|---|---|---|
| 199. | ≥69 | 9 |
| 200. | ≥69 | 10 |
| 201. | ≥69 | 11 |
| 202. | ≥69 | 12 |
| 203. | ≥69 | 13 |
| 204. | ≥69 | 14 |

| n° | Hunter L | Hunter b |
|---|---|---|
| 205. | ≥69 | 15 |
| 206. | ≥69 | 16 |
| 207. | ≥69 | 17 |
| 208. | ≥69 | 18 |
| 209. | ≥69 | 19 |
| 210. | ≥69 | 20+ |

où l'indication -5 signifie moins de 5, mais plus de 4, et l'indication +12 signifie plus de 12, mais moins de 13.

**35.** Appareil selon l'une quelconque des revendications 22 à 28, où :

le moyen de compilation comprend un moyen permettant de compiler au moins un intervalle d'un facteur de couleur dont la valeur est sensiblement celle du facteur de couleur Hunter L, ledit ou lesdits intervalles étant sélectionnés dans les intervalles sensiblement tels que les suivants :
Hunter L = 27, 27 à <30, 30 à <33, 33 à <36, 36 à <39, 39 à <42, 42 à <45, 45 à <48, 48 à <51, 51 à <54, 54 à <57, 57 à <60, 60 à <63, 63 à <66, 66 à <69 et ≥ 69.

**36.** Appareil selon l'une quelconque des revendications 22 à 35, comprenant les opérations suivantes :

(c) un moyen servant à mesurer, avec un instrument de mesure de couleur, (i) la valeur de ladite mesure de luminance dans la coloration de la peau du sujet d'essai, et (ii) la valeur dudit facteur de couleur dans la coloration de la peau du sujet d'essai.

**37.** Appareil selon la revendication 36, où la mesure de luminance est un facteur de couleur de luminance dépendant de la luminance de la coloration de ladite peau du sujet d'essai, des intervalles de valeurs dudit facteur de couleur de luminance constituant lesdits intervalles de valeurs de mesures de luminance, ledit facteur de couleur de (b) dépendant de la teneur relative en couleurs opposée dans la coloration de la peau dudit sujet d'essai, où au moins l'un des facteurs de couleurs comprend une valeur mesurée qui se corrèle à une mesure de coloration ayant une utilité établie.

**38.** Appareil selon la revendication 36 ou 37, comprenant en outre un moyen permettant de comparer les valeurs mesurées du premier facteur de couleur et du deuxième facteur de couleur avec des Intervalles de valeurs desdits facteurs de couleur que l'on trouve dans les colorations de sujets autres que ledit sujet d'essai, comme compilé et assemblé par le moyen de compilation et le moyen d'association, afin de déterminer où se situent les valeurs mesurées à l'intérieur dudit intervalle.

**39.** Appareil selon l'une quelconque des revendications 36 à 38, où le moyen de mesure comprend un moyen permettant de mesurer la valeur d'un facteur de couleur qui dépend de la teneur relative en bleu et en jaune de ladite coloration.

**40.** Appareil selon l'une quelconque des revendications 36 à 38, où le moyen de mesure comprend un moyen permettant de mesurer la valeur d'un facteur de couleur qui dépend de la teneur relative en rouge et en vert de ladite coloration.

**41.** Appareil selon l'une quelconque des revendioeuons 36 à 40, où le moyen de mesure permet de mesurer les valeurs dudit premier facteur de couleur et dudit deuxième facteur de couleur en des emplacements différents du sujet d'essai.

**42.** Appareil selon l'une quelconque des revendications 36 à 41, où le moyen de mesure comprend un moyen permettant de réaliser un ensemble de plusieurs mesures des valeurs de chacun des suivants, à savoir le premier facteur de couleur et le deuxième facteur de couleur, et la prise de la moyenne pour chaque ensemble de plusieurs mesures.

FIG. Ia

FIG. Ib

71

1. AT TIME $t_0$ MULTIPLE COLORIMETER MEASUREMENTS OF Y, x & y FROM EACH OF SEVERAL SITES

2. CALCULATE HUNTER L, a & b FOR EACH READING

3. DISCARD OUT OF RANGE L, a, b READINGS

4. DISCARD HIGHEST AND LOWEST HUNTER L AND b VALUES FROM EACH SITE AND AVERAGE REMAINING VALUES FOR EACH SITE

5. RECORD $L_0$ & $b_0$ FOR EACH SITE

6. REPEAT STEPS 1-4 AT TIME $t_1, t_2, \ldots$ TO ARRIVE AT $L_1, a_1, \& b_1, L_2, a_2, \& b_2, \ldots$ FOR EACH SITE USING CONSISTENT SITES

7. FOR EACH SITE COMPARE $L_0 \& L_1, L_2 \ldots$ FOR CONSISTENCY

8. DISCONTINUE IF $L_0 \& L_1, L_2 \ldots$ FOR A SITE ARE INCONSISTENT UNLESS THERE HAS BEEN A CHANGE IN THE CONDITION IN THE SUBJECT (i.e. ANEMIA, PHOTOTHERAPY - THEN CALCULATE WITH AN ADJUSTMENT FACTOR)

9. CALCULATE $(b_1, b_2 \ldots) - b_0$

10. DETERMINE WHETHER $(b1, b2 \ldots) - b0$ IS DEMONSTRATING AN INCREASING HUNTER b WHEN L IS REMAINING CONSISTENT (FOR EACH SITE) AND WHEN INCREASING b IS SHOWING ENOUGH OF AN INCREASE TO INDICATE THE POSSIBILITY OF HYPERBILIRUBINEMIA BEING PRESENT ACCORDING TO THE CONSISTENT L RANGE FOR THAT SITE

11. RECOMMEND BLOOD TEST BE DONE

12. COMBINE EACH L, b VALUE FOR EACH SITE TO ARRIVE AT AVERAGE L, b

13. CALCULATE ESTIMATED BILIRUBINEMIA BLOOD TEST RESULT BY USING ALGORITHM IN DISCLOSURE

14. REQUEST BLOOD TEST

15. COMPARE CALCULATIONS WITH BLOOD TEST RESULTS SHOULD MATCH WITHIN +/- 1 UNIT VALUE

FIG. 2

EP 0 832 422 B1

**FIG. 3**

1. AT TIME $t_0$ MULTIPLE COLORIMETER MEASUREMENTS OF Y, x & y FROM EACH OF SEVERAL SITES

2. CALCULATE HUNTER L, a & b FOR EACH READING

3. DISCARD OUT OF RANGE L, a, b READINGS

4. DISCARD HIGHEST AND LOWEST HUNTER L AND b VALUES FROM EACH SITE AND AVERAGE REMAINING VALUES FOR EACH SITE

5. RECORD $L_0$, $a_0$ & $b_0$

6. REPEAT STEPS 1-5 AT TIMES t1, t2 ... TO ARRIVE AT L1, a1 & b1, L2, a2 & b2 ...

7. IF SIGNIFICANT INCREASE OR DECREASE IN HUNTER a FROM $a_0$ TO $a_1$ ETC., IDENTIFY IF THE CHANGE IN THE SUBJECTS CONDITION e.g. ANEMIA, ADVANCED WARNING OF BILIRUBINEMIA, INCREASED CIRCULATION, PHOTOTHERAPY, OTHER ILLNESS- AND USE ADJUSTMENT FACTOR TO COMPENSATE FOR LIGHTENING OR DARKENING OF THE SUBJECTS COLORATION WHEN L AND b ARE BOTH INCREASING OR DECREASING IN DIRECT RATIO FROM PREVIOUSLY CALCULATED L AND b FOR EACH SITE

8. COMPARE $L_0$ & $L_1$, $L_2$ ... FOR CONSISTENCY

9. DISCONTINUE IF $L_0$ & $L_1$, $L_2$ ... INCONSISTENT UNLESS THERE HAS BEEN A CHANGE IN THE CONDITION OF THE SUBJECT (i.e. ANEMIA, PHOTOTHERAPY- THEN CALCULATE WITH AN ADJUSTMENT FACTOR)

10. CALCULATE $(b_1, b_2 ...)$-$b_0$

11. DETERMINE WHETHER (b1, b2 ...)-b0 IS DEMONSTRATING AN INCREASING HUNTER b WHEN L IS REMAINING CONSTANT (FOR EACH SITE) AND WHEN INCREASING b IS SHOWING ENOUGH OF AN INCREASE TO INDICATE THE POSSIBILITY OF HYPERBILIRUBINEMIA BEING PRESENT ACCORDING TO THE CONSISTENT L RANGE FOR THAT SITE

12. RECOMMEND BLOOD TEST BE DONE

13. COMBINE EACH L, b VALUE FOR EACH SITE TO ARRIVE AT AVERAGE L, b

14. CALCULATE ESTIMATED BILIRUBINEMIA BLOOD TEST BY USING ALGORITHM IN DISLOSURE

15. REQUEST BLOOD TEST

16. COMPARE CALCULATIONS WITH BLOOD TEST- RESULTS SHOULD MATCH WITHIN +/- 1 UNIT VALUE